# EUROPEAN PATENT APPLICATION

(11) **EP 4 835 035 A1**
(43) Date of publication of application: **04.11.2026**
(21) Application number: 26188878.8
(22) Date of filing: 10.05.2023
(51) Int. Cl.: C09K 9/02

(54) **ELECTROCHROMIC COMPOUNDS, COMPOSITIONS AND OPTICAL ARTICLES CONTAINING THEM**

(62) Document number(s) of the earlier application(s) in accordance with Art. 76 EPC: 23315183.6 / 4 461 783
(71) Applicant: ESSILOR INTERNATIONAL, 94220 Charenton-Le-Pont (FR)
(72) Inventor: BIVER, Claudine, 94220 CHARENTON-LE-PONT (FR); BERIT-DEBAT, Fabien, 94220 CHARENTON-LE-PONT (FR); ARCHAMBEAU, Samuel, 94220 CHARENTON-LE-PONT (FR); AIKEN, Stuart, York (GB); ARMITAGE, Georgina K., Newcastle upon Tyne (GB); BROADBENT, Thomas David, Lancaster (GB); CROSSLEY, Daniel Luke, Normanton (GB); GABBUTT, Christopher David, Preston (GB); HERON, Bernard Mark, Brough (GB)
(74) Representative: Plasseraud IP

(57) **Abstract**

The invention relates to a group of novel electrochromic compounds. More specifically, it relates to electrochromic polycyclic compounds comprising a six-membered ring, wherein said polycyclic compounds contain at least two nitrogen atoms in their cyclic central core. It also relates to the use of these compounds as a variable transmittance medium for the manufacture of an optical article, such as an ophthalmic lens.

## Description

The present invention relates to a group of novel electrochromic compounds. More specifically, it relates to electrochromic polycyclic compounds comprising a five, six or seven-membered ring, wherein said polycyclic compounds contain at least two nitrogen atoms in their cyclic central core. It also relates to the use of these compounds as a variable transmittance medium for the manufacture of an optical article, such as an ophthalmic lens.

Electrochromism is a well-known physical phenomenon which is observed with certain classes of chemical compounds that reversibly change colour when a voltage is applied to them. The material undergoes reversible changes in optical properties by oxidation and reduction. Advantageously, the electrochromic material is colourless when an electric field is not applied and becomes coloured when an electric field is applied.

An electrochromic device, i.e. a device containing electrochromic compounds, the absorbance of which depends only on the presence of an electric field, can thus have two states, i.e. a coloured state (when electrically activated) and a bleached state (in the inactive state). The optical transmission properties of the device depend on the nature of the electrochromic compounds.

When preparing an electrochromic composition to be used as transparent media for forming high quality optical articles, in particular high quality ophthalmic lenses, the choice of electrochromic compounds is critical. Indeed, electrochromic compounds need not only to show good electrochromic properties such as high absorption of the visible light in the coloured state, low absorption of visible radiations in the bleached state, fast colouring and fading rates, but should also have long-term stability, in particular in the presence of oxygen, and good solubility in conventional solvents. Gathering all the required properties in one single compound is a real challenge. Many studies have already been conducted for providing electrochromic compounds having the best compromise. For example, viologen compounds have been identified as compounds of particular interest due to their high molar absorption coefficient. Their molar absorption coefficient is indeed higher than other electrochromic compounds, such as ferrocene or dihydrophenazine derivatives, usually used in electrochromic devices.

Another difficulty to face when using electrochromic composition in ophthalmic applications is to meet the consumer demand, which requires a wide range of colours available, and in particular neutral colours (i.e. brown, grey, grey-green...). Such neutral colour can be preferentially chosen with respect of the ISO Standard 1836, which defines the relative visual attenuation coefficient of filters of categories 0, 1, 2 and 3. Other examples of tints defining a neutral colour are given in documents US 6,255,238 and US 5,438,024.

Electrochromic composition having the desired colour can be obtained by mixing different electrochromic compounds. These different electrochromic compounds can be oxidizing electrochromic compounds as well as reducing electrochromic compounds. However, obtaining the desired colour for an electrochromic composition is an exercise much more complex than simply mixing colours. Indeed, in addition to the numerous requirements that the electrochromic compounds should meet, the challenge of using a combination of different electrochromic compounds lays in the compatibility of said electrochromic compounds with each other. For instance, the absorption coefficient of commonly used electrochromic reducing compounds is generally far lower than electrochromic oxidizing compounds such as viologen compounds. On the other hand, the use of a combination of several electrochromic oxidizing compounds in a single composition further requires that the electrochromic oxidizing compounds have oxydo-reducing potentials close enough so that they can change colour simultaneously when a potential is applied to the composition.

There remains a need for new electrochromic materials allowing the expansion of the range of colours from blue to green, red, orange and yellow with different activation potentials, *i.e.* new electrochromic materials allowing to achieve various desired tints with no chameleon effect.

After conducting extensive research, the present inventors provide novel electrochromic polycyclic compounds comprising a five, six or seven-membered ring and containing at least two nitrogen atoms in their cyclic central core. The present inventors have indeed found that introducing an azine ring or azine moiety in the structure of traditional electrochromic viologen molecules can be very interesting. Such modification can for instance lower the absorption wavelength in the visible range. This is the case for instance with substituted diazine, triazine, and related fused rings.

The present invention thus relates to electrochromic compounds of formula (I) as defined below. These compounds are advantageously:
- colourless in their inactivated state and coloured, for example green, red, purple, blue, yellow or brown, in their activated state;
- reversibly oxidized or reduced;
- easily activated, i.e. they have an electrochemical potential from -1.5 to -0.5 V;
- stable, i.e. no generation of degradation products.

More particularly the compounds of the present invention exhibit either one low reversible reduction peak or two reversible reduction peaks, the two reversible reduction peaks separated by at least 0.1V, preferably at least 0.3 V, more preferably at least 0.4 V, even more preferably at least 0.5 V.

The present invention also relates to an electrochromic composition comprising at least one compound of formula (I).

Finally, the present invention relates to an electrochromic device, such as an ophthalmic lens, comprising an electrochromic compound of formula (I) or an electrochromic composition according to the invention.

### Definitions

As used herein, the expression "aromatic compound" or "aromatic ring" or "heteroaromatic ring" means unsaturated chemical compounds characterized by one or more planar rings of atoms joined by covalent bonds.

The expression "cyclic compound" or "ring compound" or "ring" corresponds to a compound in which one or more series of atoms in the compound is connected to form a ring. Rings may vary in size from three to many atoms, for example 5 or 6 or 7 atoms and include examples where all the atoms are carbon (i.e., are carbocycles), or where both carbon and non-carbon atoms are present (heterocyclic compounds). More precisely, a "heterocyclic compound or ring structure" is a cyclic compound that has atoms of at least two different elements as members of its ring(s).

The expression "Polycyclic compounds" means an organic compound featuring several closed rings of atoms, primarily carbon. These ring substructures include cycloalkanes, aromatics, and other ring types. They come in sizes of three atoms and upward, and in combinations of linkages that include tethering (such as in biaryls for example biphenyl), fusing (edge-to-edge, such as in anthracene and steroids), links via a single atom (such as in spiro compounds), bridged compounds. Examples of polycyclic compounds are bicyclic, tricyclic, tetracyclic compounds, etc.. The term "polycyclic" includes polycyclic aromatic compounds, including polycyclic aromatic hydrocarbons, as well as heterocyclic aromatic compounds with multiple rings (where heteroaromatic compounds are aromatic compounds that contain sulfur, selenium, nitrogen, oxygen, or other non-carbon atoms in their rings in addition to carbon). A polyaromatic hydrocarbon may have rings of various sizes, including some that are not aromatic.

The expression "hydrocarbon" means a chemical compound containing only carbon and hydrogen.

The expression "polycyclic aromatic hydrocarbon" is a hydrocarbon that is composed of multiple aromatic rings. The group is a major subset of the aromatic hydrocarbons. The simplest of such chemicals are naphthalene, having two aromatic rings, and the three-ring compounds anthracene and phenanthrene.

The expression "aryl" represents any monovalent radical of an aromatic hydrocarbon comprising 6 to 18 carbon atoms, notably 6 to 10 carbon atoms, either monocyclic or polycyclic. Examples of C₆-C₁₈ aryl groups include phenyl, naphthyl, anthracenyl and phenanthrenyl. The expressions "aryl" and "aromatic hydrocarbon ring" have the same meaning.

The expression "heteroaryl" represents any monovalent radical of a monocyclic or polycyclic 5 to 10 membered aromatic group (such as a bicyclic group) comprising from 1 to 3 heteroatoms independently selected from oxygen, nitrogen, selenium and sulfur. Thus, the expression "heteroaryl" include the optionally substituted nitrogen-containing aromatic rings, or optionally substituted bicyclic aromatic rings with two (2) nitrogen atoms. The expressions "heteroaryl" and "hetero aromatic ring" have the same meaning. Examples of 5 to 10 membered heteroaryl groups include furyl, thienyl (or thiophen-2-yl or thiophen-3-yl), selenophenyl, pyrrolyl, pyrazoyl, imidazolyl, isoxazolyl, isothiazoyl, thiazolyl, oxazolyl, 1,2,3-triazolyl, 1,2,4-triazolyl, 1-benzofuryl, 1-benzothienyl, indolyl, benzimidazolyl, benzimidazolium, benzimidazoliumyl, indazolyl, 1,2-benzisoxazolyl, 2,1-benzisoxazolyl, 1,2-benzisothiazolyl, 2,1-benzisothiazolyl, benzothiazolyl, benzoxazolyl, benzoxazoliumyl, benzoselenazoliumyl, benzotriazolyl, pyridinyl, pyridiniumyl, N-alkylpyridiniumyl, N-arylpyridiniumyl, quinolinyl, quinolinium, quinoliniumyl, isoquinolinyl, isoquinolinium, isoquinoliniumyl, pyridazinyl, cinnolinyl, phthalazinyl, pyrimidinyl, quinazolinyl, pyrazinyl and quinoxalinyl.

The expression "optionally substituted" with regard to groups, including but not limited to hydrocarbyl groups, alkyl groups, cycloalkyl groups, aryl groups and heteroaryl groups, refers to one of said groups in which at least one hydrogen thereof has been replaced or substituted with a group that is other than hydrogen, such as, but not limited to, halo groups (e.g., F, Cl, I, and Br), hydroxyl groups, ether groups, thiol groups, thio ether groups, carboxylic acid groups, carboxylic acid ester groups, phosphoric acid groups, phosphoric acid ester groups, sulfonic acid groups, sulfonic acid ester groups, nitro groups, cyano groups, hydrocarbyl groups (including, but not limited to: alkyl; alkenyl; alkynyl; cycloalkyl, including poly-fused-ring cycloalkyl and polycycloalkyl; heterocycloalkyl; aryl, including hydroxyl substituted aryl, such as phenol, and including poly-fused-ring aryl; heteroaryl, such as pyridinium or including poly-fused-ring heteroaryl: and aralkyl groups), and amine groups, such as -N(R^{a})(R^{b}) where R^{a} and R^{b} are each independently selected from hydrogen, hydrocarbyl and substituted hydrocarbyl.

In a particular embodiment "optionally substituted" means substituted by at least one moiety selected from the group consisting of C₁-C₁₈ alkyl, aryl (substituted or non-substituted), -CH₂-aryl and heteroaryl (substituted or non-substituted).

In one particular embodiment, the expression "optionally substituted" means substituted by at least one Y with Y being C₁-C₁₈ alkyl, optionally substituted aryl or -CH₂-aryl.

In another particular embodiment, the term "optionally substituted aryl" refers to a substituted or non-substituted aryl moiety, said moiety being selected from the group consisting of phenyl, naphthyl, biphenyl, halophenyl, C₁-C₁₈ alkylphenyl, C₁-C₁₈ haloalkylphenyl and C₁-C₁₈ alkoxyphenyl, more preferably an aryl moiety selected from the group consisting of phenyl, naphthyl, biphenyl, 1-fluorophenyl, 4-methylphenyl, 1,3-dimethylphenyl, 4-trifluoromethylphenyl and 4-methoxyphenyl.

In another particular embodiment, the term "optionally substituted heteroaryl" refers to a substituted or non-substituted heteroaryl moiety, said moiety being selected from the group consisting of thienyl (substituted or non-substituted), pyridiniumyl, N-alkylpyridiniumyl, N-arylpyridiniumyl, N-alkylarylpyridiniumyl and Z, wherein Z is 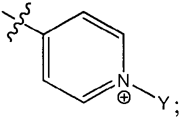 and
Y is C₁-C₁₈ alkyl, optionally substituted aryl or -CH₂-aryl, wherein optionally substituted aryl is defined as above herein. Preferably, Z is selected from the group consisting of N-hexylpyridiniumyl, N-4-methylphenylpyridiniumyl and N-benzylpyridiniumyl.

More preferably, the thienyl group is thiophen-2-yl or thiophen-3-yl, the N-alkylpyridiniumyl group is a N-hexylpyridiniumyl group and the N-alkylarylpyridiniumyl is a N-benzylpyridiniumyl group or a N-4-methylphenylpyridiniumyl group.

In another preferred embodiment, the term "substituted thienyl" group refers to a thienyl group substituted with two N-benzylpyridiniumyl groups.

The expression "hydrocarbyl" represents any univalent radical, derived from a hydrocarbon.

The expression "alkyl" or "C₁-C₁₈ alkyl" represents any monovalent radical of a linear or branched hydrocarbon chain comprising 1 to 18 carbon atoms. The expression "C₁-C₆ alkyl" represents an alkyl group having 1 to 6 carbon atoms. Examples of C₁-C₁₈ alkyl groups include C₁-C₄ alkyl groups such as methyl, ethyl, *n*-propyl, *i*-propyl, *n*-butyl, *i*-butyl, *s*-butyl or *t*-butyl, C₆-C₁₈ alkyl groups such as *n*-hexyl, *n*-heptyl or *n*-octyl, as well as *n*-pentyl, 2-ethylhexyl, 3,5,5-trimethylhexyl, *n*-nonyl, *n*-decyl, *n*-undecyl, *n*-dodecyl or *n*-octadecyl.

The expression "alkoxy" means an alkyl-O- group, wherein said alkyl is as defined herein. Examples of alkoxy groups notably include methoxy or ethoxy.

The expression "Hal" or "halo group" means a halogen atom substituent such as F, Cl, Br, I.

The expression "haloalkyl" means an alkyl group which hydrogen atoms are substituted by one more halogen atoms, said alkyl group being as defined herein. Examples of haloalkyl groups notably include the trifluoromethyl group (-CF₃).

The expression "arylalkyl" or "aralkyl" represents any aryl derivative of an alkyl group. In other words, it represents any univalent radical derived from an alkyl radical by replacing one or more hydrogen atoms by aryl groups. It represents an aryl group as defined above combined to an alkyl group as defined above. Examples of arylalkyl groups include benzyl (or CH₂-aryl), phenylethyl, phenylpropyl, phenylbutyl, phenylpentyl, phenylhexyl, naphthylmethyl, naphthylethyl, naphthylpropyl, naphthylbutyl, naphthylpentyl, naphthylhexyl, anthracenylmethyl, anthracenylethyl, anthracenylpropyl, anthracenylbutyl, anthracenylpentyl, anthracenylhexyl, phenanthrenylmethyl, phenanthrenylethyl, phenanthrenylpropyl, phenanthrenylbutyl, phenanthrenylpentyl and phenanthrenylhexyl.

The expression "pyridiniumyl group", also called herein **Z,** represents any radical of a pyridinium which is an aromatic group comprising 5 carbon atoms and a positively charged nitrogen. The pyridiniumyl group can be substituted, for example substituted by **Y** as represented below: 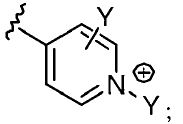 wherein **Y** are independently selected from C₁-C₁₈ alkyl, optionally substituted aryl and -CH₂-aryl.

The expression "N-alkylpyridiniumyl group" represents any radical of a pyridinium which is an aromatic group comprising 5 carbon atoms and a positively charged nitrogen, said nitrogen being substituted by an alkyl group. The N-alkylpyridiniumyl group can be substituted, for example substituted by **Y,** as represented below: 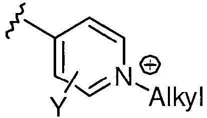 wherein **Y** are independently selected from C₁-C₁₈ alkyl, optionally substituted aryl and -CH₂-aryl.

Preferably Z is selected from the group consisting of N-hexylpyridiniumyl, N-4-methylphenylpyridiniumyl and N-benzylpyridiniumyl.

### Electrochromic compounds

As such, the electrochromic compounds of the present invention are electrochromic polycyclic compounds represented by formula (I): 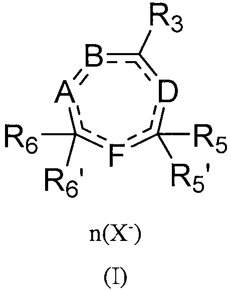 wherein:
**A is** N, N-R₁ or ⁺N-R₁;
**B is** C-R₂, or N;
**D** is N, N-R₄ or ⁺N-R₄;
**F** is present or not, and, if present, is -CH₂-;
**R₁** is C₁-C₁₈ alkyl, optionally substituted aryl or -CH₂-aryl;
**R₂** is H or optionally substituted aryl;
**R₃** is H, C₁-C₁₈ alkyl, optionally substituted aryl or optionally substituted heteroaryl, such as **Z** or -CH₂-aryl;
**R₄** is C₁-C₁₈ alkyl, optionally substituted aryl or -CH₂-aryl;
**R_{5'}** and **R_{6'}** are present or not and, if present, are both H;
**R₅** is H, C₁-C₁₈ alkyl, C₁-C₁₈ alkoxy, optionally substituted aryl or optionally substituted heteroaryl;
**R₆** is H, optionally substituted aryl, optionally substituted heteroaryl or **Z;**
and/or **R₅** and **R₆** may form together an optionally substituted aromatic hydrocarbon ring, such as an arylene, for example a benzene, or a fused ring variant like a naphthalene, or an optionally substituted heteroaromatic ring, such as a thiophene, a selenophene, a pyridine, a pyridinium fused to the central nitrogen-containing heterocyclic ring they are attached to, said aromatic hydrocarbon ring or substituted heteroaromatic ring being optionally substituted for instance by at least one **Y,** or by one or two **Z;**
and/ or **R₃** and **R₄** may form together an optionally substituted nitrogen-containing aromatic ring, such as a pyridine, a pyridinium, a pyrimidine, a pyrimidinium, a pyridazine, a pyridazinium, a pyrazine, a pyrazinium, or an optionally substituted bicyclic aromatic ring containing one or more nitrogen atoms, such as a quinolinium or a benzimidazolium, fused to the central nitrogen-containing heterocyclic ring they are attached to, said aromatic rings being for example substituted by at least one **Y;**
and/or **R₁** and **R₂** may form together an optionally substituted nitrogen-containing aromatic ring, such as a pyridinium, or an optionally substituted bicyclic aromatic ring containing one nitrogen atom and one or more heteroatoms selected from the group consisting of nitrogen, oxygen, sulfur and selenium, such as a quinolinium or a benzimidazolium, or a benzothiazolium, or a benzoselenazolium, or a benzoxazolium, fused to the central nitrogen-containing heterocyclic ring they are attached to, said aromatic rings being for example substituted by at least one **Y,** for example substituted by an optionally substituted aryl;
and/ or **R₂** and **R₃** may form together an optionally substituted heteroaromatic ring, such as a thiophene, a pyridinium, an optionally substituted aromatic hydrocarbon ring or an optionally substituted nitrogen-containing non aromatic ring, such as seven-membered ring, a saturated diazepine fused to the central nitrogen-containing heterocyclic ring they are attached to, said rings being for example substituted by at least one **Y;**
**Z** is 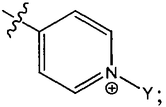
**Y** is C₁-C₁₈ alkyl, optionally substituted aryl or -CH₂-aryl;
**n** is selected to counterbalance the number of positive charges and n **is at least 2;**
**X** is an anion;
- - - - - - is a single bond or a double bond;
**with the proviso that** the central nitrogen-containing heterocyclic ring comprises at least two (2) double bonds;
   and
**with the proviso that** said electrochromic polycyclic compounds comprise at least two rings in addition to the central nitrogen-containing heterocyclic ring (fused or not to the central nitrogen-containing heterocyclic ring), at least one of said two rings being a nitrogen-containing ring.

In all the present invention, unless otherwise stated, **Y** is advantageously C₁-C₁₈ alkyl, optionally substituted aryl or -CH₂-aryl; for example, **Y** is methyl, *n*-hexyl, phenyl, 4-*ter* butyl, or benzyl.

In all the present invention, the counterion **X⁻** may be selected from halide, preferably fluoride and chloride, tetrafluoroborate, tetraphenylborate, hexafluorophosphate, nitrate, methanesulfonate, trifluoromethanesulfonate, *p*-toluenesulfonate, hexachloroantimonate, bis(trifluoromethanesulfonyl)imide, perchlorate, acetate and sulfate, or any mixture thereof; preferably, **X⁻** is tetrafluoroborate or hexafluorophosphate, or a mixture of both.

In certain aspects of the invention, there are included compounds of formula (I), wherein **R₁** and **R₂** form together a nitrogen-containing aromatic ring, or a bicyclic aromatic ring containing one nitrogen atom and one or more heteroatoms selected from the group consisting of nitrogen, oxygen, sulfur and selenium, said aromatic rings being fused to the central nitrogen-containing heterocyclic ring they are attached to, and said aromatic rings being optionally substituted by one to three **Y** groups selected from C₁-C₆ alkyl or C₆-C₁₀ aryl, said aryl group being optionally substituted by a C₁-C₆ haloalkyl, or a C₁-C₆ alkoxy. Examples of **Y** groups notably include methyl, phenyl, said phenyl being optionally substituted by trifluoromethyl, or methoxy.

In other aspects of the invention, there are included compounds of formula (I), wherein **R₃** and **R₄** form together a nitrogen-containing aromatic ring, or a bicyclic aromatic ring containing one or more nitrogen atoms, said aromatic rings being fused to the central nitrogen-containing heterocyclic ring they are attached to, and said aromatic rings being optionally substituted by one to three **Y** groups selected from C₁-C₆ alkyl or C₆-C₁₀ aryl, said aryl group being optionally substituted by a C₁-C₆ haloalkyl, or a C₁-C₆ alkoxy. In a particular embodiment, the nitrogen-containing aromatic ring, or bicyclic aromatic ring containing one or more nitrogen atoms, is not substituted.

In additional aspects of the invention, there are included compounds of formula (I) wherein **R₂** is H or C₆-C₁₀ aryl, said C₆-C₁₀ aryl being optionally substituted by C₁-C₆ alkyl, C₁-C₆ haloalkyl, or C₁-C₆ alkoxy; in particular said C₆-C₁₀ aryl is phenyl.

In additional aspects of the invention, there are included compounds of formula (I), wherein **R₃** is C₆-C₁₀ aryl or **Z** : 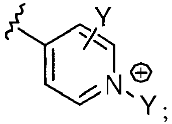
wherein said C₆-C₁₀ aryl is optionally substituted by C₁-C₆ alkyl, C₁-C₆ haloalkyl, or C₁-C₆ alkoxy; in particular said C₆-C₁₀ aryl is phenyl;
and wherein **Y** is C₁-C₁₈ alkyl or C₆-C₁₀ aryl, and wherein said C₆-C₁₀ aryl is optionally substituted by one to three groups selected from C₁-C₆ alkyl.

In other aspects of the invention, there are included compounds of formula (I), wherein **R₅** and **R₆** form together an aromatic hydrocarbon ring, or heteroaromatic ring, said aromatic hydrocarbon ring or heteroaromatic being fused to the central nitrogen-containing heterocyclic ring they are attached to, and said aromatic hydrocarbon ring or heteroaromatic ring being optionally substituted by one or two **Z** : 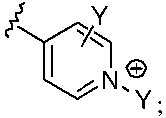 wherein **Y** is -CH₂-(C₆-C₁₀ aryl), such as benzyl. For example, **R₅** and **R₆** may form together an aromatic hydrocarbon ring, such as an aryl, for example a phenyl, or a heteroaromatic ring, such as a thiophene or a selenophene, fused to the central nitrogen-containing six-membered heterocyclic ring they are attached to, and said aromatic hydrocarbon ring or heteroaromatic ring being optionally substituted by one or two **Z** : 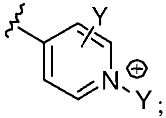 wherein Y is -CH₂-(C₆-C₁₀ aryl), such as benzyl.

In additional aspects of the invention, there are included compounds of formula (I), wherein **R₅** is H, C₁-C₆ alkyl, C₁-C₆ alkoxy, C₆-C₁₀ aryl or 5 to 10 membered heteroaryl, said aryl or heteroaryl being optionally substituted by one to three groups selected from Hal, or C₁-C₆ alkyl, C₁-C₆ haloalkyl. As an example, **R₅** may be selected from H, methyl, thienyl, or phenyl optionally substituted by Hal, for example F.

In additional aspects of the invention, there are included compounds of formula (I), wherein, **R₆** is H, C₆-C₁₀ aryl, said aryl being optionally substituted by one to three groups selected from C₁-C₆ alkyl, C₁-C₆ haloalkyl, C₆-C₁₀ aryl, or **R₆** is **Z** : 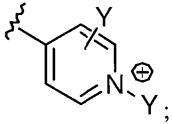 wherein **Y** is a C₁-C₁₈ alkyl, C₆-C₁₀ aryl, said aryl being optionally substituted by one to three groups selected from C₁-C₆ alkyl. As an example, **R₆** may be selected from H, naphthyl, phenyl, said phenyl being optionally substituted by phenyl, methyl and/or trifluoromethyl, or **R₆** may be selected from **Z** with **Y** being *n*-hexyl, or tolyl.

In a particular aspect of the invention, the compounds of the invention are the electrochromic polycyclic compounds of formula (I): 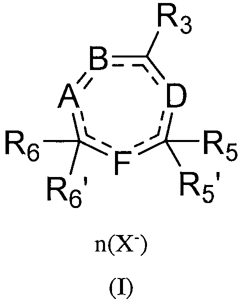 wherein:
**A** is N, N-R₁ or ⁺N-R₁;
**B is** C-R₂, or N;
**D** is N N-R₄ or ⁺N-R₄;
**F** is present or not, and, if present, is -CH₂-;
**R₃** is H, optionally substituted aryl or optionally substituted heteroaryl, such as **Z;**
**R_{5'}** and **R_{6'}** are present or not and, if present, are both H;
**R₅** is H, C₁-C₁₈ alkyl, C₁-C₁₈ alkoxy, optionally substituted aryl or optionally substituted heteroaryl;
**R₆** is H, optionally substituted aryl, optionally substituted heteroaryl or **Z;**
and/or **R₅** and **R₆** may form together an optionally substituted aromatic hydrocarbon ring, such as an arylene, for example a benzene, or a fused ring variant like a naphthalene, or an optionally substituted heteroaromatic ring, such as a thiophene, a selenophene, a pyridine, a pyridinium fused the central nitrogen-containing heterocyclic ring they are attached to, said aromatic hydrocarbon ring or substituted heteroaromatic ring being optionally substituted for instance by at least one **Y,** by one **Z** or by two **Z;**
and/ or when **D** is N-R₄ or ⁺N-R₄, **R₃** and **R₄** form together an optionally substituted nitrogen-containing aromatic ring, such as a pyridine, a pyridinium, a pyrimidine, a pyrimidinium, a pyridazine, a pyridazinium, a pyrazine, a pyrazinium, or an optionally substituted bicyclic aromatic ring containing one or more nitrogen atoms, such as a quinolinium or a benzimidazolium, fused to the central nitrogen-containing heterocyclic ring they are attached to, said aromatic rings being for example substituted by at least one **Y;**
and/or when **A** is N-R₁ or ⁺N-R₁, **R₁** and **R₂** form together an optionally substituted nitrogen-containing aromatic ring, such as a pyridinium, or an optionally substituted bicyclic aromatic ring containing one nitrogen atom and one or more heteroatoms selected from the group consisting of nitrogen, oxygen, sulfur and selenium, such as a quinolinium or a benzimidazolium, or a benzothiazolium, or a benzoselenazolium, or a benzoxazolium, fused to the central nitrogen-containing heterocyclic ring they are attached to, said aromatic rings being for example substituted by at least one **Y,** for example substituted by an optionally substituted aryl;
and/ or **R₂** and **R₃** may form together an optionally substituted heteroaromatic ring, such as a thiophene, a pyridinium, an optionally substituted aromatic hydrocarbon ring or an optionally substituted nitrogen-containing non aromatic ring, such as seven-membered ring, a saturated diazepine fused to the central nitrogen-containing heterocyclic ring they are attached to, said rings being for example substituted by at least one **Y;**
**Z** is 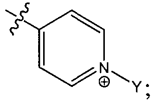
**Y** is C₁-C₁₈ alkyl, optionally substituted aryl or -CH₂-aryl;
**n** is selected to counterbalance the number of positive charges and **n is at least 2;**
**X** is an anion;
- - - - - - is a single bond or a double bond;
**with the proviso that** the central nitrogen-containing heterocyclic ring comprises at least two (2) double bonds;
   and
**with the proviso that** said electrochromic polycyclic compounds comprise at least two rings in addition to the central nitrogen-containing heterocyclic ring (fused or not to the central nitrogen-containing heterocyclic ring), at least one of said two rings being a nitrogen-containing ring.

In some particular aspects of the invention, when **D** is ⁺N-R₄, **B** is C-R₂ and **A** is ⁺N-R₁:
- **R₃** and **R₄** form together a pyridinium or an optionally substituted benzimidazolium ring fused to the central nitrogen-containing heterocyclic ring they are attached to; and
- **R₁** and **R₂** form together a pyridinium, an optionally substituted quinolinium, or an optionally substituted benzimidazolium, or an optionally substituted benzothiazolium, fused to the central nitrogen-containing heterocyclic ring they are attached to.

In another particular aspect of the invention, when **D** is ⁺N-R₄, **B** is C-R₂ and **A** is ⁺N-R₁:
- **R₃** and **R₄** form together a pyridinium or N-methyl-benzimidazolium ring fused to the central nitrogen-containing heterocyclic ring they are attached to; and
- **R₁** and **R₂** form together a pyridinium, a quinolinium, a phenyl-substituted quinolinium, an alkoxyphenyl-substituted quinolinium, a haloalkylphenyl-substituted quinolinium, a benzothiazolium, or an optionally N-alkyl substituted benzimidazolium ring fused to the central nitrogen-containing heterocyclic ring they are attached to; even more preferably wherein the alkoxyphenyl-substituted quinolinium is a 4-[4-methoxyphenyl)-quinolinium], the haloalkylphenyl-substituted quinolinium is a 4-[4-trifluoromethylphenyl)-quinolinium] and the N-alkyl substituted benzimidazolium is a N-methyl benzimidazolium;
and wherein, when **R₃** and **R₄** form together a benzimidazolium ring and when **R₁** and **R₂** form together a benzimidazolium ring, then **R₂** and **R₃** form together a saturated diazepine fused to the central nitrogen-containing heterocyclic ring they are attached to.

**In a first variant of the present invention,** the compounds of the invention are the electrochromic polycyclic compounds with a central nitrogen-containing six membered heterocyclic ring and of **Formula (II):** 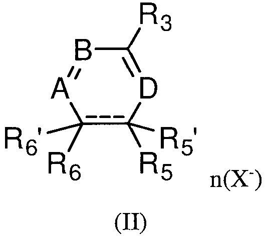 wherein:
**A** is N or ⁺N-R₁;
**B is** C-R₂, or N;
**D is** N or ⁺N-R₄;
**R₁** is C₁-C₁₈ alkyl, optionally substituted aryl or -CH₂-aryl
**R₂** is H or optionally substituted aryl;
**R₃** is H, C₁-C₁₈ alkyl, optionally substituted aryl or optionally substituted heteroaryl such as **Z** or -CH₂-aryl;
**R₄** is C₁-C₁₈ alkyl, optionally substituted aryl or -CH₂-aryl;
**R_{5'}** and **R_{6'}** are present or not and, if present, are both H;
**R₅** is H, C₁-C₁₈ alkyl, C₁-C₁₈ alkoxy, optionally substituted aryl or optionally substituted heteroaryl;
**R₆** is H, optionally substituted aryl, optionally substituted heteroaryl or **Z;**
and/or **R₅** and **R₆** may form together an optionally substituted aromatic hydrocarbon ring, such as an arylene, for example a benzene, or an optionally substituted heteroaromatic ring, such as a thiophene, a selenophene, a pyridine, a pyridinium, fused to the central nitrogen-containing six-membered heterocyclic ring they are attached to, said aromatic hydrocarbon ring or heteroaromatic ring being optionally substituted for instance by at least one **Y,** or by one or two **Z;**
and/ or **R₃** and **R₄** may form together an optionally substituted nitrogen-containing aromatic ring, such as a pyridinium, or an optionally substituted bicyclic aromatic ring containing one or more nitrogen atoms, such as a quinolinium or a benzimidazolium, fused to the central nitrogen-containing six membered heterocyclic ring they are attached to, said aromatic rings being for example substituted by at least one **Y,** for example substituted by an optionally substituted aryl;
and/or **R₁** and **R₂** may form together an optionally substituted nitrogen-containing aromatic ring, such as a pyridinium, or an optionally substituted bicyclic aromatic ring containing one nitrogen atom and one or more heteroatoms selected from the group consisting of nitrogen, oxygen, sulfur and selenium, such as a quinolinium or a benzimidazolium, or a benzothiazolium, or a benzoselenazolium, or a benzoxazolium, fused to the central nitrogen-containing six membered heterocyclic ring they are attached to, said aromatic rings being for example substituted by at least one **Y,** for example substituted by an optionally substituted aryl;
and/ or **R₂** and **R₃** may form together an optionally substituted heteroaromatic ring, such as a thiophene, a pyridinium or an optionally substituted aromatic hydrocarbon ring fused to the central nitrogen-containing six-membered heterocyclic ring they are attached to, for example substituted by at least **Y;**
**with the proviso that** said electrochromic polycyclic compounds comprise at least two rings in addition to the central nitrogen-containing six-membered heterocyclic ring (fused or not to the central nitrogen-containing six-membered heterocyclic ring), at least one of said two rings being a nitrogen-containing ring;
and **Z, Y, n and X** are as defined above.

*In one embodiment of this first variant,* the compounds of **formula (II)** are those wherein A is ⁺N-R₁; **D** is ⁺N-R₄; **R₃** and **R₄** form together a pyridinium fused to the central nitrogen-containing six-membered heterocyclic ring and are represented by **formula (III):** 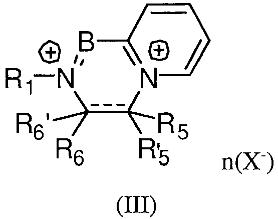 wherein:
**B** is C-R₂;
**R₁** and **R₂** form together an optionally substituted nitrogen-containing aromatic ring, such as a pyridinium, or an optionally substituted bicyclic aromatic ring with containing one nitrogen atom and one or more heteroatoms selected from the group consisting of nitrogen, oxygen, sulfur and selenium, such as a quinolinium or a benzimidazolium, or a benzothiazolium, or a benzoselenazolium, or a benzoxazolium, fused to the central nitrogen-containing six-membered heterocyclic ring they are attached to, said aromatic rings being for example substituted by at least one **Y,** for example substituted by an optionally substituted aryl;
**R₅** is H, C₁-C₁₈ alkyl, C₁-C₁₈ alkoxy, optionally substituted aryl or optionally substituted heteroaryl;
**R₆** is H, C₁-C₁₈ alkyl, optionally substituted aryl, optionally substituted heteroaryl or **Z;**
**R_{5'}** and **R_{6'}** are present or not and, if present, are both H;
and **Z, Y,** - - - - - - **, n** and **X** being as defined above.

According to this particular embodiment, compounds can be those represented by **formula (III a), (III b), (III c)** or **(III d):** 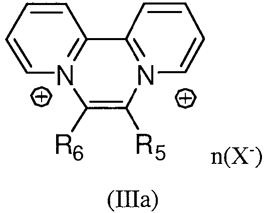 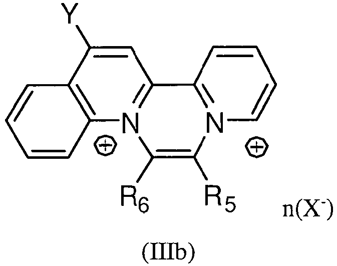 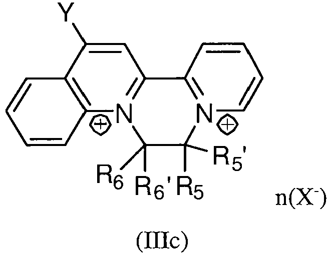 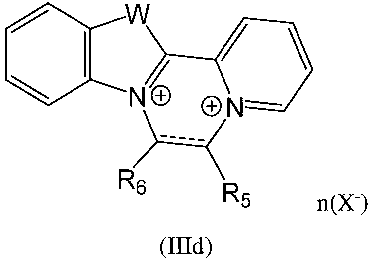 wherein
**R₅** is H, C₁-C₁₈ alkyl, C₁-C₁₈ alkoxy, optionally substituted aryl or optionally substituted heteroaryl;
**R₆** is H, C₁-C₁₈ alkyl, optionally substituted aryl, optionally substituted heteroaryl or **Z;**
**R_{5'}** and **R_{6'}** are present or not and, if present, are both H;
**W is** N-Y, S, O or Se;
and **Z, Y,** - - - - - - **, n** and **X** are as defined above.

Preferred compounds can be those of **formula (IIIa)** wherein **R₅** is H, C₁-C₆ alkyl, C₁-C₆ alkoxy optionally substituted C₆-C₁₈ aryl, optionally substituted 5-membered heteroaryl or optionally substituted 6-membered heteroaryl; preferably **R₅** is H, C₁-C₃ alkyl, optionally substituted C₆ aryl or non-substituted 5 membered heteroaryl; more preferably **R₅** is H, methyl, phenyl, naphthyl (such as naphthalen-2-yl), substituted phenyl (such as 2-fluoro-phenyl, 4-(trifluoromethyl)phenyl, 2,4-(dimethylphenyl)), non-substituted 5 membered heteroaryl with one heteroatom such as thiophen-2-yl or thiophen-3-yl; and **R₆** is also H, C₁-C₆ alkyl, optionally substituted C₆-C₁₈ aryl, optionally substituted 5-membered heteroaryl or optionally substituted 6-membered heteroaryl; preferably **R₆** is H, C₁-C₃ alkyl, optionally substituted C₆ aryl or non-substituted 5 membered heteroaryl; more preferably **R₆** is H, methyl, phenyl, naphthyl (such as naphthalen-2-yl), substituted phenyl (such as 2-fluoro-phenyl, 4-(trifluoromethyl)phenyl, 2,4-(dimethylphenyl)), non-substituted 5 membered heteroaryl with one heteroatom such as thiophen-2-yl or thiophen-3-yl; **n** is 2; and **X⁻** is tetrafluoroborate or hexafluorophosphate, preferably tetrafluoroborate.

Preferred compounds can be those of **formula (IIIb)** or **formula (IIIc)** wherein **R₅** and **R₆** are H; **Y** is optionally substituted aryl, **Y** is preferably optionally substituted phenyl, more preferably **Y** is phenyl, 4-methoxyphenyl or 4-(trifluoromethyl)phenyl; **n** is 2; and **X⁻** is tetrafluoroborate or hexafluorophosphate, preferably tetrafluoroborate.

Preferred compounds can be those of **formula (IIId)** wherein,
**W** is S or N-Y, and wherein **Y** is C₁-C₁₈ alkyl, **Y** is preferably C₁-C₆ alkyl, more preferably Y is C₁-C₄ alkyl, for example methyl;
**R₅** is H, C₁-C₆ alkyl, C₁-C₆ alkoxy optionally substituted C₆-C₁₈ aryl, optionally substituted 5-membered heteroaryl or optionally substituted 6-membered heteroaryl; preferably **R₅** is H, C₁-C₃ alkyl, optionally substituted C₆ aryl or non-substituted 5 membered heteroaryl; more preferably **R₅** is H, methyl, phenyl, naphthyl (such as naphthalen-2-yl), substituted phenyl (such as 2-fluoro-phenyl, 4-(trifluoromethyl)phenyl, 2,4-(dimethylphenyl)), non-substituted 5 membered heteroaryl with one heteroatom such as thiophen-2-yl or thiophen-3-yl; and
**R₆** is also H, C₁-C₆ alkyl, optionally substituted C₆-C₁₈ aryl, optionally substituted 5-membered heteroaryl or optionally substituted 6-membered heteroaryl; preferably **R₆** is H, C₁-C₃ alkyl, optionally substituted C₆ aryl or non-substituted 5 membered heteroaryl; more preferably **R₆** is H, methyl, phenyl, naphthyl (such as naphthalen-2-yl), substituted phenyl (such as 2-fluoro-phenyl, 4-(trifluoromethyl)phenyl, 2,4-(dimethylphenyl)), non-substituted 5 membered heteroaryl with one heteroatom such as thiophen-2-yl or thiophen-3-yl;
- - - - - - is a single bond or a double bond, preferably a single bond, n is 2; and **X⁻** is tetrafluoroborate or hexafluorophosphate, preferably tetrafluoroborate.

*In a second embodiment of this particular first variant,* the compounds of **Formula (II)** are those wherein **A** is N; **D** is N and are represented by **Formula (IV):** 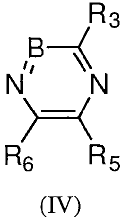 wherein
**B is** C-R₂ or N;
**R₂** is H or optionally substituted aryl; preferably H or phenyl;
**R₃** is optionally substituted aryl or **Z;**
R₅ is H;
**R₆** is **Z;**
or **R₅** and **R₆** may form together an optionally substituted heteroaromatic ring, such as a thiophene, a pyridinium or an optionally substituted aromatic hydrocarbon ring, such as an arylene, for example a benzene fused to the central nitrogen-containing six-membered heterocyclic ring they are attached to, said heteroaromatic ring or aromatic hydrocarbon ring being for example substituted by at least **Y** or for instance by two **Z;**
and **Y, Z, n** and **X** are as defined above.

According to this embodiment, **R₅** and **R₆** may form together an optionally substituted aromatic hydrocarbon ring, such as an aryl, for example a benzene, or an optionally substituted heteroaromatic ring, such as a thiophene, a selenophene, fused to the central nitrogen-containing six-membered heterocyclic ring they are attached to, said aromatic hydrocarbon ring or heteroaromatic ring being optionally substituted for instance by at least one **Y,** by one **Z** or by two **Z,** as it can be represented for example as follows: 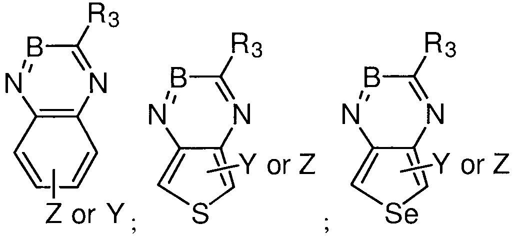 wherein **B, R₃, Z** and **Y** as defined above.

In a preferred embodiment, **R₅** and **R₆** form together a thiophene fused to the central nitrogen-containing six-membered heterocyclic ring they are attached to, said thiophene ring being substituted by one **Z** or by two **Z,** even more preferably, said thiophene ring being substituted by two N-benzylpyridiniumyl groups.

According to this particular embodiment, compounds can be, therefore, those of **formula (V)** 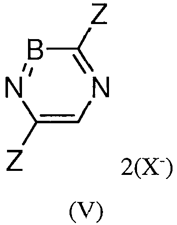 wherein
**B** is CH or N; preferably CH
and **Z, Y** and **X** are as defined above.

Preferred compounds of **formula (V)** can be those wherein **Y** is C₁-C₈ alkyl, optionally substituted aryl, -CH₂-aryl; preferably **Y** is methyl, *n*-hexyl, benzyl, phenyl or *p*-tolyl; **n** is 2; and **X⁻** is tetrafluoroborate or hexafluorophosphate, preferably hexafluorophosphate. More preferably Y is hexyl, 4-methylphenyl or benzyl.

Other preferred compounds according to this particular embodiment are typically those of **formula (VI)** or **(VII)** 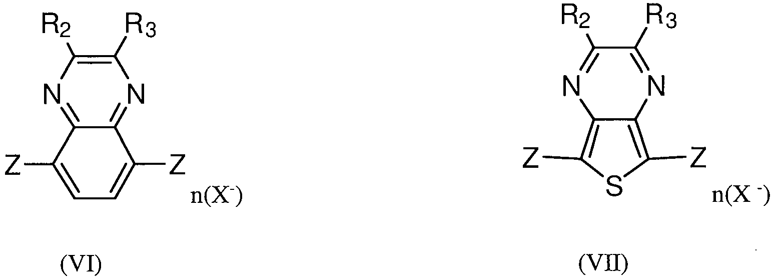 wherein
**R₂** is H or optionally substituted aryl;
**R₃** is H, optionally substituted aryl or **Z;**
And **Z, Y, n** and **X** are as defined above.

Preferred compounds of **formula (VI) and (VII)** are those wherein **R₂** is optionally substituted aryl (for example phenyl); **R₃** is also optionally substituted aryl (for example phenyl); **Y** is C₁-C₈ alkyl, optionally substituted aryl, -CH₂-aryl; preferably **Y** is methyl, *n-*hexyl, benzyl, phenyl or *p*-tolyl; **n** is 2; and **X⁻** is tetrafluoroborate or hexafluorophosphate, preferably hexafluorophosphate.

*In a third embodiment of this first variant,* the compounds of **Formula (II)** are those wherein **A** is ⁺N-R₁; **B** is C-R₂; and represented by **formula (III').** 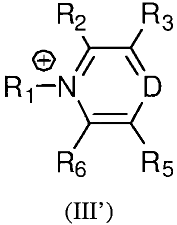 with
**R₁** is C₁-C₁₈ alkyl, optionally substituted aryl or -CH₂-aryl;
**D** is N or ⁺N-R₄;
**R₄** is C₁-C₁₈ alkyl, optionally substituted aryl or -CH₂-aryl;
**R₅** and **R₆** form together an optionally substituted heteroaromatic ring, such as a thiophene, a pyridinium or an optionally substituted aromatic hydrocarbon ring fused to the central nitrogen-containing six-membered heterocyclic ring they are attached to, for example substituted by at least one **Y;**
**R₂** and **R₃** form together an optionally substituted heteroaromatic ring, such as a thiophene, a pyridinium or an optionally substituted aromatic hydrocarbon ring fused to the central nitrogen-containing six-membered heterocyclic ring they are attached to, for example substituted by at least one **Y;**
and/ or **R₁** and **R₆** may form together an optionally substituted a nitrogen containing six-membered ring, fused to the central nitrogen-containing six-membered heterocyclic ring they are attached to;
and **Y, n and X** are as defined above.

According to this third embodiment, compounds can be for example pyrido[2,3-*b*] quinoxalines, pyrido[3,4-*b*] quinoxalines, pyrido[2,3-*b*] thieno [3,4-*e*] pyrazines or pyrido[3,4-*b*] thieno [3,4-*e*] pyrazines.

Thus, according to this third embodiment, compounds of the present invention can be compounds of **formula (III')** wherein **R₂** and **R₃** form together an optionally substituted aromatic hydrocarbon ring fused to the central nitrogen-containing six-membered heterocyclic ring they are attached to, such a six-membered ring, for example a benzene, for example substituted by at least one **Y; D** is N; **R₅** and **R₆** form together an optionally substituted heteroaromatic ring, such a pyridinium, fused to the central nitrogen-containing six-membered heterocyclic ring they are attached to, for example substituted by at least one **Y.** These compounds can be represented as follows: or 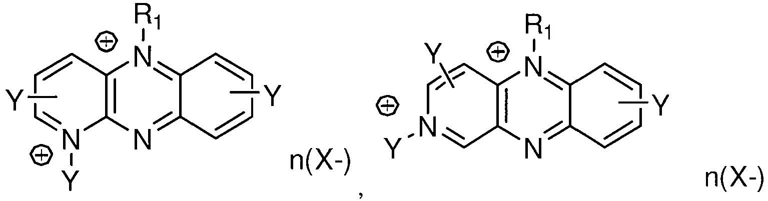 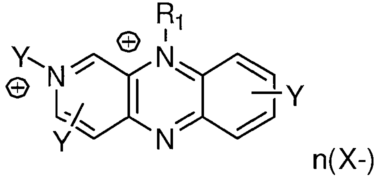 with **R₁, Y,** X and **n** as defined above.

Still according to this third embodiment, compounds of the present invention can be compounds of **formula (III')** wherein **R₂** and **R₃** form together an optionally substituted aromatic hydrocarbon ring fused to the central nitrogen-containing six-membered heterocyclic ring they are attached to, such a six-membered ring, for example a benzene, for example substituted by at least one **Y; D** is N; **R₅** and **R₆** form together an optionally substituted heteroaromatic ring, such a pyridinium, fused to the central nitrogen-containing six-membered heterocyclic ring they are attached to, for example substituted by at least one **Y;** and **R₁** and **R₆** form together an optionally substituted nitrogen containing six-membered ring, fused to the central nitrogen-containing six-membered heterocyclic ring they are attached to. These compounds can be represented as follows: 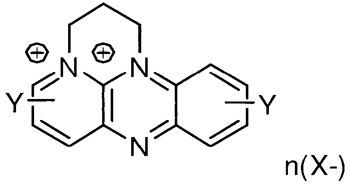 with **R₁, Y, X** and **n** as defined above.

Compounds of the present invention can also be compounds of **formula (III')** wherein **R₂** and **R₃** form together an optionally substituted heteroaromatic ring, a five-membered ring; a sulfur containing aromatic ring such as a thiophene, fused to the central nitrogen-containing six-membered heterocyclic ring they are attached to, for example substituted by at least one **Y; D** is N; **R₅** and **R₆** form together an optionally substituted heteroaromatic ring, such a pyridinium, fused to the central nitrogen-containing six-membered heterocyclic ring they are attached to, for example substituted by at least one **Y.** These compounds can be represented as follows: 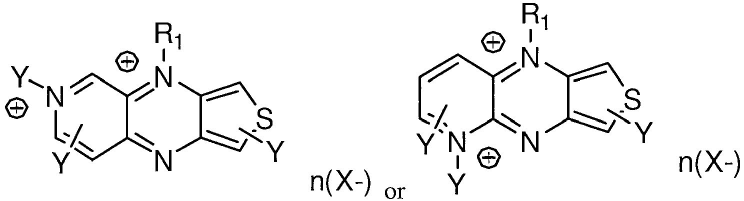 with **R₁, Y, X** and **n** as defined above.

Compounds of the present invention can also be compounds of **formula (III')** wherein **R₂** and **R₃** form together an optionally substituted heteroaromatic ring, a five-membered ring; a sulfur containing aromatic ring such as a thiophene, fused to the central nitrogen-containing six-membered heterocyclic ring they are attached to, for example substituted by at least one **Y; D** is N; **R₅** and **R₆** form together an optionally substituted heteroaromatic ring, such a pyridinium, fused to the central nitrogen-containing six-membered heterocyclic ring they are attached to, for example substituted by at least one **Y** and **R₁** and **R₆** form together an optionally substituted nitrogen containing six-membered ring, fused to the central nitrogen-containing six-membered heterocyclic ring they are attached to. These compounds can be represented as follows: 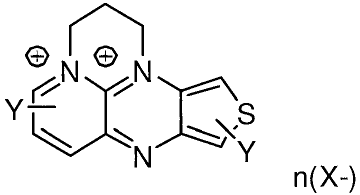 with **R₁, Y, X** and **n** as defined above.

Compounds of the present invention can also be compounds of **formula (III')** wherein **R₂** and **R₃** form together an optionally substituted heteroaromatic ring, a five-membered ring; a sulfur containing aromatic ring such as a thiophene, fused to the central nitrogen-containing six-membered heterocyclic ring they are attached to, for example substituted by at least one **Y;** D is ⁺N-R₄; **R₅** and **R₆** form together an optionally substituted heteroaromatic ring, such a pyridinium, fused to the central nitrogen-containing six-membered heterocyclic ring they are attached to, for example substituted by at least one **Y.** These compounds can be represented as follows: 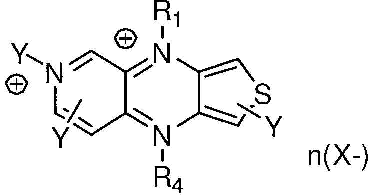 with **R₁, R₄, Y, X** and **n** as defined above.

**In a second variant of the present invention,** the compounds of the invention are those with a central nitrogen-containing seven-membered heterocyclic ring and of **Formula (IX)** 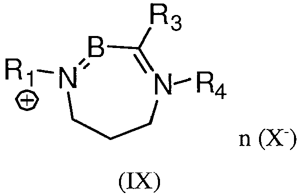 wherein
**B** is C-R₂;
**R₃** and **R₄** form together an optionally substituted nitrogen-containing aromatic ring, such as a pyridinium or a benzimidazolium, fused to the central nitrogen-containing seven-membered heterocyclic ring they are attached to, said aromatic ring being for example substituted by at least one **Y,** preferably said aromatic ring is not substituted;
**R₁** and **R₂** form together an optionally substituted nitrogen-containing aromatic ring, such as a pyridinium, a quinolinium, or a benzimidazolium fused to the central nitrogen-containing seven-membered heterocyclic ring they are attached to, for example substituted by an optionally substituted aryl;
and **R₂** and **R₃** may form together an optionally substituted nitrogen-containing non-aromatic ring, such as seven-membered ring, a saturated diazepine, fused to the central nitrogen-containing seven-membered heterocyclic ring they are attached to, said non-aromatic ring being preferably not substituted;
and **Y, n** and **X** are as defined above.

In a particularly preferred embodiment of the present invention, the compound of formula (I) is selected from the group consisting of:

| | |
|---|---|
| Compound 1 | 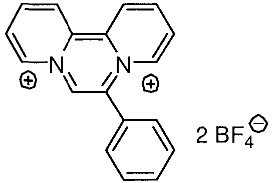 |
| Compound 2 | 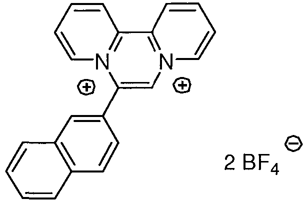 |
| Compound 3 | 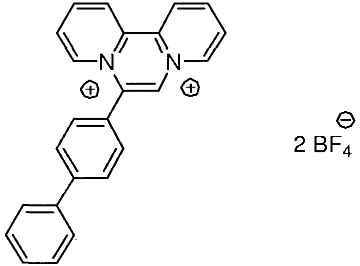 |
| Compound 4 | 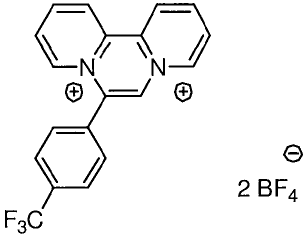 |
| Compound 5 | 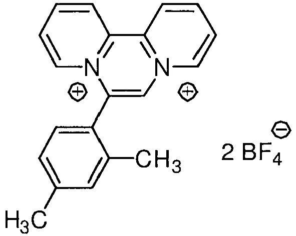 |
| Compound 6 | 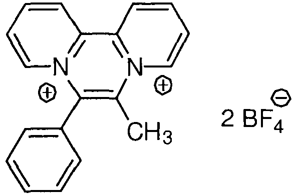 |
| Compound 7 | 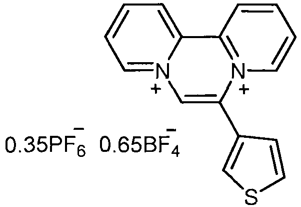 |
| Compound 8 | 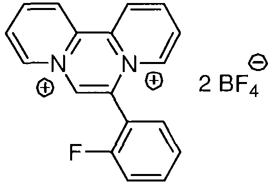 |
| Compound 9 | 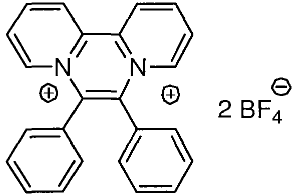 |
| Compound 10 | 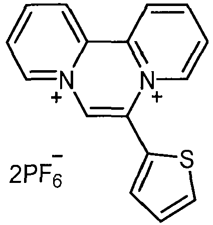 |
| Compound 11 | 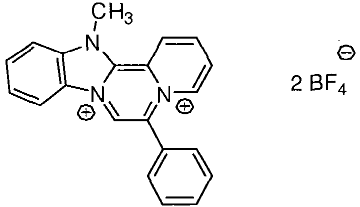 |
| Compound 12 | 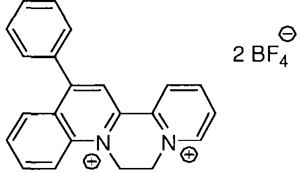 |
| Compound 13 | 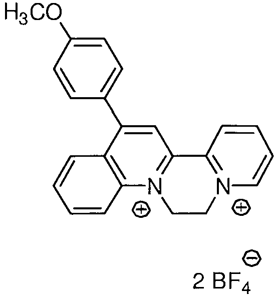 |
| Compound 14 | 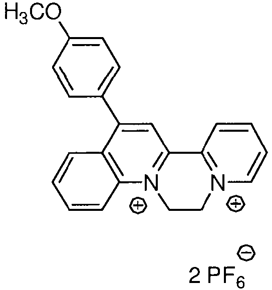 |
| Compound 15 | 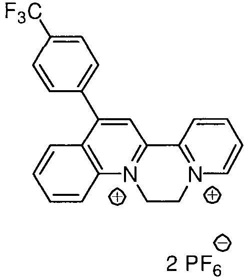 |
| Compound 16 | 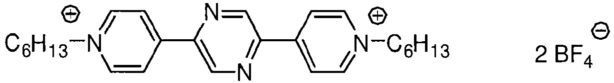 |
| Compound 17 | 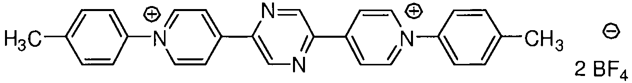 |
| Compound 18 | 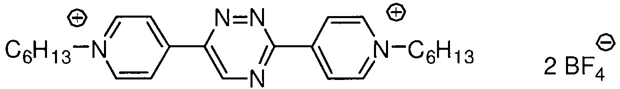 |
| Compound 19 | 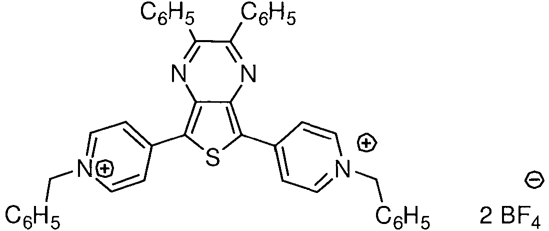 |
| Compound 20 | 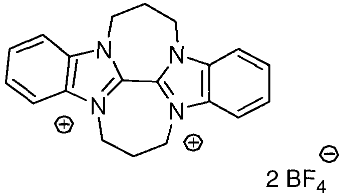 |
| Compound 21 | 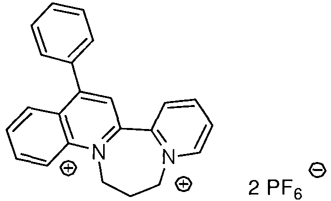 |
| Compound 22 | 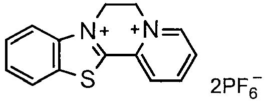 |

Compounds represented by formula (I) may be prepared according to various methods well known in the art.

For example, compounds represented by formula (I) may be obtained according to the synthetic routes detailed hereinafter.

2,5-Dibromopyrazine (R. C. Ellingson and R. L. Henry, J. Am. Chem. Soc., 1949, 71, 2798; A.-M. Stadler, F. Funtoriero, F. Nastasi, S. Campagna and J.-M. Lehn, Chem. Eur. J., 2010, 16, 5645) was subjected to Suzuki-Miyaura coupling with 4-(4,4,5,5-tetramethyl-1,3,2-dioxaborolan-2-yl)pyridine to afford 2,5-bis(4-pyridyl)pyrazine (PCT WO2016/14753) in good yield. Subsequent conversion to the bis-pyridinium derivatives was accomplished either by alkylation with an iodoalkane or by conversion to the N,N-bis(2,4-dinitrophenyl) salt and subsequent displacement with an aromatic amine (Scheme 1).

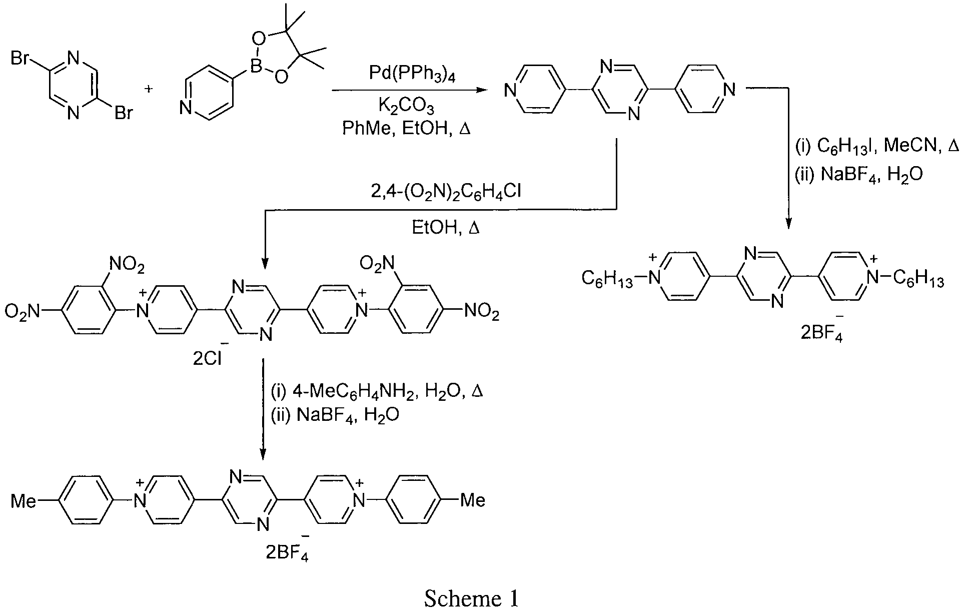

Synthetic routes to and the chemistry of 1,2,4-triazines have been reviewed (S.A. Raw and R. J. K. Taylor, Adv. Heterocycl. Chem., 2010, 100, 75). Compounds such as 3,6-diaryl-1,2,4-triazines are often prepared by the condensation of amidrazones with 1,2-dicarbonyl compounds and many variants of this procedure have been developed. Although 2-pyridyl-linked 1,2,4-triazines (J. Sauer, D. K. Heldmann and G. R. Pabst, Eur. J. Org. Chem., 1999, 313) have been described and reactions of the 3,6-bis(2-pyridyl)- and 3,6-bis(4-pyridyl)-1,2,4-triazines have also been reported (M. Takahashi, Y. Hikita and M. Fukui, Heterocycles, 1989, 29, 1379) the synthesis of the latter has not been reported previously. The procedure used in the present work is based upon that for the synthesis of 6-aryl-3-pyridyl-1,2,4-triazines from oximinohydrazones (V. N. Kozhevnikov, O. V. Shabunina, D. S. Kopchuk, M. M. Ustinove, B. König and D. N. Kozhennikov, Tetrahedron, 2008, 64, 8963). Thus, 4-acetylpyridine was converted into 2-hydrazinylidene-2-(4-pyridyl)acetaldehyde oxime following the literature procedure (V. N. Kozhevnikov, D. N. Kozhennikov, O. V. Shabunina, V. L. Rusinov and O. N. Chupakhin, Tetrahedron Lett., 2005, 46, 1791) and condensed with pyridine-4-carboxaldehyde to give 3,6-bis(4-pyridyl)-1,2,4-triazine in good yield. Subsequent N-alkylation proceeds straightforwardly (Scheme 2).

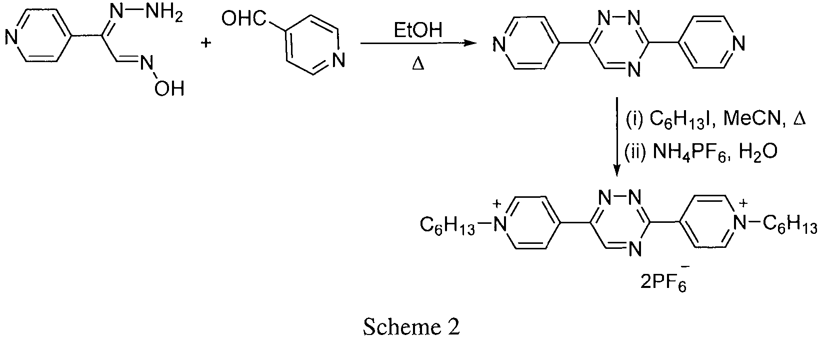

6-Substituted- and 6,7-disubstituted dipyrido[1,2-*a*:2',1'-*c*]pyrazine-5,8-diiums are accessible from the alkylation of 2,2'-bipyridine with an appropriate α-halo ketone followed by cyclodehydration of the resulting bipyridinium salt with phosphorus tribromide according to the method described in the literature (D. H. Corr and E. E. Glover, J. Chem. Soc., 1965, 5816; I. C. Calder and W. H. F. Sasse, Aust. J. Chem., 1968, 21, 2951; A. L. Black and L. A. Summers, J. Heterocycl. Chem., 1971, 8, 29). The resulting dibromides though stable as such, could be conveniently used to generate a variety of salts by anion exchange. The sequence is shown in scheme 3.

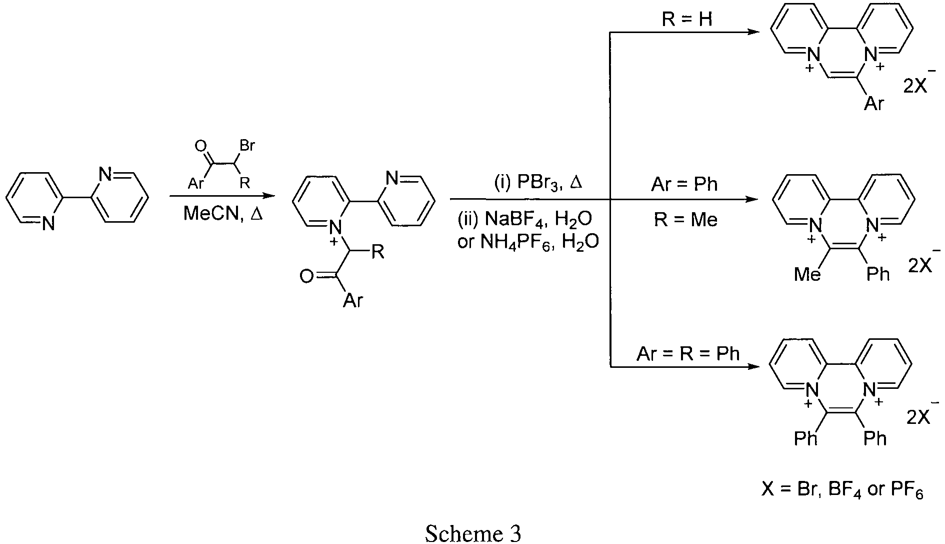

### Electrochromic composition

The present invention also relates to electrochromic compositions comprising at least one compound of formula (I) as defined above as an oxidizing electrochromic compound and a host medium. One or more additional oxidizing electrochromic compounds can be added to the composition of the invention so as to adapt the colour or the intensity of the coloured state of the composition. Said additional compound can be another compound of formula (I) or a different compound such as compatible dyes or pigments. For example, the additional oxidizing electrochromic compound can be selected from alkylviologens, arylviologens, alkylarylviologens anthraquinone, benzazoles, imidazo[1,2-α]pyridines, 2,1,3-benzothiadiazoles, imidazoles, benzoselenadiazoles, benzoselenazoles and derivatives. Preferably, the additional compound has a redox potential close to the compound of formula (I).

The composition may also comprise at least one reducing compound. The reducing compound may also be an electrochromic compound. Example of reducing compounds include 5,10-dihydrophenazine, phenothiazine, phenoxazine, N,N,N',N'-tetramethyl-p-phenylenediamine, thioanthrene, tetrathiafulvalene, ferrocene and their derivatives.

The composition of the invention may comprise a host medium that may be a fluid, a mesomorphous medium or a gel. The host medium is introduced in the composition of the invention to dissolve the electrochromic compounds. The host medium is preferably selected from the group consisting of organic solvents, liquid crystals, polymers, liquid crystal polymers and mixtures thereof.

Examples of suitable organic solvents that can be used as host medium are redox-compatible solvents which cannot react with the electrochromic compounds of the composition, such as ethylene carbonate, propylene carbonate, γ-butyrolactone, γ-valerolactone, acetronitrile, propionitrile, benzonitrile, glutaronitrile, methylglutaronitrile, dimethylformamide, N-methylpyrrolidone, sulfolane, 3-methylsulfolane, benzene, toluene, methyl ethyl ketone, acetone, ethanol, tetrahydrofurfuryl alcohol, 2-methoxyethyl ether, xylene, cyclohexane, 3-methylcyclohexanone, ethyl acetate, ethyl phenylacetate, tetrahydrofuran, methanol, methyl propionate, ethylene glycol, ethylene carbonate, ionic liquids, and mixtures thereof. Preference is given to carbonates and particularly propylene carbonate.

Examples of suitable liquid crystals that can be used as host medium are nematic or chiral nematic media.

Examples of suitable polymers that can be used as host medium are polymers which are soluble with the solvent, in particular PMMA or other acrylate polymers, polyurethane, polyethylene oxide, polypropylene oxide, polyvinyl acetate, poly(N-vinyl pyrrolidone), and polyvinylidene fluoride.

Examples of suitable liquid crystal polymers that may be used as host medium are Merck RM257 (Merck), LC242 (BASF) or SLM 90519 (Wacker). These liquid crystal polymers are generally used in combination with an organic solvent, for example one of the organic solvents mentioned above.

### Electrochromic device

The present invention also relates to an electrochromic device comprising a compound of formula (I) or a composition according to the invention. Said device may be selected from an optical article; preferably an optical lens, or an optical filter; a window, preferably an aircraft or an automotive window; a visor, a mirror, a variable transmission device and a display element or device, in particular a segmented or matrix display. Preferably, the device of the invention is an optical article, more preferably an optical lens, and even more preferably an ophthalmic lens.

Non-limiting examples of ophthalmic lens include corrective and non-corrective lenses, including single vision or multi-vision lenses, which may be either segmented or non-segmented, as well as other optical articles used to correct, protect, or enhance vision, including without limitation contact lenses, intra-ocular lenses, sunglasses, ski goggles, magnifying lenses, filters, protective lenses and visors, for example motorcycle visors and helmets. Non-limiting examples of display elements and devices include screens and monitors. Non-limiting examples of windows include automotive, marine, aircraft or building windows. Variable transmission devices include any optical article without limitation (contact lenses, intra-ocular lenses, sunglasses, ski goggles, magnifying lenses, filters, protective lenses and visors, for example motorcycle visors and helmets) which can quickly change between a high-transmission (or "clear") state and a low-transmission (or "dark") state. An optical switch, as used herein, is a variable transmission device, having thus a high-transmission (or "clear") state and a low-transmission (or "dark") state, which is used to control the transmission state of a larger device.

Preferably, the device of the invention comprises a mechanism for holding the compound or composition of the invention in a mechanically stable environment. More preferably, said device may comprise a pair of opposed substrates having a gap there between for receiving the mixture of the host medium and said compound or said composition of the present invention, and a frame for holding said pair of substrates adjacent one another.

The device of the present invention may thus comprise an optical component provided with at least one transparent cell arrangement juxtaposed in a parallel direction to the surface thereof, as disclosed in WO 2006/013250, each cell being tightly closed and containing at least one compound or composition of the present invention.

Other devices according to the invention can be devices as described in FR 2937154 or FR2950710 comprising at least one compound or composition of the invention.

This invention will be further illustrated by the following non-limiting examples which are given for illustrative purposes only and should not restrict the scope of the appended claims.

### Synthesis of compounds of the invention

### 6-Phenyldipyrido[1,2-a:2',1'-c]pyrazine-5,8-diium bis(tetrafluoroborate) Compound 1

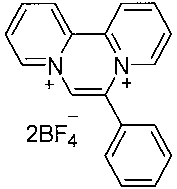

6-Phenyldipyrido[1,2-*a*:2',1'-*c*]pyrazine-5,8-diium dibromide (1.5 g, 3.58 mmol) (prepared according to D. H. Corr and E. E. Glover, J. Chem. Soc., 1965, 5816) was dissolved in the minimum volume of water and added dropwise with stirring to a solution of NaBF₄ (2.36 g, 21.5 mmol) in water (70 mL) to give a dark solution. After 2 h additional NaBF₄ (1.00 g, 9.1 mmol) was added and stirring was continued until precipitation of the product was complete (ca. 4 h). The product was isolated by filtration and washed with the filtrate, a small volume of water and then with diethyl ether and air dried. The *title compound* (0.80g, 52%) was obtained as a purple microcrystalline powder; δ_{H} [(CD₃)₂CO, 400 MHz] 7.78 - 7.90 (3H, m), 7.93 - 7.99 (2H, m), 8.81 (1H, dt, *J =* 1.3 and 6.8 Hz), 8.96 (1H, dt, *J* = 1.2 and 6.4 Hz), 9.32 - 9.40 (2H, m), 9.50 (1H, s), 9.64 (1H, dd, *J* = 0.9 and 6.6 Hz), 9.89 (1H, bd, *J* = 6.6 Hz) and 10.04 - 10.14 (2H, m); δ_{F} [(CD₃)₂CO, 376 MHz] -151.69, -151.64; δ_{C} (DMSO-d₆, 100 MHz) 126.34, 126.62, 126.75, 127.10, 130.69, 130.98, 131.05, 131.23, 132.99, 135.81, 137.56, 138.65, 139.96, 142.00, 146.91 and 146.9

### 1-[2-(Naphthalen-2-yl)-2-oxoethyl]-[2,2'-bipyridin]-1-ium bromide

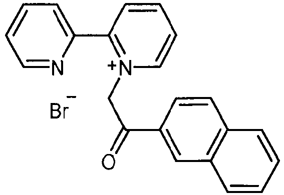

A mixture of 2,2'-bipyridine (2 g, 12.8 mmol) and 2-(bromoacetyl)naphthalene (3.30 g, 13.3 mmol) was heated at 100 °C for 45 min. The resulting solid was cooled and crystallised from EtOH/Et₂O twice and air dried to give the *title compound* (4.27 g, 82 %) as a tan powder; δ_{H} (D₂O, 400 MHz) 6.62 (2H, s), 7.48 - 7.54 (1H, m), 7.62 - 7.70 (1H, m), 7.70 - 7.78 (1H, m), 7.96 - 8.17 (6H, m), 8.24 - 8.39 (2H, m), 8.51 (1H, d, *J =* 8 Hz), 8.66 (1H, s), 8.88 (1h, app. t, *J* = 7.8 Hz) and 9.16 (1H, app. d, *J* = 6 Hz); δ_{C} (D₂O, 100 MHz) 121.63, 124.70, 125.14, 125.78, 125.80, 126.36, 127.49, 127.84, 128.15, 128.59, 128.93, 129.56, 131.29, 134.95, 137.16, 145.67, 147.73, 148.20, 151.08 and 188.32.

### 6-(Naphthalen-2-yl)dipyrido[1,2-a:2',1'-c]pyrazine-5,8-diium dibromide

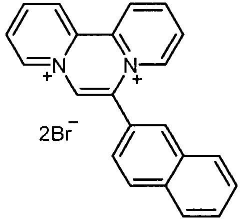

A mixture of 1-[2-(naphthalen-2-yl)-2-oxoethyl]-[2,2'-bipyridin]-1-ium bromide (3.04 g, 7.5 mmol) in PBr₃ (15 mL) was heated at reflux for 15 min, cooled, and the residue filtered off and washed with Et₂O (5 × 50 mL). The residue was twice triturated with hot MeOH/diisopropyl ether and air dried to give the *title compound* (2.18 g, 62 %) as dark yellow powder; δ_{H} (D₂O, 400 MHz) 7.61 - 7.76 (3H, m), 8.01 - 8.08 (2H, m), 8.21 (1H, app. d, *J* = 8.6 Hz), 8.33 (1H, app. s), 8.47 (1H, app t. *J* = 7.3 Hz), 8.65 (1H, app. t, *J* = 6.9 Hz), 9.05 - 9.14 (2H, m), 9.24 (1H, s), 9.46 (1H, app. d, *J* = 6.6 Hz), 9.53 (1H, app. d, *J* = 6.2 Hz), 9.72 (1H, app. d, *J* = 8.7 Hz) and 9.77 (1H, app. d, *J* = 8.6 Hz); δ_{C} (O₂O, 100 MHz) 122.44, 125.13, 126.25, 126.45, 127.08, 128.04, 128.16, 128.74, 129.19, 130.41, 130.75, 130.93, 131.94, 132.85, 134.50, 136.66, 136.91, 137.92, 139.89, 141.81, 146.81 and 147.03.

### 6-(Naphthalen-2-yl)dipyrido[1,2-a:2',1'-c]pyrazine-5,8-diium bis(tetrafluoroborate) Compound 2

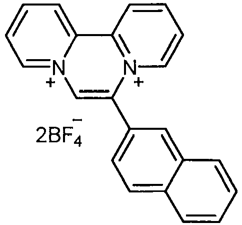

A solution of 6-(naphthalen-2-yl)dipyrido[1,2-*a*:2',1'-*c*]pyrazine-5,8-diium bromide (1.50 g, 3.2 mmol) in water (40 mL) was added dropwise to a solution of NaBF₄ (3.53 g, 21.1 mmol) in water (100 mL) with stirring. Stirring was continued for 0.5 h and the resulting precipitate filtered, washed with water (10 mL) and air dried. The resulting powder was extracted with acetone (6 × 100 mL) and the solvent reduced in volume at 40 °C (~100 mL). The residue was filtered off to give the *title compound* (0.80 g, 52 %) as an ochre powder; δ_{H} [(CD₃)₂CO, 400 MHz] 7.75 - 7.86 (2H, m), 7.97 (1H, dd, *J* = 1.8 and 8.5 Hz), 8.13 - 8.22 (2H, m), 8.35 (1H, app. d, *J =* 8.5 Hz), 8.59 (1H, bs), 8.81 (1H, app. dt. *J* = 1 and 7.7 Hz), 8.98 (1H, app. dt, *J* = 1.0 and 7.7 Hz), 9.34 - 9.43 (2H, m), 9.59 (1H, s), 9.77 (1H, app. d, *J* = 6 Hz), 9.91 (1H, app. d, *J* = 6.2 Hz), 10.09 (1H, app. d, *J* = 8.6 Hz) and 10.14 (1H, app. d, *J* = 8.4 Hz); δ_{F} [(CD₃)₂CO, 376 MHz] -152.16, 152.11; δ_{C} [(CD₃)₂CO, 100 MHz] 123.65, 124.46, 125.83, 126.54, 127.00, 127.33, 127.88, 128.21, 128.88, 128.98, 130.21, 130.96, 132.28, 133.23, 134.80, 136.72, 137.93, 138.80, 140.12, 142.19, 146.71 and 146.81.

### 1-{2-([1,1'-Biphenyl]-4-yl)-2-oxoethyl}-[2,2'-bipyridin]-1-ium bromide

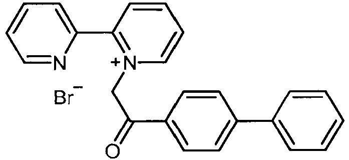

A mixture of 2,2'-bipyridine (2 g, 12.8 mmol) and 1-([1,1'-biphenyl]-4-yl)-2-bromoethan-1-one (3.65 g, 13.3 mmol) was heated at 100 °C for 45 min. The resulting solid was cooled and crystallised from EtOH/Et₂O twice and air dried to give the *title compound* (4.64 g, 84 %) as a light tan powder; δ_{H} (CD₃OD, 400 MHz) 6.51 (2H, s), 7.41 - 7.63 (4H, m), 7.72 - 7.79 (2H, m), 7.88 (2H, d, 8.3 Hz), 8.04 - 8.17 (4H, m), 8.33 (1H, app. t, *J =* 6.7 Hz), 8.40 (1H, app. d, *J = 4.5* Hz), 8.50 (1H, app. d, *J =* 8 Hz), 8.87 (1H, app. dt, *J =* 0.7 and 8 Hz) and 9.14 (1H, app. d, *J* = 6 Hz); δ_{C} (CD₃OD, 100 MHz) 63.34, 124.74, 125.16, 125.63, 125.79, 125.97, 127.09, 127.39, 127.55, 128.59, 130.93, 137.18, 137.98, 145.66, 145.79, 147.75, 147.77, 148.22 and 187.96.

### 6-([1,1'-Biphenyl]-4-yl)dipyrido[1,2-a:2',1'-c]pyrazine-5,8-diium dibromide

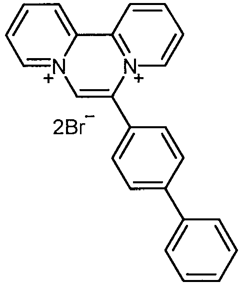

A mixture of 1-{2-([1,1'-biphenyl]-4-yl)-2-oxoethyl}-[2,2'-bipyridin]-1-ium bromide (3.23 g, 7.5 mmol) in PBr₃ (15 mL) was heated at reflux for 15 min, after cooling, the residue was filtered off, washed with Et₂O (3 × 50 mL) and crystallised from MeOH. The liquors were diluted with diisopropyl ether and filtered. The combined solids were recrystallised from hot MeOH washed with Et₂O (20 mL) and dried under vacuum to give the *title compound* (1.57 g, 42 %) as a green/yellow powder; δ_{H} (D₂O, 400 MHz) 7.37 - 7.44 (1H, m), 7.45 - 7.51 (2H, m), 7.70 - 7.79 (4H, m), 7.96 (2H, d, *J* = 8.2 Hz), 8.49 (1H, app.t, *J* = 7.1 Hz), 8.61 (1H, app. d, *J* = 6.9 Hz), 9.03 - 9.10 (2H, m), 9.16 (1H, s), 9.44 - 9.51 (2H, m), 9.68 (1H, d, *J* = 8.7 Hz) and 9.73 (1H, d, *J* = 8.4 Hz); δ_{C} (D₂O, 100 MHz) 124.02, 126.05, 126.46, 127.12, 127.27, 128.66, 128.83, 129.33, 130.80, 130.94, 131.00, 136.47, 136.86, 137.88, 138.95, 139.81, 141.77, 145.01, 146.81 and 147.03.

### 6-([1,1'-Biphenyl]-4-yl)dipyrido[1,2-a:2',1'-c]pyrazine-5,8-diium bis(tetrafluoroborate) Compound 3

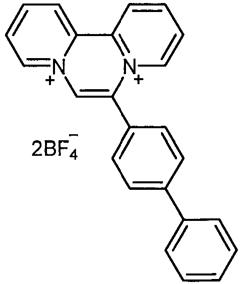

A solution of 6-([1,1'-biphenyl]-4-yl)dipyrido[1,2-*a*:2',1'-*c*]pyrazine-5,8-diium dibromide (1.20 g, 2.4 mmol) in water (40 mL) was added dropwise to a solution of NaBF₄ (1.60 g, 14.5 mmol) in water (40 mL) with stirring. Stirring was continued for 0.5 h and the resulting precipitate filtered, washed with water (2 × 5 mL), then with MeOH (2 mL) and air dried to give the *title compound* (1.14 g, 93 %) as an orange powder; δ_{H} (D₂O/CD₃OD, 400 MHz) 7.50 - 7.67 (3H, m), 7.83 - 7.90 (2H, m), 7.95 (2H, d, *J* = 8.1 Hz), 8.13 (2H, d, *J* = 8.1 Hz), 8.68 (1H, app. t, *J* = 7.1 Hz), 8.79 (1H, app. t, *J* = 6.9 Hz), 9.21 - 9.29 (2H, m), 9.34 (1H, s), 9.58 (1H, app. d, *J* = 6.5 Hz), 9.69 (1H, app. d, *J* = 6.2 Hz), 9.86 (1H, d, *J* = 8.6 Hz) and 9.92 (1H, d, *J* = 8.7 Hz); δ_{F} (D₂O/CD₃OD, 376 MHz) -150.38, -150.32; δ_{C} (D₂O/CD₃OD, 100 MHz) 125.51, 127.49, 127.81, 128.52, 130.09, 130.20, 130.63, 132.26, 132.35, 132.44, 138.04, 138.49, 139.53, 140.33, 141.06, 143.27, 146.90, 148.24 and 148.51.

### 1-{2-Oxo-2-[4-(trifluoromethyl)phenyl]ethyl}-[2,2'-bipyridin]-1-ium bromide

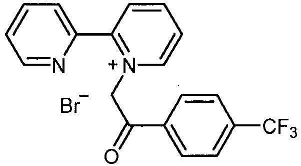

A mixture of 2,2'-bipyridine (2 g, 12.8 mmol) and 2-bromo-1-(4-trifluoromethylphenyl)ethan-1-one (3.54 g, 13.3 mmol) was heated at 100 °C for 45 min. The resulting solid was cooled and crystallised from EtOH/Et₂O twice and air dried to give the *title compound* (4.08 g, 75 %) as a yellow powder which was used without further purification in the next step.

### 6-[4-(Trifluoromethyl)phenyl]dipyrido[1,2-a:2',1'-c]pyrazine-5,8-diium dibromide

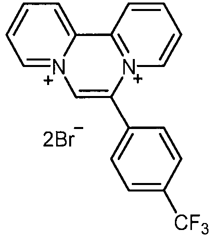

A mixture of 1-{2-oxo-2-[4-(trifluoromethyl)phenyl]ethyl}-[2,2'-bipyridin]-1-ium bromide (3.17 g, 7.5 mmol) in PBr₃ (15 mL) was heated at reflux for 15 min then cooled. The residue was filtered off, washed with Et₂O (3 × 50 mL) and crystallised from MeOH/diisopropyl ether. The residue was triturated with hot EtOH, cooled, filtered, triturated again with isopropanol (5 mL), filtered off and air dried to give the *title compound* (1.26 g, 35 %) as a yellow powder; δ_{H} (D₂O, 400 MHz) 7.90 (2H, bd, *J* = 8.1 Hz), 8.05 (2H, bd, *J* = 8.1 Hz), 8.51 (1H, bt, *J* = 6.6 Hz), 8.66 (1H, bt, *J* = 6.9 Hz), 9.07 - 9.15 (2H, m), 9.23 (1H, s), 9.34 (1H, bd, *J* = 6.5 Hz), 9.53 (1H, bd, *J* = 6.2 Hz), 9.72 (1H, bd, *J* = 8.6 Hz) and 9.77 (1H, bd, *J* = 8.4 Hz); δ_{F} (D₂O, 376 MHz) -63.13; δ_{C} (D₂O, 100 MHz) 123.46 (q, *J* = 272 Hz), 126.55, 126.57, 127.22, 127.37 (q, *J* = 3.8 Hz), 128.82 (q, *J* = 1.4 Hz), 130.89, 131.03, 131.36, 133.94 (q, *J* = 32.9 Hz), 135.22, 136.99, 137.86, 139.85, 142.09, 147.23 and 147.25.

### 6-[4-(Trifluoromethyl)phenyl]dipyrido[1,2-a:2',1'-c]pyrazine-5,8-diium bis(tetrafluoroborate) Compound 4

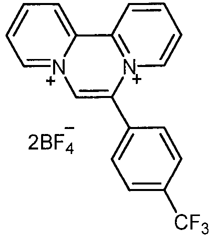

A solution of 6-[4-(trifluoromethyl)phenylldipyrido[1,2-*a*:2',1'-*c*]pyrazine-5,8-diium dibromide (1.20 g, 2.5 mmol) in water (40 mL) was added dropwise to a solution of NaBF₄ (3.26 g, 29.6 mmol) in water (40 mL) with stirring. Stirring was continued for 0.5 h and the resulting precipitate filtered, washed with water (2 × 5 mL) and air dried to give the *title compound* (0.96 g, 78 %) as a purple powder; δ_{H} (D₂O/ CD₃OD, 400 MHz) 7.99 (2H, bd, *J* = 8.3 Hz), 8.07 (2H, bd, *J* = 8.3 Hz), 8.55 (1H, bt, *J* = 6.9 Hz), 8.69 (1H, bt, *J* = 6.8 Hz), 9.11 - 9.19 (2H, m), 9.27 - 9.33 (2H, m), 9.59 (1H, bd, *J =* 6.2 Hz), 9.79 (1H, bd, *J* = 8.6 Hz) and 9.84 (1H, bd, *J* = 8.4 Hz); δ_{F} (D₂O/ CD₃OD, 376 MHz) -151.65 (q, *J* = 1.1 Hz), -151.60 (bs), - 62.85 (s). The ¹³C spectrum could not be obtained owing to low solubility.

### 1-[2-(2,4-Dimethylphenyl)-2-oxoethyl]-[2,2'-bipyridin]-1-ium bromide

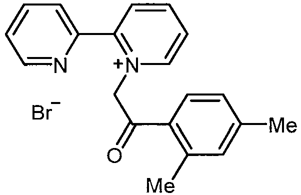

A mixture of 2,2'-bipyridine (2 g, 12.8 mmol) and 2-bromo-1-(2,4-dimethylphenyl)ethan-1-one (3.44 g, 15.1 mmol) was heated at 100 °C for 45 min. The resulting solid was cooled and crystallised from EtOH/Et₂O twice, filtered off and washed with Et₂O (30 mL) and air dried to give the *title compound* (4.24 g, 86 %) as a light tan powder; δ_{H} (CD₃OD, 400 MHz) 2.42 (3H, s), 2.48 (3H, s), 6.35 (2H, m), 7.19 - 7.27 (2H, m), 7.57 (1H, ddd, *J* = 1.0, 4.8 and 7.7 Hz), 7.73 (1H, d, *J* = 8 Hz), 8.06 (1H, app. d, *J* = 8.1 Hz), 8.13 (1H, dt, *J* = 1.7 and 7.9 Hz), 8.32 (1H, dt, *J* = 1.4 and 6.4 Hz), 8.38 - 8.41 (1H, m), 8.48 (1H, dd, *J* = 1.0 and 8.0 Hz), 8.86 (1H, dt, *J* = 1.4 and 8.1 Hz) and 9.12 (1H, dd, *J* = 0.8 and 6.2 Hz); δ_{C} (CD₃OD, 100 MHz) 18.88, 18.93, 124.68, 125.19, 125.26, 125.73, 127.61, 128.56, 129.11, 131.63, 137.17, 138.83, 142.81, 145.54, 147.62, 148.34, 150.99 and 189.57.

### 6-(2,4-Dimethylphenyl)dipyrido[1,2-a:2',1'-c]pyrazine-5,8-diium dibromide

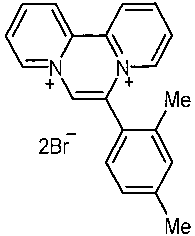

A mixture of 1-[2-(2,4-dimethylphenyl)-2-oxoethyl]-[2,2'-bipyridin]-1-ium bromide (3.11 g, 6.4 mmol) in PBr₃ (15 mL) was heated at reflux for 15 min, cooled, decanted, and the residue triturated with Et₂O (3 × 50 mL)and twice precipitated from EtOH using diisopropyl ether. The resulting solid was crystallised from hot MeOH/ diisopropyl ether, filtered, washed with Et₂O (2 × 30mL) and dried under vacuum to give the *title compound* (2.65 g, 74 %) as a yellow powder; δ_{H} (D₂O, 400 MHz) 2.08 (3H, s), 2.39 (3H, s), 7.28 - 7.41 (3H, m), 8.48 (1H, app. t, *J* = 7.3 Hz), 8.63 (1H, app. t, *J* = 6.9 Hz), 9.04 - 9.16 (4H, m), 9.49 (1H, app. d, *J* = 6.3 Hz), 9.70 (1H, app. d, *J* = 8.7 Hz) and 9.75 (1H, app. d, *J* = 8.5 Hz); δ_{C} (D₂O, 100 MHz) 18.37, 20.65, 121.31, 126.35, 126.63, 127.22, 128.20, 130.84, 131.02, 131.15, 132.20, 136.19, 137.17, 138.00, 138.90, 139.42, 141.75, 144.27, 146.85 and 146.91.

### 6-(2,4-Dimethylphenyl)dipyrido[1,2-a:2',1'-c]pyrazine-5,8-diium bis(tetrafluoroborate) Compound 5

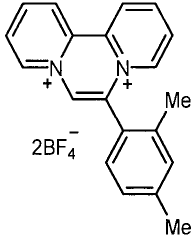

A solution of 6-(2,4-dimethylphenyl)dipyrido[1,2-*a*:2',1'-*c*]pyrazine-5,8-diium bromide (2.60 g, 5.8 mmol) in water (40 mL) was added dropwise to a solution of NaBF₄ (7.69 g, 69.9 mmol) in water (40 mL) with stirring. Stirring was continued for 0.5 h and the resulting precipitate filtered, washed with water (2 × 5 mL) and air dried to give the *title compound* (2.05 g, 76 %) as a pale tan powder; δ_{H} (D₂O/CD₃OD, 400 MHz) 2.14 (3H, s), 2.42 (3H, s), 7.32 - 7.46 (3H, m), 8.53 (1H, app. t, *J* = 6.9 Hz), 8.67 (1H, app. t, *J* = 7.0 Hz), 9.03 - 9.18 (4H, m), 9.55 (1H, app. d, *J* = 6.2 Hz), 9.75 (1H, app. d, *J* = 8.7 Hz) and 9.81 (1H, app. d, *J* = 8.5 Hz); δ_{C} (D₂O/CD₃OD, 376 MHz) -150.97, -150.91; δ_{C} (D₂O/CD₃OD, 100 MHz) 19.56, 21.87, 122.98, 127.70, 128.09, 128.64, 129.74, 132.17, 132.50, 132.52, 133.74, 137.73, 138.87, 139.74, 140.37, 140.48, 143.23, 145.66, 148.19 and 148.31.

### 1-(1-Oxo-1-phenylpropan-2-yl)-[2,2'-bipyridin]-1-ium bromide

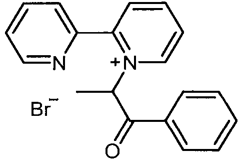

A solution of 2,2'-bipyridine (8 g, 41.3 mmol) and 2-bromo-1-phenylpropan-1-one (8 g, 37.7 mmol) in MeCN (50 mL) was heated at reflux for 7 days. The resulting solution was cooled, the solvent removed under reduced pressure and the residue washed with acetone. The resulting solid was crystallised from EtOH/Et₂O and dried under vacuum to give the *title compound* (2.13 g, 15 %) as a colourless powder; δ_{H} (CDCl₃, 400 MHz) 2.15 (3H, d, *J* = 7.2 Hz), 6.92 (1H, q, *J* = 7.2 Hz), 7.33 - 7.38 (1H, m), 7.47 - 7.56 (2H, m), 7.66 (1H, app. t, *J* = 7.5 Hz), 7.81 - 8.89 (2H, app. d, *J* = 7.7 Hz), 7.95 - 8.04 (2H, m), 8.20 (1H, app. s, *J* = 8 Hz), 8.25 - 8.37 (2H, m), 8.68 (1H, app. dt, *J* = 0.7 and 7.8 Hz) and 9.09 (1H, app. d, *J* = 6.3 Hz); δ_{C} (CDCl₃, 100 MHz) 17.08, 65.60, 123.22, 124.71 125.33, 126.32, 126.68, 127.70, 129.97, 131.92, 136.13, 142.72, 143.26, 146.03, 147.32, 150.52 and 190.35.

### 6-Methyl-7-phenyldipyrido[1,2-a:2',1'-c]pyrazine-5,8-diium dibromide

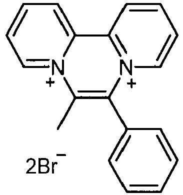

A mixture of 1-(1-oxo-1-phenylpropan-2-yl)-[2,2'-bipyridin]-1-ium bromide (2.13 g, 5.8 mmol) in PBr₃ (15 mL) was heated at reflux for 15 min. After cooling the residue was filtered off, washed with Et₂O (3 × 50 mL), twice crystallised from EtOH/diisopropyl ether, filtered and dried under vacuum to give the *title compound* (2.06 g, 82 %) as a yellow powder which was used without further purification in the next step.

### 6-Methyl-7-phenyldipyrido[1,2-a:2',1'-c]pyrazine-5,8-diium bis(tetrafluoroborate) Compound 6

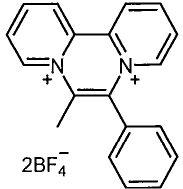

A solution of 6-methyl-7-phenyldipyrido[1,2-*a*:2',1'-*c*]pyrazine-5,8-diium bromide (2.06 g, 4.8 mmol) in water (50 mL) was added dropwise to a solution of NaBF₄ (6.29 g, 57.2 mmol) in water (50 mL) with stirring. Stirring was continued for 0.5 h and the resulting precipitate filtered, washed with water (2 × 10 mL) and air dried to give the *title compound* (0.83 g, 39 %) as a grey powder; δ_{H} (CDCl₃, 400 MHz) 3.16 (3H, s), 7.56 - 7.64 (2H, bm), 7.76 - 7.87 (3H, bm), 8.43 (1H, bt, *J* = 8.1 Hz), 7.74 (1H, bt, *J* = 7.5 Hz), 8.95 - 9.09 (2H, m), 9.16 (1H, bt, *J* = 8 Hz) and 9.74 - 9.90 (3H, m); δ_{F} (CDCl₃, 376 MHz) -150.78, -150.73; δ_{C} (CDCl₃, 100 MHz) 19.10, 128.04, 128.39, 128.68, 131.47, 131.88, 132.35, 132.49, 134.18, 135.57, 135.78, 138.87, 138.94, 140.46, 140.95, 147.19 and 147.74.

### 3-(Bromoacetyl)thiophene

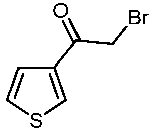

A solution of Br₂ (12 g, 75 mmol) in DCM (50 mL) was added dropwise over 1 h to a solution of 3-acetylthiophene (9.45 g, 75 mmol) in DCM (50 mL) with stirring. The resulting solution was stirred at rt for 1 h, washed with sat. NaHCO₃ (2 × 100 mL), dried (Na₂SO₄) and the solvent removed under reduced pressure. The residue was chromatographed on silica using DCM (30 % in hexanes) as eluent. The solvent was removed under reduced pressure to give the *title compound* (3.62 g, 23 %) as a colourless powder; δ_{H} (CDCl₃, 400 MHz) 4.34 (2H, s), 7.37 (1H, dd, *J* = 2.8 and 5.1 Hz), 7.58 (1H, dd, *J* = 1.2 and 5.1 Hz) and 8.18 (1H, dd, *J* = 1.2 and 2.8 Hz).

### 1-[2-Oxo-2-(thiophen-3-yl)ethyl]-[2,2'-bipyridin]-1-ium bromide

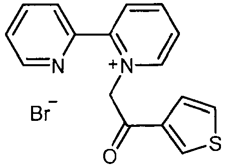

A mixture of 2,2'-bipyridine (2 g, 12.8 mmol) and 3-(bromoacetyl)thiophene (2.73 g, 13.3 mmol) was heated at 100 °C for 45 min. The resulting solid was cooled, triturated with hot Et₂O/EtOH cooled, decanted and triturated with acetone (3 × 40 mL), filtered and air dried to give the *title compound* (3.02 g, 65 %) as a yellow powder; δ_{H} (D₂O, 400 MHz) 6.06 (2H, bs), 7.40 - 7.51 (3H, m), 7.79 (1H, bd, *J* = 7.9 Hz), 7.97 (1H, bt, *J* = 7.9 Hz), 8.15 (1H, bt, *J* = 7.2 Hz), 8.20 - 8.31 (2H, m), 8.69 (1H, bt, *J* = 7.9 Hz) and 8.84 (1H, bd, *J* = 6.2 Hz); δ_{F} (D₂O, 376 MHz) -150.97, -150.91; δ_{C} (D₂O/CD₃OD, 100 MHz) 126.05, 126.35, 126.49, 127.64, 128.26, 129.98, 135.99, 136.89, 139.09, 147.43, 148.29, 148.58, 149.38, 152.06 and 186.43.

### 6-(Thiophen-3-yl)dipyrido[1,2-a:2',1'-c]pyrazine-5,8-diium dibromide

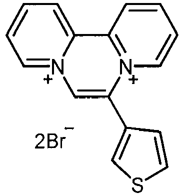

A mixture of 1-[2-oxo-2-(thiophen-3-yl)ethyl]-[2,2'-bipyridin]-1-ium bromide (2.71 g, 7.5 mmol) in PBr₃ (15 mL) was heated at reflux for 15 min. After cooling, the residue was filtered off, washed with Et₂O (3 × 50 mL), three times dissolved in hot MeOH (20 mL) and the methanol solution added to diisopropyl ether (40 mL) with stirring. The resulting precipitate was filtered off and dissolved in hot EtOH (25 mL). The solution was then filtered to remove particulates and diisopropyl ether (30 mL) was added. The resulting precipitate was filtered, washed with diisopropyl ether and dried under vacuum to give the *title compound* (1.25 g, 39 %) as a tan powder which was used without further purification in the next step; δ_{H} (D₂O, 400 MHz) 7.42 (1H, bd, *J* = 5.0 Hz), 7.84 (1H, bt, *J* = 4.0 Hz), 8.14 (1H, bs), 8.52 (1H, bt, *J* = 7.1 Hz), 8.62 (1H, bt, *J* = 7.0 Hz), 9.04 - 9.12 (2H, m), 9.18 (1H, s), 9.47 - 9.53 (2H, m), 9.67 (1H, bd, *J* = 8.7 Hz) and 9.73 (1H, bd, *J* = 8.5 Hz); δ_{C} (D₂O/CD₃OD, 100 MHz) 124.47, 125.88, 126.42, 127.03, 127.40, 130.31, 130.83, 130.89, 132.66, 133.03, 136.81, 137.79, 139.77, 141.67, 146.71 and 146.97.

### 6-(Thiophen-3-yl)dipyrido[1,2-a:2',1'-c]pyrazine-5,8-diium tetrafluoroborate hexafluorophosphate Compound 7

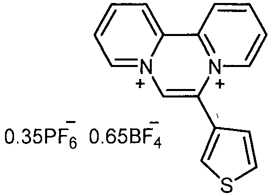

A solution of 6-(thiophen-3-yl)dipyrido[1,2-*a*:2',1'-*c*]pyrazine-5,8-diium dibromide (1.16 g, 2.7 mmol) in water (50 mL) was added dropwise with filtration through a cotton wool plug to a solution of NaBF₄ (10.83 g, 98.4 mmol) in water (50 mL) with stirring. Stirring was continued for 0.5 h and the resulting precipitate filtered off and washed with water (2 × 5 mL). Ammonium hexafluorophosphate (3.95 g, 24.2 mmol) was added to the liquors with stirring. The resulting precipitate was filtered, washed with water (2 × 5 mL), air dried, then washed with MeOH (5 mL) and air dried to give the *title compound* (1.40 g, 92 %) as a grey powder; δ_{H} (D₂O, 400 MHz) 7.43 (1H, bd, *J =* 4.6 Hz), 7.82 - 7.88 (1H, bs), 8.14 (1H, bs), 8.54 (1H, bt, *J* = 7.4 Hz), 8.63 (1H, bt, *J* = 7.1 Hz), 9.05 - 9.13 (2H, m), 9.18 (1H, s), 9.47 - 9.55 (2H, m), 9.68 (1H, bd, *J* = 8.8 Hz) and 9.74 (1H, bd, *J* = 8.3 Hz); δ_{F} (D₂O, 376 MHz) - 150.53, -150.48, -72.19 (d, *J =* 709.6 Hz); δ_{P} (D₂O, 162 MHz) -145.14 (sept *J* = 709.6). The ¹³C spectrum could not be obtained owing to low solubility.

### 1-[2-(2-Fluorophenyl)-2-oxoethyl]-[2,2'-bipyridin]-1-ium bromide

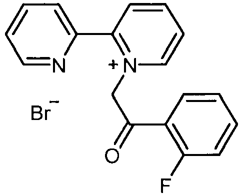

A mixture of 2,2'-bipyridine (2 g, 12.8 mmol) and 2-bromo-1-(2-fluorophenyl)ethan-1-one (2.88 g, 13.3 mmol) was heated at 100 °C for 45 min. The resulting solid was triturated with hot Et₂O/EtOH. The solid product was collected filtration, washed with Et₂O (2 × 50 mL) and air dried to give the *title compound* (4.06 g, 85 %) as a cream powder; δ_{H} (CD₃OD, 400 MHz) 6.22 - 6.27 (2H, m), 7.35 (1H, ddd, *J* = 1.0, 8.3 and 11.9 Hz), 7.43 (1H, dt, *J* = 1.1 and 7.7 Hz), 7.54 (1H, ddd, *J =* 1.4, 4.8 and 7.5 Hz), 7.74 - 7.80 (1H, m), 8.00 (1H, dt, *J* = 1.9 and 7.5 Hz), 8.04 - 8.14 (2H, m), 8.25 - 8.33 (2H, m), 8.48 (1H, dd, *J* = 1.5 and 8.1 Hz), 8.84 (1H, dt, *J* = 1.5 and 7.9 Hz) and 9.12 (1H, dd, *J* = 1.5 and 6.2 Hz); δ_{F} (CD₃OD, 376 MHz) -110.84 - - 110.70 (m); δ_{C} (CD₃OD, 100 MHz) 116.57 (d, *J* = 23.2 Hz), 122.02 (d, *J =* 13.0 Hz), 125.06 (d, *J = 3.2* Hz), 126.03, 126.54, 127.02, 129.84, 130.58 (d, *J* = 2.4 Hz), 136.41 (d, *J* = 9.4 Hz), 138.53, 147.06, 148.74, 148.96, 149.32, 152.01, 160.86, 163.38 and 187.42 (d, *J* = 4 Hz).

### 6-(2-Fluorophenyl)dipyrido[1,2-a:2',1'-c]pyrazine-5,8-diium dibromide

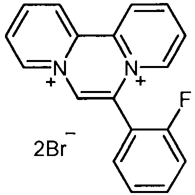

A mixture of 1-[2-(2-fluorophenyl)-2-oxoethyl]-[2,2'-bipyridin]-1-ium bromide (3.81 g, 10.2 mmol) in PBr₃ (15 mL) was heated at reflux for 15 min then cooled and the residue filtered, washed with Et₂O (3 × 50 mL). The crude product was triturated with EtOH then with hot EtOH/diisopropyl ether, filtered off and washed with diisopropyl ether and then triturated three times with hot EtOH (20 mL). The product was collected by filtration, washed with Et₂O (2 × 20 mL) and air dried to give the *title compound* (2.34 g, 53 %) as a dark grey powder; δ_{H} (D₂O, 400 MHz) 7.42 - 7.59 (2H, m), 7.73 (1H, bt, *J* = 6.6 Hz), 7.81 - 7.88 (1H, m), 8.55 (1H, bt, *J* = 7.0 Hz), 8.67 (1H, bt, *J* = 6.9 Hz), 9.08 - 9.18 (2H, m), 9.29 (1H, s), 9.35 (1H, bd, J = 6.6 Hz), 9.55 (1H, bd, *J =* 6.3 Hz), 9.73 (1H, bd, *J* = 8.6 Hz) and 9.78 (1H, bd, *J* = 8.5 Hz); δ_{F} (D₂O, 376 MHz) -110.70; δ_{C} (D₂O, 100 MHz) 112.98 (d, *J =* 14.6 Hz), 117.11 (d, *J* = 19.7 Hz), 126.41 (d, *J* = 3.5 Hz), 126.48, 127.21, 127.60, 131.01 (d, *J* = 6.0 Hz), 131.78, 132.48 (d, *J =* 0.9 Hz), 136.15 (d, *J* =8.7 Hz), 137.23, 137.97, 139.81, 142.09, 147.25, 147.37, 159.14 and 161.64.

### 6-(2-Fluorophenyl)dipyrido[1,2-a:2',1'-c]pyrazine-5,8-diium bis(tetrafluoroborate) Compound 8

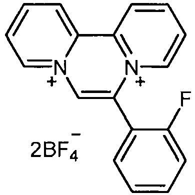

A solution of 6-(2-fluorophenyl)dipyrido[1,2-*a*:2',1'-*c*]pyrazine-5,8-diium dibromide (1.75 g, 4 mmol) in water (40 mL) was added dropwise to a solution of NaBF₄ (6.62 g, 60.2 mmol) in water (40 mL) with stirring. Stirring was continued for 0.5 h and the resulting precipitate filtered off, washed with water (3 × 5 mL) and air dried to give the *title compound* (1.26 g, 70 %) as a purple powder; δ_{H} (D₂O, 400 MHz) 7.42 - 7.58 (2H, m), 7.69 (1H, bt, *J* = 7.5 Hz), 7.80 - 7.88 (1H, m), 8.53 (1H, bt, *J* = 7.2 Hz), 8.65 (1H, bt, *J* = 7.0 Hz), 9.08 - 9.17 (2H, m), 9.27 (1H, s), 9.34 (1H, bd, *J* = 6.3 Hz), 9.53 (1H, bd, *J* = 6.3 Hz), 9.71 (1H, bd, *J =* 8.6 Hz) and 9.76 (1H, bd, *J* = 8.5 Hz); δ_{F} (D₂O, 376 MHz) -150.53 (bq, *J* = 1.4 Hz), -150.48, -110.88. The ¹³C spectrum could not be obtained owing to low solubility.

### 1-(2-Oxo-1,2-diphenylethyl)-[2,2'-bipyridin]-1-ium bromide

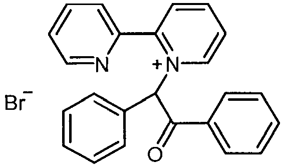

A solution of 2,2'-bipyridine (3.20 g, 20.5 mmol) and desyl bromide (4.00 g, 14.6 mmol) in MeCN (40 mL) was heated at reflux for 2 days, cooled and the solvent removed under reduced pressure. The residue was triturated with acetone/Et₂O (1/1, 40 mL), filtered off and crystallised from EtOH/Et₂O. The resulting precipitate was crystallised from EtOH (5 mL), filtered and air dried to give the *title compound* (0.96 g, 15 %) as a yellow powder which was used without further purification in the next step.

### 6,7-Diphenyldipyrido[1,2-a:2',1'-c]pyrazine-5,8-diium dibromide

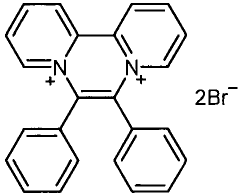

A solution of 1-(2-oxo-1,2-diphenylethyl)-[2,2'-bipyridin]-1-ium bromide (0.96 g, 2.2 mmol) in in PBr₃ (10 mL) was heated at reflux for 15 min. After cooling the product was filtered off and triturated with hot EtOH (5 mL). The solid product was collected by filtration, washed with EtOH (2 mL) and air dried to give the *title compound* (0.63 g, 57 %) as a pale green powder; δ_{H} (D₂O, 400 MHz) 7.43 - 7.50 (10H, m), 8.43 (2H, bt, *J* = 7.2 Hz), 9.09 (2H, bt, *J =* 8.0 Hz), 9.15 (2H, bd, *J* = 6.6 Hz) and 9.81 (2H, bd, *J =* 8.5 Hz); δ_{C} (D₂O, 100 MHz) 126.39, 126.90, 130.13, 130.59, 130.72, 131.98, 135.87, 137.54, 140.27 and 146.76.

### 6,7-Diphenyldipyrido[1,2-a:2',1'-c]pyrazine-5,8-diium bis(tetrafluoroborate) Compound 9

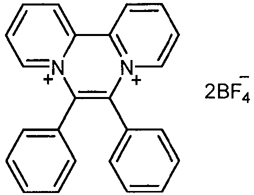

A solution of 6,7-diphenyldipyrido[1,2-*a*:2',1'-*c*]pyrazine-5,8-diium dibromide (0.63 g, 1.3 mmol) in water (20 mL) was added dropwise to a solution of NaBF₄ (2.80 g, 25.4 mmol) in water (20 mL) with stirring. Stirring was continued for 0.5 h and the resulting precipitate collected by filtration, washed with water (2 × 2 mL) and air dried to give the *title compound* (0.56 g, 86 %) as a grey powder; δ_{H} (D₂O, 400 MHz) 7.43 - 7.50 (10H, m), 8.42 (2H, bt, *J* = 7.1 Hz), 9.08 (2H, bt, *J* = 8.1 Hz), 9.15 (2H, bd, *J* = 6.6 Hz) and 9.80 (2H, bd, *J* = 8.6 Hz); δ_{F} (D₂O, 376 MHz) -150.56 (q, *J =* 1.35 Hz), -150.50 (bs); δ_{C} (D₂O, 100 MHz) 126.36, 126.87, 130.13, 130.56, 130.70, 131.98, 135.86, 137.50, 140.28 and 146.77.

### 2-(Bromoacetyl)thiophene

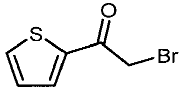

A solution of bromine (19.05 g, 6.1 ml, 119 mmol) in DCM (80 mL) was added dropwise to 2-acetylthiophene (15 g, 119 mmol) in DCM (80 mL) over 1 h with stirring. The resulting solution was stirred for an additional hour, washed with sat. NaHCO₃ (2 × 200 mL), brine (100 mL), dried (Na₂SO₄) and the solvent removed under reduced pressure. The residue was distilled (100 °C, 4 mbar) and chromatographed on silica using DCM (40 % in hexanes) as eluent. The solvent was removed under reduced pressure to give the *title compound* (9.92 g, 41 %) as a pale yellow oil; δ_{H} (CDCl₃, 400 MHz) 4.36 (2H, s), 7.17 (1H, dd, *J* = 3.9 and 4.9 Hz), 7.72 (1H, dd, *J* = 1 and 4.9 Hz) and 7.81 (1H, dd, *J* = 1 and 3.9 Hz).

### 1-[2-Oxo-2-(thiophen-2-yl)ethyl]-[2,2'-bipyridin]-1-ium bromide

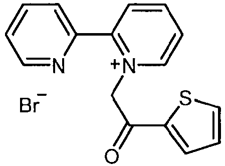

A mixture of 2,2'-bipyridine (2 g, 12.8 mmol) and 2-(bromoacetyl)thiophene (2.73 g, 13.3 mmol) was heated at 100 °C for 45 min. The resulting solid was cooled, stirred with Et₂O/EtOH for 16 h, filtered off and dried under vacuum to give the *title compound* (2.38 g, 51 %) as a tan powder which was used without further purification in the next step.

### 6-(Thiophen-2-yl)dipyrido[1,2-a:2',1'-c]pyrazine-5,8-diium dibromide

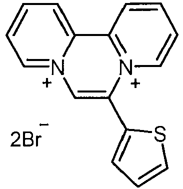

A mixture of 1-[2-oxo-2-(thiophen-2-yl)ethyl]-[2,2'-bipyridin]-1-ium bromide (2.38 g, 6.6 mmol) in PBr₃ (15 mL) was heated at reflux for 15 min. After cooling, the residue was filtered off, washed with Et₂O (3 × 50 mL) and triturated with Et₂O containing a few drops of EtOH. The resulting solid was triturated with warm EtOH (160 mL), collected and air dried to give the *title compound* (1.48 g, 53 %) as a dark green powder which was used without further purification in the next step.

### 6-(Thiophen-2-yl)dipyrido[1,2-a:2',1'-c]pyrazine-5,8-diium bis(hexafluorophosphate) Compound 10

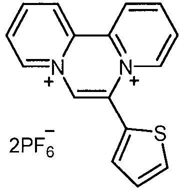

A solution of 6-(thiophen-2-yl)dipyrido[1,2-*a*:2',1'-*c*]pyrazine-5,8-diium dibromide (1.42 g, 3.3 mmol) in water (100 mL) was added dropwise with filtration through a cotton wool plug to a solution of NH₄PF₆ (5.46 g, 33.4 mmol) in water (100 mL) with stirring. The resulting precipitate was filtered, washed with water (2 × 5 mL) and air dried to give the *title compound* (1.34 g, 72 %) as a tan powder; δ_{H} (D₂O, 400 MHz) 7.41 (1H, bt, *J =* 4.1 Hz), 7.73 (1H, bd, *J* = 3.4 Hz), 8.03 (1H, bd, *J =* 5.1 Hz), 8.56 (1H, bt, *J* = 7.1 Hz), 8.65 (1H, bt, *J* = 7.1 Hz), 9.08 - 9.15 (2H, m), 9.30 (1H, bs), 9.51 (1H, bd, *J* = 6.3 Hz) and 9.64 - 9.77 (3H, m); δ_{F} (D₂O, 376 MHz) -72.15 (d, *J =* 710 Hz); δ_{P} (D₂O, 162 MHz) -145.11 (sept *J =* 710 Hz). The ¹³C spectrum could not be obtained owing to low solubility.

### 2-(Pyridin-2-yl)-1H-benzimidazole

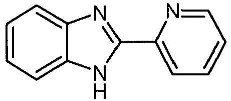

A mixture of pyridine-2-carboxaldehyde (6.00 g, 56 mmol) and o-phenylenediamine (6.06 g, 56 mmol) in EtOH (300 mL) was left to stand under air for 2 days. The solvent was reduced in volume (~50 mL) and the resulting precipitate filtered, washed with MeOH (10 mL) and air dried to give the *title compound* (2.26 g, 21 %) as a yellow powder; δ_{H} (CDCl₃, 400 MHz) 7.27 - 7.34 (2H, m), 7.35 - 7.41 (1H, ddd, *J* = 1.1, 4.8 and 7.6 Hz), 7.45 - 7.52 (1H, m), 7.82 - 7.91 (2H, m), 8.45 (1H, dt, *J* = 1.1 and 7.9 Hz), 8.64 (1H, ddd, *J* = 1, 1.7 and 5.8 Hz) and 10.84 (1H, bs); δ_{C} (CDCl₃, 100 MHz) 111.29, 120.14, 121.70, 122.73, 123.96, 124.64, 133.87, 137.38, 144.39, 148.30, 149.14 and 150.78.

### 1-Methyl-2-(pyridin-2-yl)-1H-benzimidazole

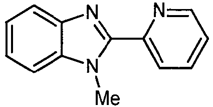

A mixture of 2-(pyridin-2-yl)-1*H*-benzimidazole (3.00 g, 15.4 mmol) and K₂CO₃ (3.18 g, 23 mmol) in dry DMF (30 mL) was stirred at rt. After 0.5 h MeI (2.62 g, 18.4 mmol) was added and stirring continued for 16 h. The resulting mixture was poured into water (100 mL) and extracted with EtOAc (3 × 300 mL). The combined extracts were washed with water (100 mL), dried (Na₂SO₄) and the solvent removed under reduced pressure. The residue was filtered through silica using EtOAc as eluent and the solvent was removed under reduced pressure to give the *title compound* (2.89 g, 90 %) as a viscous orange oil; δ_{H} (DMSO-*d*₆, 400 MHz) 4.22 (3H, s), 7.23 - 7.35 (2H, m), 7.51 (1H, ddd, *J* = 1.2, 4.9 and 7.5 Hz), 7.61 - 7.66 (1H, m), 7.69 - 7.74 (1H, m), 7.99 (1H, dt, *J* = 1.8, 7.7 Hz), 8.30 (1H, dt, *J* = 1 and 7.9 Hz) and 8.74 (1H, ddd, *J =* 1, 1.7 and 5.8 Hz); δ_{C} (DMSO-*d*₆, 100 MHz) 33.07, 111.24, 119.87, 122.80, 123.57, 124.67, 124.84, 137.58, 137.86, 142.50, 149.27, 150.18 and 150.57.

### 1-Methyl-3-(2-oxo-2-phenylethyl)-2-(pyridin-2-yl)-1H-benzimidazol-3-ium bromide

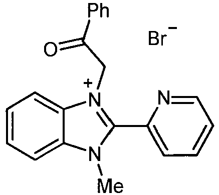

A mixture of 1-methyl-2-(pyridin-2-yl)-1*H*-benzimidazole (2.82 g, 13.5 mmol) and phenacyl bromide (2.68 g, 13.5 mmol) was heated at 100 °C for 16 h, allowed to cool, then crystallised from EtOH at 0 °C to give the *title compound* (4.12 g, 75 %) as a colourless prisms; δ_{H} (DMSO-*d*₆, 400 MHz) 4.18 (3H, s), 6.39 (2H, s), 7.57 - 7.63 (2H, m), 7.71 - 7.84 (4H, m), 8.00 - 8.06 (2H, m), 8.14 - 8.26 (4H, m) and 8.70 (1H, m); δ_{C} (DMSO-*d*₆, 100 MHz) 34.22, 53.19, 114.34, 114.49, 127.72, 127.93, 127.98, 128.60, 128.92, 129.48, 132.12, 132.46, 134.10, 135.01, 138.78, 140.91, 147.97, 151.23 and 191.52.

### 6-Phenylbenzimidazo[1,2-a]pyrido[2,1-c]pyrazin-5-ium tetrafluoroborate

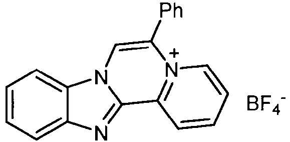

A mixture of 1-methyl-3-(2-oxo-2-phenylethyl)-2-(pyridin-2-yl)-1*H*-benzimidazol-3-ium bromide (2.00 g, 4.9 mmol) in PBr₃ (15 mL) was heated at reflux for 25 min, cooled and diluted with Et₂O (50 mL). The residue was filtered and washed with Et₂O (3 × 50 mL). The residue was dissolved in MeOH (15 mL) and added dropwise to a solution of NaHCO₃ (4.12 g, 4.9 mmol) in water (100 mL) with stirring. Solid NaBF₄ (10.78 g, 98 mmol) was added and the resulting mixture stirred for 0.5 h before filtering. The filtrand was dissolved in MeOH (100 mL), poured into water (150 mL) and the solvent reduced. The resulting precipitate was filtered and triturated with MeOH (20 mL), then collected by filtration and air dried to give the *title compound* (0.84 g, 45 %) as a dull green powder; δ_{H} (DMSO-*d*₆, 400 MHz) 7.68 - 7.82 (7H, m), 8.15- 8.27 (2H, m), 8.45 - 8.54 (1H, m), 8.80 (1H, t, *J* = 8 Hz), 8.96 (1H, d, *J* = 6.7 Hz), 9.39 (1H, dd, *J* = 1.4 and 8.3 Hz) and 9.56 (1H, s); δ_{C} (DMSO-*d*₆, 100 MHz) 113.33, 121.09, 121.26, 124.67, 126.29, 127.21, 127.66, 127.74, 129.01, 130.22, 130.37, 131.77, 137.39, 137.64, 139.79, 143.65 and 144.09.

### 13-Methyl-6-phenyl-13H-benzimidazo[1,2-a]pyrido[2,1-c]pyrazine-5,8-diium bis(tetrafluoroborate) Compound 11

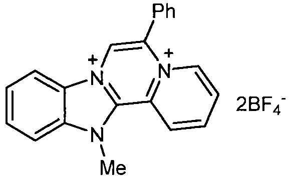

A mixture of 6-phenylbenzimidazo[1,2-*a*]pyrido[2,1-*c*]pyrazin-5-ium tetrafluoroborate (0.70 g, 1.86 mmol) in MeOTs (2.77 g, 14.9 mmol) was heated at 180 °C for 1 h, cooled and diluted with Et₂O (40 mL). The residue was filtered and triturated with Et₂O (3 × 20 mL) and air dried. The filtrand was dissolved in MeOH (20 mL) containing a few drops of water and added dropwise to a solution of NaBF₄ (4.09 g, 37.2 mmol) in water (100 mL) with stirring. Stirring was continued for 0.5 h and the resulting precipitate filtered, washed with water (2 × 5 mL) and air dried. The solid was triturated with MeOH (10 mL), filtered and air dried to give the *title compound* (0.69 g, 77 %) as a khaki powder; δ_{H} (DMSO-*d*₆, 400 MHz) 4.89 (3H, s), 7.78 - 7.90 (5H, m), 8.09 (1H, t, *J =* 8 Hz), 8.18 (1H, t, *J* = 8 Hz), 8.57 - 8.64 (2H, m), 8.82 (1H, t, *J* = 8.3 Hz), 9.14 (1H, t, *J* = 8 Hz), 9.47 (1H, d, *J* = 6.6 Hz), 9.84 (1H, d, *J* = 8.4 Hz) and 10.01 (1H, s); δ_{C} (DMSO-*d*₆, 100 MHz) 36.72, 114.97, 115.04, 120.25, 126.54, 127.45, 127.87, 129.65, 130.67, 131.01, 131.51, 131.84, 131.91, 132.35, 132.66, 134.24, 135.63, 140.75 and 145.56.

### 4-Phenyl-2-(pyridin-2-yl)quinoline

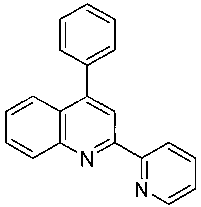

A solution of 2-aminobenzophenone (6.51 g, 33 mmol) and 2-acetylpyridine (4.00 g, 33 mmol) in AcOH (80 mL) containing H₂SO₄ (2 mL) was heated at reflux for 24 h. The mixture was cooled, poured into ice/NH₄OH solution and extracted with CHCl₃ (3 × 200 mL). The combined organic extracts were washed with water (100 mL), dried (Na₂SO₄) and the solvent removed under reduced pressure. The residue was chromatographed on silica using EtOAc (0 - 100 % in DCM) gradient as eluent. The fluorescent band was collected and the solvent removed under reduced pressure. The residue was triturated with hot EtOAc/hexanes, then cooled and collected by filtration, washed with hexanes and air dried to give the *title compound* (5.80 g, 62 %) as yellow prisms.

### 4-Phenyl-2-(pyridin-2-yl)quinoline dibromide

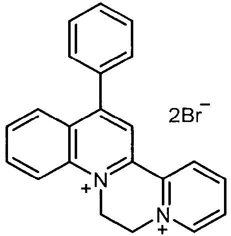

A solution of 4-phenyl-2-(pyridin-2-yl)quinoline (1.05 g, 3.7 mmol) in 1,2-dibromoethane (30 mL) was heated at reflux for 24 h, After cooling the precipitate was filtered off, washed with hexanes and air dried to give the *title compound* (1.43 g, 82 %) as a dull yellow powder; δ_{H} (CD₃OD, 400 MHz) 5.55 (2H, bt, *J* = 6.1 Hz), 5.81 (2H, bt, *J* = 6.1 Hz), 7.75 - 7.83 (3H, m), 7.86 - 7.94 (2H, m), 8.18 (1H, bt, *J* = 8 Hz), 8.45 - 8.56 (3H, m), 8.85 (1H, bd, *J* = 9.3 Hz), 8.96 - 9.05 (2H, m), 9.34 (1H, bd, *J* = 8.3 Hz) and 9.45 (1H, bd, *J* = 5.7 Hz); δ_{C} (D₂O, 100 MHz) 29.69, 52.00, 118.74, 122.13, 129.01, 129.10, 129.19, 129.94, 130.11, 130.73, 131.27, 131.69, 134.67, 137.29, 139.77, 140.98, 141.63, 147.03, 147.75 and 161.30.

### 4-Phenyl-2-(pyridin-2-yl)quinoline bis(tetrafluoroborate) Compound 12

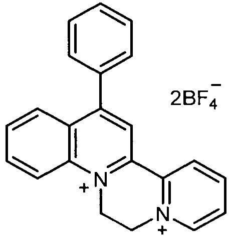

A solution of 4-phenyl-2-(pyridin-2-yl)quinoline dibromide (1.43 g, 3 mmol) in MeOH (20 mL) was added dropwise to a solution of NaBF₄ (4 g, 36.6 mmol) in water (100 mL) with stirring. Stirring was continued for 0.5 h, additional NaBF₄ (13.39 g, 122 mmol) was added and stirring continued for 0.5 h. Additional NaBF₄ (13.29 g, 122 mmol) was again added and stirring continued for 0.5 h to induce precipitation. The resulting precipitate was filtered, washed with water (2 × 20 mL) and air dried to give the *title compound* (1.47 g, 100 %) as a lime green powder; δ_{H} (DMSO-*d*₆, 400 MHz) 4.68 (2H, bs), 4.93 (2H, bs), 6.94 - 7.11 (5H, m), 7.39 (1H, bt, *J* = 7.6 Hz), 7.64 - 7.74 (3H, m), 7.97 (1H, bd, *J* = 8.7 Hz), 8.12 (1H, s), 8.20 (1H, bt, *J* = 7.5 Hz), 8.39 (1H, bd, *J* = 8 Hz) and 8.56 (1H, bd, *J* = 5.6 Hz); δ_{F} (DMSO-*d*₆, 376 MHz) -153.53, -153.48; δ_{C} (DMSO-*d*₆, 100 MHz) 45.53, 51.18, 117.67, 120.99, 128.24, 128.42, 128.54, 129.02, 129.33, 130.21, 130.70, 131.12, 133.70, 136.96, 138.97, 139.86, 140.31, 146.19, 147.38 and 160.95.

### 2-Amino-4'-methoxybenzophenone

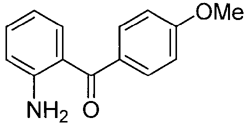

A solution of 4-bromoanisole (30.00 g, 160.4 mmol) in THF (100 mL) was added to Mg (4.29 g, 176.5 mmol), pretreated with I₂, and heated at 70 °C for 1 h. The resulting solution of 4-MeOC₆H₄MgBr was cooled and solid 2-aminobenzonitrile (6.31 g, 53.5 mmol) was added and the mixture stirred at rt for 16 h. A solution of concentrated HCl (40 mL) and water (50 mL) was added cautiously. The resulting mixture was heated at reflux for 1 h, then cooled, and neutralised (NaOH, 2 M) and extracted with DCM (4 × 50 mL). The combined extracts were washed with water (100 mL), dried (Na₂SO₄) and the solvent removed under reduced pressure. The residue was chromatographed on silica using DCM as eluent and the yellow band collected. The solvent was removed under reduced pressure to give the *title compound* (11.16 g, 92 %) as a viscous yellow oil.

### 4-(4-Methoxyphenyl)-2-(pyridin-2-yl)quinoline

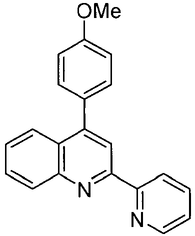

A solution of 2-amino-4'-methoxybenzophenone (5.00 g, 22 mmol) and 2-acetylpyridine (2.66 g, 22 mmol) in AcOH (80 mL) containing H₂SO₄ (2 mL) was heated at reflux for 16 h. The resulting solution was poured onto ice/NH₄OH (100 mL), extracted with DCM (3 × 80 mL). The combined extracts were washed with water (100 mL), dried (Na₂SO₄) and the solvent removed under reduced pressure. The residue was filtered through neutral alumina using DCM as eluent. The solvent was removed under reduced pressure and the residue was triturated with EtOAc (5 mL) and air dried to give the *title compound* (3.59 g, 52 %) as a light tan powder.

### 14-(4-Methoxyphenyl)-6,7-dihydropyrido[2',1':3,4]pyrazino[1,2-a]quinoline-5,8-diium dibromide

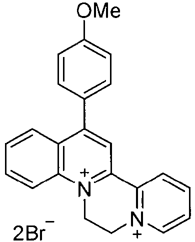

A mixture of 4-(4-methoxyphenyl)-2-(pyridin-2-yl)quinoline (1.00 g, 3.2 mmol) in 1,2-dibromoethane (15 mL) was heated at reflux for 16 h. After cooling the residue was filtered off, washed with Et₂O (2 × 30 mL) and air dried to give the *title compound* (0.90 g, 56 %) as an orange powder.

### 14-(4-Methoxyphenyl)-6,7-dihydropyrido[2',1':3,4]pyrazino[1,2-a]quinoline-5,8-diium bis(tetrafluoroborate) and bis(hexafluorophosphate) Compound 13 and Compound 14

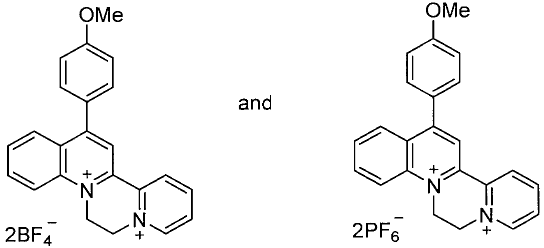

A solution of 14-(4-methoxyphenyl)-6,7-dihydropyrido[2',1':3,4]pyrazino[1,2-*a*]quinoline-5,8-diium dibromide (0.90 g, 1.8 mmol) in water (20 mL) was added dropwise to a solution of NaBF₄ (3.96 g, 36 mmol) in water (100 mL) with stirring. Stirring was continued for 0.5 h, then MeOH (10 mL) was added and the solution warmed to effect dissolution. Solid NaBF₄ (39.6 g, 180 mmol) was added to the solution and the mixture stirred for 0.5 h. The precipitate was collected by filtration, washed with water (2 × 5 mL) and air dried to give 14-(4-methoxyphenyl)-6,7-dihydropyrido[2',1':3,4]pyrazino[1,2-a]quinoline-5,8-diium bis(tetrafluoroborate) (0.56 g, 61 %) as an orange powder; ¹H NMR (400 MHz, CD₃OD/D₂O) δ_{H} 3.99 (3H, s), 5.40 - 5.47 (2H, m), 5.62 - 5.70 (2H, m), 7.34 (2H, d, *J =* 8.8 Hz), 7.88 (2H, d, *J* = 8.8 Hz), 8.14 (1H, br. t, *J =* 7.7 Hz), 8.39 - 8.50 (2H, m), 8.54 (1H, bd, *J =* 8 Hz), 8.68 (1H, br. d, *J =* 9 Hz), 8.83 (1H, s), 8.98 (1H, br. t, *J =* 7.8 Hz), 9.14 (1H, br. d, *J =* 8 Hz) and 9.32 (1H, br. d, *J =* 5.7 Hz); ¹⁹F NMR (376 MHz, CD₃OD/D₂O) δ_{F} -151.65 (bs), -151.60 (bs); ¹³C NMR (100 MHz, CD₃OD/D₂O) δ_{C} 46.06, 52.12, 55.55, 115.11, 118.23, 121.24, 126.98, 128.73, 129.61, 129.65, 130.98, 131.71, 132.62, 137.68, 139.85, 140.43, 140.81, 146.91, 148.25, 161.44 and 162.59. The aqueous washings were combined and solid NH₄PF₆ (5.87 g, 36 mmol) was added; the resulting precipitate was collected by filtration, washed with water (2 × 5 mL), MeOH (2 mL) and air dried to give 14-(4-methoxyphenyl)-6,7-dihydropyrido[2',1':3,4]pyrazino[1,2-a]quinoline-5,8-diium bis(hexafluorophosphate) (0.33 g, 20 %) as an orange powder; ¹H NMR (400 MHz, CD₃OD/D₂O) δ_{H} 4.01 (3H, s), 5.43 - 5.50 (2H, m), 5.65 - 5.72 (2H, m), 7.36 (2H, d, *J =* 8.8 Hz), 7.90 (2H, d, *J =* 8.8 Hz), 8.17 (1H, dd, *J = 6.7* and 8.9 Hz), 8.44 - 8.51 (2H, m), 8.56 (1H, dd, *J =* 1.5 and 8.6 Hz), 8.71 (1H, d, *J =* 8.8 Hz), 8.86 (1H, s), 9.00 (1H, app. dt, *J =* 1.5 and 8.1 Hz), 9.17 (1H, dd, *J* = 1.3 and 8.3 Hz) and 9.34 (1H, dd, *J* = 1.3 and 6.0 Hz); ¹⁹F NMR (376 MHz, CD₃OD/D₂O) δ_{F} -70.15 (d, J= 710 Hz); The low solubility of this material precluded recording of a ¹³C NMR spectrum.

### 2-Amino-4'-(trifluoromethyl)benzophenone

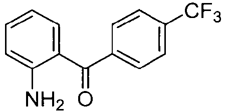

A solution of 4-bromotrifluorobenzene (15.0 g, 66.7 mmol) in THF (70 mL) was added to Mg (1.6 g, 70 mmol) pretreated with I₂ and heated at 70 °C for 1 h. Solid 2-aminobenzonitrile (2.62 g, 22.2 mmol) was added to the solution of 4-F₃CC₆H₄MgBr and the mixture stirred at rt for 16 h. A mixture of concentrated HCl (10 mL) and water (50 mL) was added cautiously. The resulting mixture was heated at reflux for 1 h, then cooled and neutralised (NaOH, 2 M). The mixture was extracted with EtOAc (3 × 50 mL), and the combined extracts washed with water (100 mL), dried (Na₂SO₄) and the solvent removed under reduced pressure. The residue was triturated with hexanes to give the *title compound* (4.41 g, 75 %) as an orange powder.

### 2-(Pyridin-2-yl)-4-[4-(trifluoromethyl)phenyl]quinoline

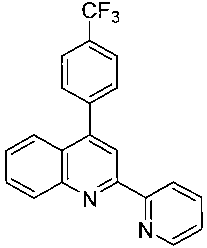

A solution of 2-amino-4'-(trifluoromethyl)benzophenone (4.41 g, 16.6 mmol) and 2-acetylpyridine (2.01 g, 16.6 mmol) in AcOH (60 mL) containing H₂SO₄ (1.5 mL) was heated at reflux for 16 h. The resulting solution was poured onto ice/NH₄OH (100 mL) and extracted with DCM (4 × 50 mL). The combined extracts were washed with water (2 × 100 mL), dried (Na₂SO₄) and the solvent removed under reduced pressure. The residue was chromatographed on neutral alumina using DCM (35 % in petroleum ether bp 40 - 60 °C) as eluent. The band with Rf ~ 0.4 was collected and the solvent removed under reduced pressure. The residue was crystallised from hot petroleum ether (bp 40 - 60 °C), filtered, washed with petroleum ether (bp 40 - 60 °C) and air dried to give the *title compound* (2.05 g, 35 %) as a pale yellow powder.

### 14-[4-(Trifluoromethyl)phenyl]-6,7-dihydropyrido[2',1':3,4]pyrazino[1,2-a]quinoline-5,8-diium dibromide

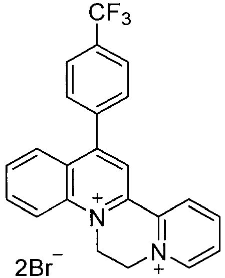

A mixture of 2-(pyridin-2-yl)-4-[4-(trifluoromethyl)phenyl]quinoline (1.00 g, 2.8 mmol) in 1,2-dibromoethane (15 mL) was heated at reflux for 16 h. After cooling the residue was filtered off, washed with Et₂O (2 × 30 mL) and air dried to give the *title compound* (0.44 g, 29 %) as an orange powder.

### 14-[4-(Trifluoromethyl)phenyl]-6,7-dihydropyrido[2',1':3,4]pyrazino[1,2-a]quinoline-5,8-diium bis(hexafluorophosphate) Compound 15

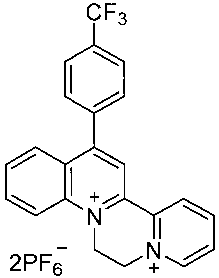

A solution of 14-[4-(trifluoromethyl)phenyl]-6,7-dihydropyrido[2',1':3,4]pyrazino[1,2-*a*]quinoline-5,8-diium dibromide (0.44 g, 0.8 mmol) in water (20 mL) was added dropwise to a solution NH₄PF₆ (1.33 g, 8 mmol) in water (20 mL) with stirring. Stirring was continued for 0.5 h and the resulting precipitate filtered off, washed with water (2 × 5 mL) and air dried to give the *title compound* (0.45 g, 83 %) as pale lime powder; ¹H NMR (400 MHz, CD₃OD/D₂O) δ_{H} 5.47 - 5.55 (2H, m), 5.75 - 5.82 (2H, m), 8.05 (2H, d, *J =* 8.2 Hz), 8.11 (2H, d, *J* = 8.2 Hz), 8.22 (1H, br. t, *J =* 7.3 Hz), 8.38 (1H, br. d, *J =* 8.3 Hz), 8.48 - 8.57 (2H, m), 8.83 (1H, br. d, *J* = 91 Hz), 8.97 - 9.06 (2H, m), 9.23 (1H, br. d, *J =* 8 Hz) and 9.40 (1H, br. d, *J =* 5.9 Hz); ¹⁹F NMR (376 MHz, CD₃OD/D₂O) δ_{F} -73.82 (d, *J =* 710 Hz), -63.95 (s); The low solubility of this material precluded recording of a ¹³C NMR spectrum.

### 4,4'-(Pyrazine-2,5-diyl)bis(1-hexylpyridin-1-ium) bis(tetrafluoroborate) Compound 16

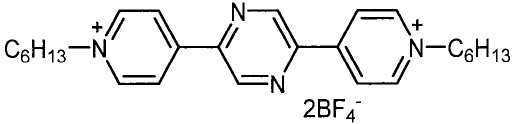

A solution of 4,4'-(pyrazine-2,5-diyl)bis(1-hexylpyridin-1-ium) diiodide (1.90 g, 2.9 mmol) in MeOH (20 mL) was added dropwise to NaBF₄ (3.81 g, 34.6 mmol) in water (120 mL) with rapid stirring. The resulting precipitate was filtered, dissolved in warm MeOH (50 mL) and added dropwise to NaBF₄ (7.62 g, 69.2 mmol) in water (300 mL) with rapid stirring. The resulting precipitate was filtered, washed with water (2 × 10 mL) and air dried to give the *title compound* (1.23 g, 74 %) as a light tan powder. The filtrate liquors were reduced in volume (~80 mL) and the residue filtered, washed with water (5 mL) then dissolved in warm MeOH (10 mL). This solution was added dropwise to NaBF₄ (7.62 g, 69.2 mmol) in water (100 mL) with rapid stirring and the residue filtered, washed with water (2 × 5 mL) and air dried to give a second crop 0.37 g (22 %) as a pale yellow powder; δ_{H} (CD₃OD, 400 MHz) 0.95 (6H, t, *J =* 7 Hz), 1.33 - 1.53 (12H, m), 2.06 - 2.17 (4H, m), 4.73 (4H, t, *J =* 7.6 Hz), 8.95 (4H, d, *J =* 6.7 Hz), 9.18 (4H, d, *J* = 6.7 Hz) and 9.78 (2H, s); δ_{F} (CD₃OD, 376 MHz) -154.31, -154.26; δ_{C} (CD₃OD, 100 MHz) 12.85, 22.07, 25.49, 30.90, 31.05, 61.57, 125.26, 143.85, 145.26, 147.66 and 150.75.

### 4,4'-(Pyrazine-2,5-diyl)bis[1-(2,4-dinitrophenyl)pyridin-1-ium] dichloride

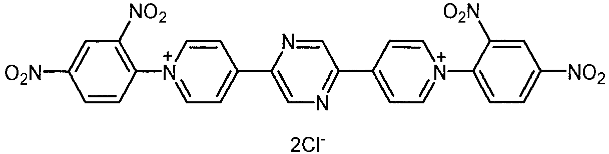

A solution of 2,5-di(pyridin-4-yl)pyrazine (1.71 g, 7.3 mmol) and 1-chloro-2,4-dinitrobenzene (8.88 g, 43.8 mmol) in EtOH (30 mL) was refluxed for 16 h, cooled, filtered, washed with EtOH (10 mL) and air dried to give the *title compound* (1.79 g, 38 %) as a yellow powder. The liquors were reduced in volume (~20 mL), heated at reflux for 16 h, then cooled (~40 °C). The residue was filtered washed with EtOH (5 mL) and air dried to give a second crop (1.78 g, 38 %) as a yellow powder; δ_{H} (CD₃OD, 400 MHz) 8.42 (2H, d, *J =* 8.6 Hz), 8.99 (2H, t, *J =* 8.6 Hz), 9.30 (4H, d, *J =* 6.1 Hz), 9.35 (2H, bs), 9.54 (4H, bd, *J* = 6.1 Hz) and 10.02 (2H, s); δ_{C} (CD₃OD, 100 MHz) 121.87, 125.24, 129.82, 131.23, 138.59, 143.26, 144.69, 146.67, 147.70, 149.93 and 153.44.

### 4,4'-(Pyrazine-2,5-diyl)bis[1-(p-tolyl)pyridin-1-ium] dichloride

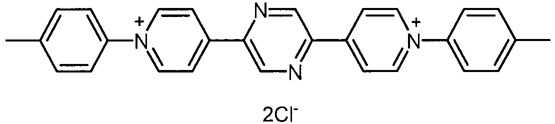

A solution of 4,4'-(pyrazine-2,5-diyl)bis[1-(2,4-dinitrophenyl)pyridin-1-ium] dichloride (0.70 g, 1.1 mmol) and p-toluidine (0.70 g, 6.5 mmol) in water (50 mL) was refluxed for 16 h. The mixture was cooled then filtered and the aqueous filtrate was washed with CHCl₃ (3 × 50 mL). The CHCl₃ extracts were discarded and the water was removed under reduced pressure. The residue was triturated with acetone, filtered and air dried to give the *title compound* (0.40 g, 75 %) as a green powder; δ_{H} (CD₃OD, 400 MHz) 2.56 (6H, s), 7.63 (4H, d, *J* = 7.8 Hz), 8.83 (4H, d, *J* = 7.8 Hz), 9.14 (4H, d, *J* =6.2 Hz), 9.45 (4H, d, *J* = 6.2 Hz) and 9.94 (2H, s); δ_{C} (CD₃OD, 100 MHz) 19.80, 123.77, 125.26, 130.88, 140.46, 142.71, 144.26, 145.18, 147.68 and 151.17.

### 4,4'-(Pyrazine-2,5-diyl)bis[1-(p-tolyl)pyridin-1-ium] bis(tetrafluoroborate) Compound 17

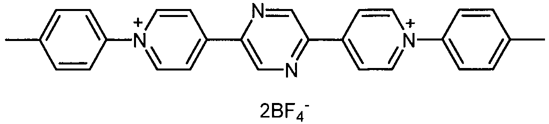

A solution of 4,4'-(pyrazine-2,5-diyl)bis[1-(p-tolyl)pyridin-1-ium] dichloride (0.40 g, 0.8 mmol) in hot water (30 mL) was added dropwise to a solution of NaBF₄ (1.08 g, 9.8 mmol) in water (20 mL) with stirring. Stirring was continued for 0.5 h and the resulting precipitate filtered, washed with water (2 × 3 mL) and air dried to give the *title compound* (0.37 g, 77 %) as a tan powder; δ_{H} [(CD₃)₂CO, 400 MHz] 2.53 (6H, s), 7.64 (4H, d, *J =* 8.2 Hz), 7.94 (4H, d, *J* = 8.2 Hz), 9.24 (4H, bs), 9.58 (4H, bs) and 10.04 (2H, s); δ_{F} [(CD₃)₂CO, 376 MHz] -151.50 and -151.45; δ_{C} [(CD₃)₂CO, 100 MHz] 20.24, 124.33, 125.73, 131.01, 140.67, 142.54, 144.64, 145.59, 147.82, and 151.16.

### 3,6-Di(pyridin-4-yl)-1,2,4-triazine

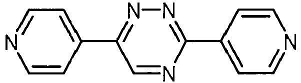

Pyridine-4-carboxaldehyde (2.36 g, 22 mmol) was added to a suspension of (1*Z*,2*Z*)-2-hydrazinylidene-2-(pyridin-4-yl)acetaldehyde oxime (3.62 g, 22 mmol) in AcOH (25 mL). The resulting mixture was stirred at rt for 1 h, heated at reflux for 0.5 h then cooled and diluted with water (60 mL). The residue was filtered off, washed with water (3 × 10 mL) and air dried. The residue was triturated with hot EtOH (30 mL), cooled, filtered and air dried to give the *title compound* (3.64 g, 70 %) as a yellow powder; δ_{H} (DMSO-*d*₆, 400 MHz) 8.27 (2H, dd, *J =* 1.6 and 4.5 Hz), 8.39 (2H, dd, *J =* 1.6 and 4.5 Hz), 8.86 - 8.91 (4H, m) and 9.71 (1H, s); δ_{C} (DMSO-*d*₆, 100 MHz) 121.40, 121.93, 140.58, 142.00, 149.25, 151.25, 151.41, 154.77 and 161.53.

### 4,4'-(1,2,4-Triazine-3,6-diyl)bis(1-hexylpyridin-1-ium) diiodide

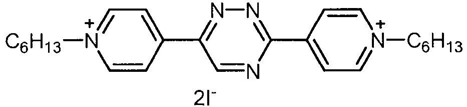

A solution of 3,6-di(pyridin-4-yl)-1,2,4-triazine (1.50 g, 6.4 mmol) and 1-iodohexane (8.12 g, 38.3 mmol) in MeCN (30 ml) was heated at reflux for 16 h, cooled and the solvent removed under reduced pressure. The residue was triturated with Et₂O (3 × 50 mL) and dried under vacuum to give the *title compound* (4.08 g, 97 %) as a brown powder; δ_{H} (DMSO-*d*₆, 400 MHz) 0.79 - 0.92 (6H, m), 1.18 - 1.39 (12H, m), 1.92 - 2.07 (4H, m), 4.68 - 4.81 (4H, m), 9.05 (2H, d, *J =* 6.9 Hz), 9.09 (2H, d, *J =* 6.8 Hz), 9.36 (2H, d, *J =* 6.9 Hz), 9.41 (2H, d, *J =* 6.9 Hz) and 10.04 (1H, s); δ_{C} (d₆-DMSO, 100 MHz) 14.33, 22.35, 22.36, 25.56, 29.98, 30.55, 31.06, 31.15, 31.22, 33.30, 61.40, 61.47, 125.92, 126.51, 146.31, 146.65, 147.80, 148.85, 150.95, 153.14 and 159.90.

### 4,4'-(1,2,4-Triazine-3,6-diyl)bis(1-hexylpyridin-1-ium) bis(hexafluorophosphate) Compound 18

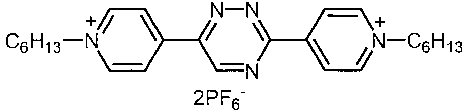

A solution of 4,4'-(1,2,4-triazine-3,6-diyl)bis(1-hexylpyridin-1-ium) diiodide (4.08 g, 6.2 mmol) in MeOH (30 mL) was added dropwise to a solution of NH₄PF₆ (6.05 g, 37.1 mmol) in water (100 mL) with stirring. Stirring was continued for 0.5 h and the resulting precipitate filtered, washed with water (3 × 10 mL) and air dried to give the *title compound* (3.01 g, 68 %) as a yellow powder; δ_{H} (CD₃OD, 400 MHz) 0.88 - 0.97 (6H, m), 1.31 - 1.60 (12H, m), 2.21-2.33 (4H, m), 4.99 - 5.07 (4H, m), 9.20 (2H, d, *J* = 6.6 Hz), 9.32 (2H, d, J = 6.5 Hz), 9.47 - 9.53 (4H, m) and 10.05 (1H, s); δ_{F} (CD₃OD, 376 MHz) -72.46 (d, *J* = 710 Hz); δ_{C} (CD₃OD, 100 MHz) 13.29, 22.15, 25.55, 30.97, 31.24, 31.28, 62.23, 62.29, 126.00, 126.61, 145.99, 146.19, 148.45, 149.54, 149.86, 153.16 and 159.90.

### 2,3-Diphenylthieno[3,4-b]pyrazine

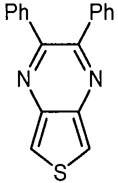

2,5-Dibromo-3,4-dinitrothiophene (6.00 g, 18.1 mmol) was suspended in aqueous HCl (12 M, 110 mL) and stirred at 0 °C. Tin powder (15.10 g, 127.3 mmol) was then added portion wise maintaining the temperature below 20 °C. The reaction mixture was stirred until all the tin was consumed. The reaction mixture was then left to stand at -20 °C for 16 hours whereupon a colourless precipitate had formed. The foregoing precipitate was collected by vacuum filtration and then washed with ether (2 × 20 mL). The resulting colourless solid was then dissolved in EtOH (100 mL), containing benzil (3.80 g, 18.1 mmol) and trimethylamine (5 mL). The foregoing solution was refluxed for 4 hours. The reaction mixture was then cooled to room temperature and water (200 mL) was added. The reaction mixture was then extracted with DCM (3 × 100 mL) and the organic layers were combined, dried (Na₂SO₄), filtered and the solvent removed under reduced pressure. The resulting residue was chromatographed on silica gel [EtOAc:Hexane = 1:9] to afford the title compound as a pale tan solid. Yield 3.00 g, 57 %. δ_{H} [CDCl₃, 400 MHz] 8.05 (2H, s), 7.50 - 7.38 (4H, m) and 7.38 - 7.22 (6H, m); δ_{C} [CDCl₃, 100 MHz] 153.5, 141.8, 139.3, 129.8, 129.0, 128.3 and 117.7.

### 5,7-Dibromo-2,3-diphenylthieno[3,4-b]pyrazine

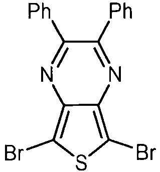

N-Bromosuccinimide (4.00 g, 22.5 mmol) was added to a solution of 2,3-diphenylthieno[3,4-b]pyrazine (3.00 g, 10.4 mmol) in a mixture of CHCl₃ and trifluoroacetic acid (1:1 v/v, 64 mL). The reaction mixture was then stirred for 16 hours at room temperature. Water (100 mL) was added to the reaction mixture which was then extracted with EtOAc (3 × 100 mL) and the organic layers were combined, dried (Na₂SO₄), filtered and the solvent removed under reduced pressure. The resulting residue was chromatographed on silica gel [eluent = 3:7 graduated to 1:1 DCM:Hexane] to afford the title compound as a green solid. Yield 1.20 g, 26 %. δ_{H} [CDCl₃, 300 MHz] 7.51 - 7.42 (4H, m) and 7.41 - 7.27 (6H, m); δ_{C} [CDCl₃, 75 MHz] 154.8, 139.4, 138.4, 130.0, 129.5, 128.3 and 1051.

### 2,3-Diphenyl-5,7-di(pyridin-4-yl)thieno[3,4-b]pyrazine

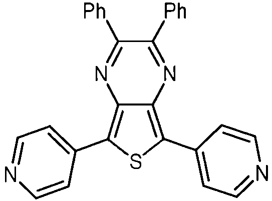

5,7-Dibromo-2,3-diphenylthieno[3,4-b]pyrazine (1.20 g, 2.7 mmol) and 4-pyridineboronic acid pinacol ester (1.40 g, 6.8 mmol) were dissolved in a mixture of DME and H₂O (2:1, 111 mL). The solution was then degassed with N₂ for 30 minutes and then Na₂CO₃ (0.86 g, 8.1 mmol) and Pd(PPh₃)₄ (0.32 g, 0.27 mmol) were added. The reaction mixture was then stirred and heated at 90 °C for 5 days under N₂. The reaction mixture was cooled to room temperature and water (100 mL) was added. The reaction mixture was extracted with EtOAc (3 × 100 mL) followed by DCM (2 × 200 mL) and the organic layers were combined, dried (Na₂SO₄), filtered and the solvent removed under reduced pressure. The resulting residue was chromatographed on silica gel [eluent = 1:19 MeOH:EtOAc] to afford the title compound as a red solid. δ_{H} [CDCl₃, 400 MHz] 8.72 (4H, dd, *J =* 4.6, 1.5 Hz), 8.23 (4H, dd, *J =* 4.6, 1.5 Hz), 7.62 - 7.51 (4H, m) and 7.48 - 7.33 (6H, m); δ_{C} [CDCl₃, 100 MHz] 154.1, 150.6, 140.9, 139.9, 138.8, 130.3, 130.0, 129.6, 128.4 and 121.6.

### 4,4'-(2,3-Diphenylthieno[3,4-b]pyrazine-5,7-diyl)bis(1-benzylpyridin-1-ium) bis(tetrafluoroborate) Compound 19

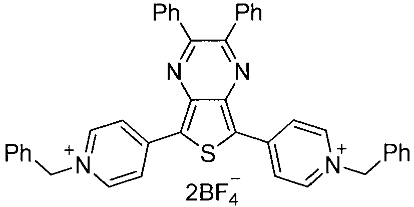

A mixture of 2,3-diphenyl-5,7-di(pyridin-4-yl)thieno[3,4-b]pyrazine (0.2 g, 0.45 mmol), benzyl bromide (0.77 g, 0.54 mL, 4.52 mmol) and MeCN (10 mL) were heated at 80 °C for 16 hours. After cooling to room temperature, the reaction mixture was filtered and the resulting orange precipitate was washed with MeCN (2 × 5 mL). The orange solid was dissolved in hot MeOH (25 mL) and added dropwise through a cotton wool plug to a stirred solution of NaBF₄ (10 g) in H₂O (200 mL) whereupon an orange precipitate formed. The resulting precipitate was collected by vacuum filtration and washed with water (50 mL) and then dried under reduced pressure to give the title compound as an orange powder. Yield 0.31 g, 86 %. δ_{H} [(CD₃)₂SO, 400 MHz] 9.32 (4H, br. app. s), 9.08 (4H, br. app. s), 7.93 - 7.15 (20H, br. m) and 5.90 (4H, br. s); δ_{C}* [(CD₃)₂SO, 100 MHz] 175.2, 155.8, 146.1, 145.1, 1426, 137.9, 134.3, 130.0 (br. s), 129.5, 129.3, 129.0, 128.4, 124.9 and 62.9; δ_{F} [(CD₃)₂SO, 376 MHz] -148.19 (br. app. s).

*Not all ¹³C{¹H} signals observed due to broad coincidental resonances.

### 2,5-Di(pyridin-4-yl)pyrazine

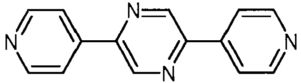

A mixture of 2,5-dibromopyrazine (2.25 g, 9.5 mmol), 4-(4,4,5,5-tetramethyl-1,3,2-dioxaborolan-2-yl)pyridine (5.81 g, 28.3 mmol), K₂CO₃ (3.91, g, 28.3 mmol) and Pd(PPh₃)₄ (0.55 g, 5 mol%) in degassed PhMe and EtOH (80 ml, 1:1) under N₂ was heated at reflux for 48 h. The cooled solution was filtered through celite and the solvent removed under reduced pressure. The residue was chromatographed on silica using MeOH (10 % in EtOAc) to MeOH (20 % in DCM) gradient as eluent. The band Rf = 0.5 (5 % MeOH in DCM) was collected and the solvent removed under reduced pressure. The residue was triturated with hot EtOAc (30 mL), cooled and filtered, then washed with EtOAc (20 mL), hexanes (20 mL) and air dried to give the *title compound* (1.32 g, 60 %) as a light salmon pink powder; δ_{H} (CDCl₃, 400 MHz) 8.01 (4H, dd, *J =* 1.4 and 4.6 Hz), 8.84 (4H, dd, *J =* 1.4 and 4.6) and 9.23 (2H, s); δ_{C} (CDCl₃, 100 MHz) 120.83, 141.80, 142.92, 149.76 and 150.87.

### 4,4'-(Pyrazine-2,5-diyl)bis(1-hexylpyridin-1-ium) diiodide

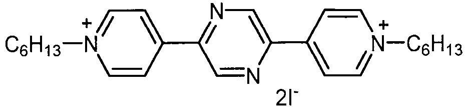

A mixture of 2,5-di(pyridin-4-yl)pyrazine (0.70 g, 3 mmol) and 1-iodohexane (3.80 g, 17.9 mmol) in MeCN (40 mL) under N₂ was heated at reflux for 24 h, then cooled and diluted with Et₂O (40 mL). The residue was filtered, washed with Et₂O (3 × 30 mL) and air dried to give the *title compound* (1.90 g, 96 %) as a red powder; δ_{H} (CD₃OD, 400 MHz) 0.96 (6H, t, *J =* 7 Hz), 1.34 - 1.54 (12H, m), 2.06 - 2.17 (4H, m), 4.74 (4H, t, *J* = 7.6 Hz), 8.98 (4H, d, *J =* 6.7 Hz), 9.23 (4H, d, *J =* 6.7) and 9.81 (2H, s); δ_{C} (CD₃OD, 100 MHz) 12.85, 22.08, 25.52, 30.91, 31.08, 61.58, 125.29, 143.92, 145.32, 147.65 and 150.74.

### 1,4-Dihydroquinoxaline-2,3-dione

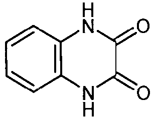

o-Phenylenediamine (15.00 g, 138.8 mmol) was added to a mixture of oxalic acid (14.60 g, 162.4 mmol) and aq. HCl (12 M) (30 mL) in water (150 mL) which had been heated to 100 °C. The reaction mixture was then heated at 100 °C for 1 hour. The reaction mixture was then cooled to room temperature and crushed ice was added (70 g). The resulting colourless needles that precipitated were collected by filtration, washed with water (3 × 50 mL) and dried under reduced pressure to give the title compound as colourless needles. Yield 15.23 g, 68 %. δ_{H} [(CD₃)₂SO, 300 MHz] 11.96 (2H, br. s) and 7.22 - 7.01 (4H, m); δ_{C} [(CD₃)₂SO, 75 MHz] 155.2, 125.6, 123.01 and 115.2.

### 1H,1'H-2,2'-Bibenzo[d]imidazole

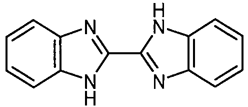

1,4-Dihydroquinoxaline-2,3-dione (8.00 g, 50 mmol) was added to diethylene glycol (50 mL) and the mixture was heated to 250 °C. o-Phenylenediamine (5.84 g, 138.8 mmol) was then added and the reaction mixture was heated at 250 °C for 2.5 hours. The reaction mixture was cooled to 50 °C and water (30 mL) was added. The reaction mixture was stirred for 20 minutes and the resulting yellow precipitate was collected by filtration. The yellow solid was washed with water (3 × 30 mL) followed by methanol (200 mL) and dried under reduced pressure. The solid was then recrystallized from hot acetic acid and then washed with hot ethyl acetate/MeOH to give the *title compound* as a yellow solid. Yield 3.66g, 32 %. δ_{H} [(CD₃)₂SO, 300 MHz] 13.58 (br. 2H, s), 7.69 (4H, br. app. s) and 7.44 - 7.17 (4H, m); δ_{C} [(CD₃)₂SO, 75 MHz] 155.2, 143.8, 125.6, 123.1 and 115.2.

### 5,6,7,12,13,14-Hexahydro-4b,7a,11b,14a-tetraazadiindeno[1,2,3-ef:1',2',3'-kl]heptalene-7a,14a-diium diiodide

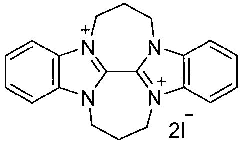

1*H*,1'*H*-2,2'-Bibenzo[*d*]imidazole (1.50 g, 6.4 mmol) and NaOH (0.67 g, 16.7 mmol) were suspended in DMF (25 mL) and the mixture was heated to 40 °C. 1,3-Diiodopropane (10.93 g, 4.24 mL, 36.9 mmol) was then added and the reaction mixture was heated at 40 °C for 0.5 h. The reaction mixture was cooled to room temperature and the resulting dark red/brown precipitate was collected by filtration, triturated with acetone (3 × 20 mL) and dried under reduced pressure to give the title compound as a dark red/brown solid. Yield 1.46g, 40 %. δ_{H} [(CD₃)₂SO, 300 MHz] 8.44 (4H, dd, *J =* 6.4, 3.1 Hz), 8.01 (4H, dd, *J =* 6.4, 3.1 Hz), 5.09 (8H, t, *J =* 6.1 Hz) and 2.99 (4H, quin, *J =* 6.0 Hz); δ_{C} [(CD₃)₂SO, 75 MHz] 135.3, 132.5, 129.2, 114.4, 46.1 and 27.1.

### 5,6,7,12,13,14-Hexahydro-4b,7a,11b,14a-tetraazadiindeno[1,2,3-ef:1',2',3'-kl]heptalene-7a,14a-diium bis(tetrafluoroborate) Compound 20

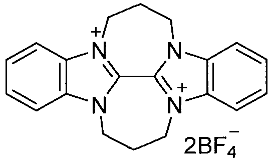

5,6,7,12,13,14-Hexahydro-4b,7a,11b,14a-tetraazadiindeno[1,2,3-*ef*:1',2',3'-*kl*]heptalene-7a,14a-diium diiodide (0.5 g, 0.88 mmol) was dissolved in hot MeOH (35 mL) and added dropwise through a cotton wool plug to a stirred solution of NaBF₄ (15.0 g) in H₂O (300 mL) whereupon an yellow precipitate formed. The foregoing precipitate was collected by vacuum filtration and washed with water (20 mL) and then dried under reduced pressure to give the title compound as a yellow powder. Yield 0.43 g, 86 %. δ_{H} [(CD₃)₂SO, 300 MHz] 8.42 (4H, dd, *J =* 6.3, 3.0 Hz), 8.02 (4H, dd, *J =* 6.3, 3.0 Hz), 5.05 (8H, t, *J =* 6.2 Hz) and 2.95 (4H, quin, *J =* 6.0 Hz); δ_{C} [(CD₃)₂SO, 75 MHz] 135.2, 132.5, 129.2, 114.3, 45.9 and 27.2; δ_{F} [(CD₃)₂SO, 376 MHz] -148.27 (8F, br. m).

### 10-Phenyl-2,3-dihydro-1H-pyrido[2',1':3,4][1,4]diazepino[1,2-a]quinoline-4,15-diium bis(hexafluorophosphate) Compound 21

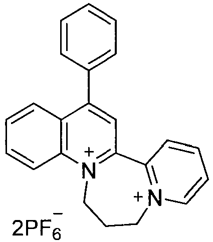

A solution of 4-phenyl-2-(pyridin-2-yl)quinoline (1.50 g, 5.6 mmol) and 1,3-diiodopropane (8.28 g, 28 mmol) in MeCN (100 mL) was heated at reflux for 3 days; the solvent was then removed under reduced pressure. The residue was dissolved in warm MeOH (100 mL) and the solution added dropwise via filtration to a solution of NH₄PF₆ (9.12 g, 55.9 mmol) in water (100 mL) with stirring. The resulting precipitate was collected by filtration and washed with water. The solid was dissolved in water/MeOH (1/1, 150 mL) and added dropwise to a solution of NH₄PF₆ (9.12 g, 55.9 mmol) in water (150 mL) with stirring. The resulting precipitate was collected by filtration, washed with water (2 × 10 mL) and air dried to give the *title compound* (1.16 g, 35 %) as a pale yellow powder; ¹H NMR (400 MHz, DMSO-*d*₆) δ_{H} 2.82 - 2.96 (2H, m), 4.42 - 4.55 (1H, m), 4.60 - 4.72 (1H, m), 5.00 - 5.13 (1H, m), 5.78 - 5.89 (1H, m), 7.72 - 7.82 (5H, m), 8.16 (1H, ddd, *J =* 0.7, 6.9 and 8.6 Hz), 8.40 (1H, dd, *J =* 1.4 and 8.6 Hz), 8.46 (1H, ddd, *J =* 1.4, 6.9 and 9.1 Hz), 8.52 - 8.57 (2H, m), 8.79 (1H, app. dd, *J =* 1.5 and 8.0 Hz), 8.94 (1H, app. d, *J =* 9.2 Hz), 8.99 (1H, app. dt, *J =* 1.4 and 8.0 Hz) and 9.39 (1H, app. dd, *J =* 1.3 and 6.1 Hz); ¹⁹F NMR (376 MHz, DMSO-*d*₆) δ_{F} -69.98 (d, *J =* 710 Hz); ¹³C NMR (100 MHz, DMSO-*d*₆) δ_{C} 30.96, 50.49, 56.47, 120.00, 126.35, 128.93, 129.04, 129.92, 130.71, 131.57, 131.75, 131.97, 132.65, 134.94, 137.46, 140.24, 144.78, 147.07, 147.58, 148.50 and 159.38.

### 2-(Pyridin-2-yl)benzothiazole

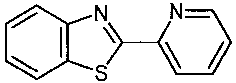

A solution of 2-aminothiophenol (3.50 g, 28 mmol) and pyridine-4-carboxaldehyde (3.00 g, 28 mmol) in EtOH (50 mL) was rapidly stirred (vortexing) under air for 6 h. The solvent was reduced in volume and the solid that separated was heated to dissolution. The mixture was allowed to cool and filtered to give the *title compound* (4.40 g, 74 %) as a beige needles, δ_{H} (CDCl₃, 400 MHz) 7.36 - 7.45 (2H, m), 7.51 (1H, ddd, *J =* 1.0, 7.3 and 8.0 Hz), 7.85 (1H, ddd, *J* = 1.6, 7.6 and 7.8 Hz), 7.96 (1H, ddd, *J =* 0.6, 1.1 and 8.0 Hz), 8.10 (1H, app. br. d, *J =* 8.2 Hz), 8.38 (1H, app. br. d, *J* = 8.0 Hz) and 8.69 (1H, app. br. d, *J =* 4.8 Hz); δ_{C} (CDCl₃, 100 MHz) 120.79, 122.05, 123.60, 125.31, 125.68, 126.31, 136.13, 137.06, 149.69, 151.29, 154.29 and 169.41.

### 6,7-Dihydrobenzothiazolo[3,2-a]pyrido[2,1-c]pyrazine-5,8-diium bis(hexafluorophosphate) - Compound 22:

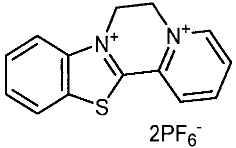

A mixture of 2-(pyridin-2-yl)benzothiazole (1.86 g, 8.8 mmol) in dibromoethane (10 mL) was heated at reflux for 16 h, cooled and filtered. The liquors were then heated at reflux for 24 h, cooled and again filtered. The combined filtrands were washed with Et₂O (2 × 10 mL) and triturated with hot MeOH (3 mL), cooled and the resulting solid filtered to give 6,7-dihydrobenzothiazolo[3,2-*a*]pyrido[2,1-*c*]pyrazine-5,8-diium dibromide (0.99 g) as a yellow powder. The yellow powder was dissolved in water (30 mL) and added dropwise to a solution of NH₄PF₆ (4.81 g, 29.5 mmol) in water (50 mL) with stirring. Stirring was continued for 0.5 h and the resulting precipitate filtered, washed with water (2 × 20 mL) and air dried to give the *title compound* (1.54 g, 33 %) as a sage green powder, δ_{H} [(CD₃)₂CO, 600 MHz] 5.64 - 5.74 (2H, m), 5.74-5.74 (2H, m), 8.02 (1H, app. t, *J =* 7.5 Hz), 8.11 (1H, ddd, *J =* 1.1, 7.3 and 8.5 Hz), 8.53 - 8.68 (3H, m), 8.03 (1H, app. t, *J* = 7.9 Hz), 9.21 (1H, app. d, *J* = 7.9 Hz) and 9.55 (1H, app. br. d, *J* = 6.0 Hz); δ_{F} [(CD₃)₂CO, 470 MHz] -72.55 (d, *J* = 710 Hz); δ_{C} [(CD₃)₂CO, 125 MHz] 45.36, 53.68, 117.82, 125.47, 130.19, 131.42, 131.87, 132.03, 132.70, 136.62, 141.09, 148.83 and 159.55.

### Evaluation of oxido-reduction potentials and absorption spectra of the compounds of the invention

### Method for measuring oxido-reduction potentials

The oxido-reduction potentials of the compounds are measured by cyclic voltammetry with 3 electrodes.

The 3 electrodes used are:
- 1 Platinum working electrode
- 1 Platinum auxiliary or counter electrode
- 1 Platinum reference electrode which is immersed into a solution constituted of 0.01 M AgNO₃ + 0.1 M TBAP (tetrabutylammonium perchlorate) in acetonitrile.

The scan rate of the potential is fixed to 100mV/s.

E₁^{red} corresponds to the first reduction peak of the analyzed compound.

E₂^{red} corresponds to the second reduction peak of the analyzed compound.

E₁ ^{1/2} corresponds to the oxido-reduction potential of an oxidant/reductor system as calculated below: ${{E}_{1}}^{1 / 2} = \left({{E}_{1}}^{red}+{{E}_{1}}^{ox}\right) / 2$ wherein E₁^{ox} corresponds to the first oxidation peak of the analyzed compound.

ΔE^{red} corresponds to the difference between E₁^{red} and E₂^{red} as calculated below: ${ΔE}^{red} = \left|{{E}_{2}}^{red}\right| - \left|{{E}_{1}}^{red}\right| .$

The indicated potential values are the first reduction potentials for the compounds, with respect to the standard hydrogen reference electrode (SHE).

The analyzed solution comprises 0.005 M of the compound to be analyzed and 0.25 M of TBABF₄ salt in propylene carbonate as solvent.

### Method for measuring absorption spectra

This solution is introduced into a quartz cell where at least one working electrode in the form of a platinum grid is placed to colour the analysed compound on this electrode. The absorption spectrum of the analysed compound in the time domain is measured by uv-visible spectroscopy.

The results for each of the synthesized compounds are indicated in Table 1 below. E₁^{red} corresponds to the first reduction potential. The colour indicated in Table 1 is the visual colour perceived by emmetropic eyes under daylight conditions. It should be noted that the λₘₐₓ value just gives an approximate indication of the colour of a particular compound. However, as a consequence of the broad nature of the absorption bands, the whole absorption spectrum has to be taken into account in order to understand the final perceived colour of any one compound.

In comparison, Table 2 shows the results obtained for 7 known compounds (comparative compounds).

By comparing the known compound (comparative compound) **COMP 2** (green) to the new compound **17** (red), we see that introducing a diazine ring in the structure has shifted the colour from green to red. The molecule is red instead of green at its activated state.

By comparing known compounds **COMP 3** (blue) and **COMP 7** (blue) to new compounds **16** (orange), **20** (clear), and **18** (yellow), we see that including different groups like diazine or triazine, alone or included in more complex molecular structures have also this effect of shifting the maximum absorption wavelength to lower values. These groups can be either introduced in between two alkyl bi pyridinium groups or could replace the central pyridinium group of a terpyridinium. The molecules thus obtained are yellow, orange, red, green or purple rather than blue.

We observed that the already known compound **COMP 4,** which contains a phenyl group in between two alkyl bi pyridinium groups, is also orange with an activation potential of -1.21V. The new molecules according to the present invention have a much lower activation potential than this known compound **COMP 4.**

We observed also that the known compounds (comparative compounds) **5** and **6**, already known, contain two nitrogen atoms but with a lower degree of aromaticity or of conjugation than new compound **16** and new compound **17**. Comparative compounds 5 and **6** are blue molecules in their activated state while new compound **16** is orange and new compound **17** is red.

**Table 1**

| **Compound** | **Structure** | **E₁^{1/2} (V)** | **E₁ ^{1/2} (V) vs SHE** | **reversi bility** | **Colour** | **E₁^{red} peak potenti al (V)** | **E1 peak potential (V) vs SHE** | **E₂^{red} peak potential (V)** | **E2 peak potential (V) vs SHE** | **ΔE^{red}** |
|---|---|---|---|---|---|---|---|---|---|---|
| 1 | 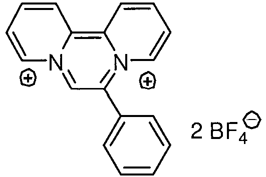 | -0.66 | -0.118 | Y | red/purple | -0.71 | -0.168 | -1.23 | -0.688 | 0.52 |
| 2 | 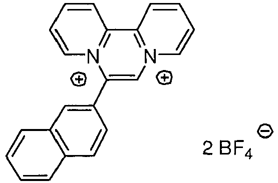 | NA | NA | N | Orange | -0.68 | -0.138 | -1.45 | -0.908 | 0.77 |
| 3 | 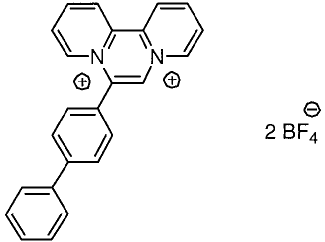 | NA | NA | N | red/purple | -0.68 | -0.138 | -1.4 | -0.858 | 0.72 |
| 4 | 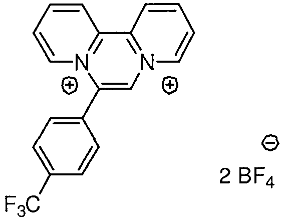 | -0.63 | -0.088 | Y | red/purple | -0.67 | -0.128 | -1.16 | -0.618 | 0.49 |
| 5 | 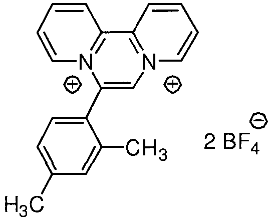 | -0.65 | -0.108 | Y | Purple | -0.69 | -0.148 | -1.26 | -0.718 | 0.57 |
| 6 | 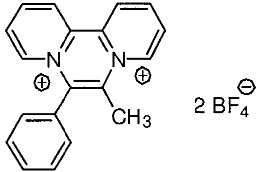 | -0.66 | -0.118 | Y | Purple | -0.7 | -0.158 | -1.23 | -0.688 | 0.53 |
| 7 | 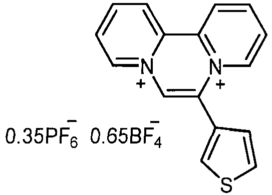 | -0.65 | -0.108 | Y | purple | -0.68 | -0.138 | -1.23 | -0.688 | 0.55 |
| 8 | 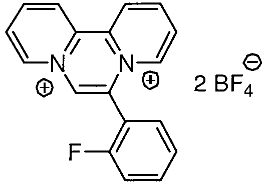 | -0.61 | -0.068 | Y | red/purple | -0.65 | -0.108 | -1.21 | -0.668 | 0.56 |
| 9 | 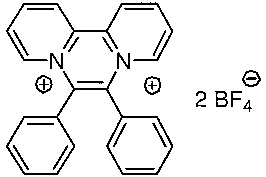 | -0.63 | -0.088 | Y | purple | -0.67 | -0.128 | -1.16 | -0.618 | 0.49 |
| 10 | 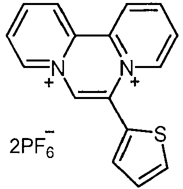 | NA | NA | N | orange | -0.64 | -0.098 | -1.13 | -0.588 | 0.49 |
| 11 | 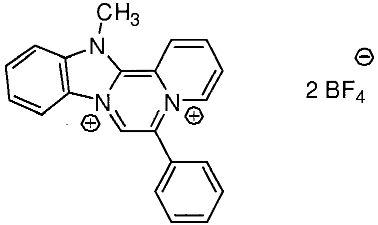 | NA | NA | N | grey | -0.74 | -0.198 | -1.3 | -0.758 | 0.56 |
| 12 | 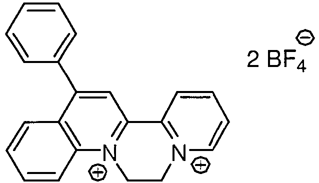 | -0.52 | 0.022 | Y | green | -0.56 | -0.018 | -0.98 | -0.438 | 0.42 |
| 13 | 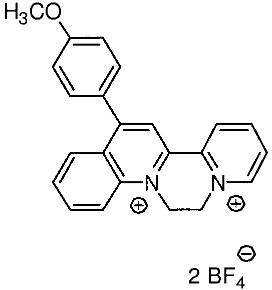 | -0.56 | -0.018 | Y | blue | -0.588 | -0.046 | -1.00 | -0.458 | 0.412 |
| 14 | 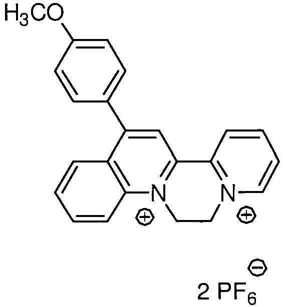 | -0.55 | -0.011 | Y | blue | -0.587 | -0.045 | -1.01 | -0.468 | 0.423 |

| **Compound** | **Structure** | **E₁^{1/2} (V)** | **E₁ ^{1/2} (V) vs SHE** | **reversibility** | **Colour** | **E₁^{red} peak potential (V)** | **E1 peak potential (V) vs SHE** | **E₂^{red} peak potential (V)** | **E2 peak potential (V) vs SHE** | **ΔE^{red}** |
|---|---|---|---|---|---|---|---|---|---|---|
| 15 | 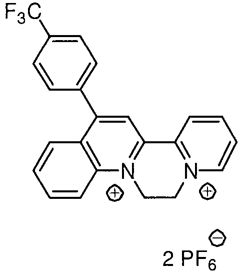 | -0.49 | 0.052 | Y | green | -0.52 | 0.022 | -0.97 | -0.428 | 0.45 |
| 16 | 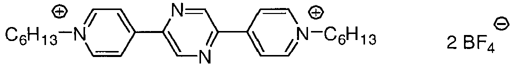 | -0.85 | -0.308 | Y | orange | -0.94 | -0.398 | -1.1 | -0.558 | 0.16 |
| 17 | 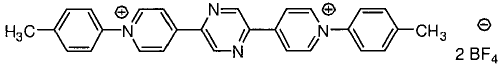 | -0.7 | -0.158 | Y | red | -0.72 | -0.178 | -0.86 | -0.318 | 0.14 |
| 18 | 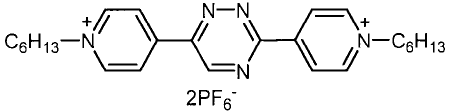 | -0.79 | -0.248 | Y | yellow | -0.83 | -0.288 | -1.01 | -0.468 | 0.18 |

| **Compound** | **Structure** | **E₁^{1/2} (V)** | **E₁ ^{1/2} (V) vs SHE** | **reversi bility** | **Colour** | **E₁^{red} peak potential (V)** | **E1 peak potential (V) vs SHE** | **E₂^{red} peak potential (V)** | **E2 peak potential (V) vs SHE** | **ΔE^{red}** |
|---|---|---|---|---|---|---|---|---|---|---|
| 19 | 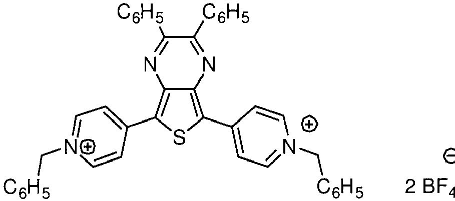 | -0.56 | -0.018 | Y | blue | -0.59 | -0.048 | -0.79 | -0.248 | 0.2 |
| 20 | 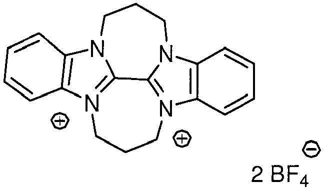 | NA | NA | N | red | -0.97 | -0.428 | -1.27 | -0.728 | 0.3 |
| 21 | 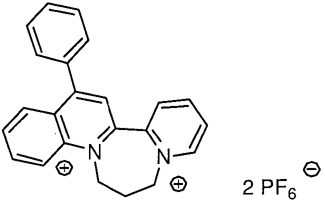 | -0.66 | -0.118 | Y | Yellow/clear | -0.70 | -0.158 | -1.00 | -0.458 | 0.3 |

| **Compound** | **Structure** | **E₁ ^{1/2} (V)** | **E₁ ^{1/2} (V) vs SHE** | **reversibility** | **Colour** | **E₁^{red} peak potential (V)** | **E1 peak potential (V) vs SHE** | **E₂^{red} peak potential (V)** | **E2 peak potential (V) vs SHE** | **ΔE^{red}** |
|---|---|---|---|---|---|---|---|---|---|---|
| 22 | 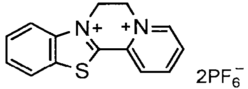 | -0.44 | 0.102 | Y | yellow | -0.48 | -1.022 | -0.93 | -1.472 | 0.45 |

**Table 2**

| **Ref** | **Structure** | **E₁^{1/2} (V)** | **E₁^{1/2} (V) vs SHE** | **reversibility** | **colour** | **E₁^{red} peak potential (V)** | **E1 peak potential (V) vs SHE** | **E₂^{red} peak potential (V)** | **E2 peak potential (V) vs SHE** | **ΔE^{red}** |
|---|---|---|---|---|---|---|---|---|---|---|
| COMP 1 | 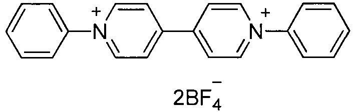 | -0.62 | -0.078 | Y | green | -0.64 | -0.098 | -0.93 | -0.388 | 0.29 |
| COMP 2 | 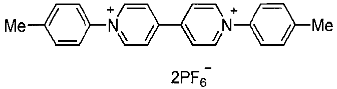 | -0.58 | -0.038 | Y | green | -0.72 | -0.178 | -1.0 | -0.458 | 0.28 |
| COMP 3 | 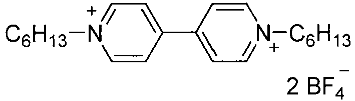 | -0.79 | -0.248 | Y | blue | -0.84 | -0.298 | -1.2 | -0.658 | 0.36 |
| COMP 4 | 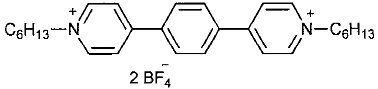 | -1.21 | -0.668 | Y | orange | -1.28 | -0.738 | NA | NA | NA |
| COMP 5 | 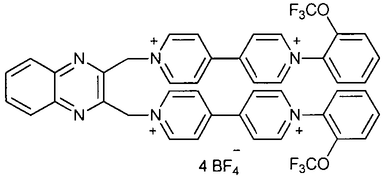 | -0.58 | -0.038 | Y | blue | -0.61 | -0.068 | -1,01 | -0.468 | 0.4 |
| COMP 6 | 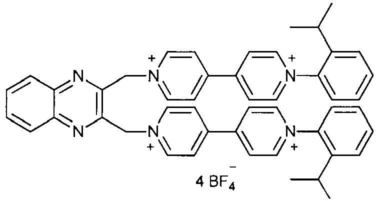 | -0.64 | -0.098 | Y | blue | -0.69 | -0.148 | -1.06 | -0.518 | 0.37 |
| COMP 7 | 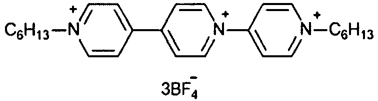 | -0.41 | 0.132 | Y | blue | -0.47 | 0.072 | -0.69 | -0.148 | 0.22 |

The present invention furthermore relates to the following aspects:
1. An electrochromic polycyclic compound represented by Formula (I): 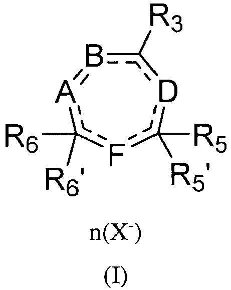 wherein
**A** is N, N-R₁ or ⁺N-R₁;
**B** is C-R₂, or N;
**D** is N, N-R₄ or ⁺N-R₄;
**F** is present or not, and, if present, is -CH₂-;
**R₁** is C₁-C₁₈ alkyl, optionally substituted aryl or -CH₂-aryl;
**R₂** is H or optionally substituted aryl;
**R₃** is H, C₁-C₁₈ alkyl, optionally substituted aryl optionally or substituted heteroaryl such as **Z** or -CH₂-aryl;
**R₄** is C₁-C₁₈ alkyl, optionally substituted aryl or -CH₂-aryl;
**R_{5'}** and **R**_{**6**'} are present or not and, if present, are both H;
**R₅** is H, C₁-C₁₈ alkyl, C₁-C₁₈ alkoxy, optionally substituted aryl or optionally substituted heteroaryl;
**R₆** is H, optionally substituted aryl, optionally substituted heteroaryl or **Z**;
and/or **R₅** and **R₆** may form together an optionally substituted aromatic hydrocarbon ring, such as an arylene, for example a benzene, or a fused ring variant like a naphthalene, or an optionally substituted heteroaromatic ring, such as a thiophene, a selenophene, a pyridine, a pyridinium fused to the central nitrogen-containing heterocyclic ring they are attached to, said aromatic hydrocarbon ring or substituted heteroaromatic ring being optionally substituted for instance by at least one **Y**, or by one or two **Z**;
and/ or **R₃** and **R₄** may form together an optionally substituted nitrogen-containing aromatic ring, such as a pyridine, a pyridinium, a pyrimidine, a pyrimidinium, a pyridazine, a pyridazinium, a pyrazine, a pyrazinium, or an optionally substituted bicyclic aromatic ring containing one or more nitrogen atoms, such as a quinolinium or a benzimidazolium, fused to
the central nitrogen-containing heterocyclic ring they are attached to, said aromatic rings being for example substituted by at least one **Y**;
and/or **R₁** and **R₂** may form together an optionally substituted nitrogen-containing aromatic ring, such as a pyridinium, or an optionally substituted bicyclic aromatic ring containing one nitrogen atom and one or more heteroatoms selected from the group consisting of nitrogen, oxygen, sulfur and selenium, such as a quinolinium or a benzimidazolium, or a benzothiazolium, or a benzoselenazolium, or a benzoxazolium, fused to the central nitrogen-containing heterocyclic ring they are attached to, said aromatic rings being for example substituted by at least one **Y**, for example substituted by an optionally substituted aryl;
and/ or **R₂** and **R₃** may form together an optionally substituted heteroaromatic ring, such as a thiophene, a pyridinium, an optionally substituted aromatic hydrocarbon ring or an optionally substituted nitrogen-containing non aromatic ring, such as seven-membered ring, a saturated diazepine fused to the central nitrogen-containing heterocyclic ring they are attached to, said rings being for example substituted by at least one **Y**;
**Z** is 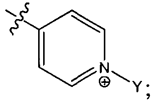
**Y** is C₁-C₁₈ alkyl, optionally substituted aryl or -CH₂-aryl;
**n** is selected to counterbalance the number of positive charges and **n is at least 2**;
**X** is an anion or a mixture thereof;
- - - - - - is a single bond or a double bond;
**with the proviso that** the central nitrogen-containing heterocyclic ring comprises at least two (2) double bonds;
   and
**with the proviso that** said electrochromic polycyclic compound comprises at least two rings in addition to the central nitrogen-containing heterocyclic ring (fused or not to the central nitrogen-containing heterocyclic ring), at least one of said two rings being a nitrogen-containing ring.
2. The electrochromic polycyclic compound of Formula (I) according to aspect 1, wherein **R₁** and **R₂** form together a nitrogen-containing aromatic ring, or a bicyclic aromatic ring containing one nitrogen atom and one or more heteroatoms selected from the group consisting of nitrogen, oxygen, sulfur and selenium, said aromatic rings being fused to the central nitrogen-containing heterocyclic ring they are attached to, and said aromatic rings being optionally substituted by one to three **Y** groups selected from C₁-C₆ alkyl or C₆-C₁₀ aryl, said aryl group being optionally substituted by a C₁-C₆ haloalkyl, or a C₁-C₆ alkoxy.
3. The electrochromic polycyclic compound of Formula (I) according to any of aspects 1 or 2, wherein **R₃** and **R₄** form together a nitrogen-containing aromatic ring, or a bicyclic aromatic ring containing one or more nitrogen atoms, said aromatic rings being fused to the central nitrogen-containing heterocyclic ring they are attached to, and said aromatic rings aromatic rings being optionally substituted by one to three **Y** groups selected from C₁-C₆ alkyl or C₆-C₁₀ aryl, said aryl group being optionally substituted by a C₁-C₆ haloalkyl, or a C₁-C₆ alkoxy.
4. The electrochromic polycyclic compound of Formula (I) according to any of aspects 1 or 3, wherein is **R₂** is H or C₆-C₁₀ aryl, said C₆-C₁₀ aryl being optionally substituted by C₁-C₆ alkyl, C₁-C₆ haloalkyl, or a C₁-C₆ alkoxy.
5. The electrochromic polycyclic compound of Formula (I) according to any of aspects 1, 2 or 4, wherein **R₃** is C₆-C₁₀ aryl or **Z** : 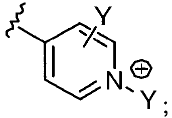 wherein **Y** is is a C₁-C₁₈ alkyl, C₆-C₁₀ aryl, said C₆-C₁₀ aryl being optionally substituted by one to three groups selected from C₁-C₆ alkyl.
6. The electrochromic polycyclic compound of Formula (I) according to any of aspects 1 to 5, wherein **R₅** and **R₆** form together an aromatic hydrocarbon ring, or heteroaromatic ring, said aromatic hydrocarbon ring or heteroaromatic being fused to the central nitrogen-containing heterocyclic ring they are attached to, and said aromatic hydrocarbon ring or heteroaromatic ring being optionally substituted by one or two **Z** : 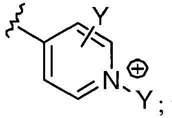 wherein **Y** is -CH₂-(C₆-C₁₀ aryl).
7. The electrochromic polycyclic compound of Formula (I) according to any of aspects 1 to 5, wherein **R₅** is H, C₁-C₆ alkyl, C₁-C₆ alkoxy, C₆-C₁₀ aryl or 5 to 10 membered heteroaryl, said aryl or heteroaryl being optionally substituted by one to three groups selected from Hal, C₁-C₆ alkyl, C₁-C₆ haloalkyl.
8. The electrochromic polycyclic compound of Formula (I) according to any of aspects 1 to 5 and 7, wherein **R₆** is H, C₆-C₁₀ aryl, said aryl being optionally substituted by one to three groups selected from C₁-C₆ alkyl, C₁-C₆ haloalkyl, C₆-C₁₀ aryl, or **R₆** is **Z** : 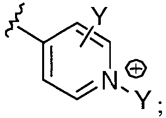 wherein **Y** is a C₁-C₁₈ alkyl, C₆-C₁₀ aryl, said aryl being optionally substituted by one to three groups selected from C₁-C₆ alkyl.
9. The electrochromic polycyclic compound of Formula (I) according to aspect 3, wherein wherein said compound is represented by formula (**III**): 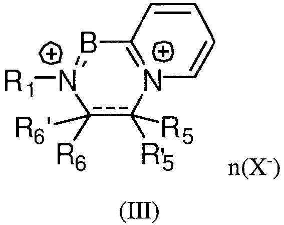 wherein:
**B** is C-R₂;
**R₁** and **R₂** form together an optionally substituted nitrogen-containing aromatic ring,
such as a pyridinium, or an optionally substituted bicyclic aromatic ring with containing one nitrogen atom and one or more heteroatoms selected from the group consisting of nitrogen, oxygen, sulfur and selenium, such as a quinolinium, or a benzimidazolium, or
a benzothiazolium, or a benzoselenazolium, or a benzoxazolium, fused to the central nitrogen-containing six-membered heterocyclic ring they are attached to, said aromatic rings being for example substituted by at least one **Y**, for example substituted by an optionally substituted aryl;
and **R₅, R₆, R_{5'} , R_{6'} , Z**, **Y**, **n** and **X** being as defined in aspect 1.
10. The electrochromic polycyclic compound of Formula (I) according to aspect 9, wherein **R₁** and **R₂** form together an aromatic group selected from a pyridinium, a quinolinium, a benzothiazolium or a benzimidazolium, said aromatic group being fused to the central nitrogen-containing heterocyclic ring they are attached to, and said aromatic group being optionally substituted by one to three **Y** groups selected from C₁-C₆ alkyl or C₆-C₁₀ aryl, said aryl group being optionally substituted by a C₁-C₆ haloalkyl, or a C₁-C₆ alkoxy.
11. The electrochromic polycyclic compound of Formula (I) according to any of aspects 9 or 10, wherein said compound is represented by one of Formulae (IIIa), (IIIb), (IIIc), and (IIId): 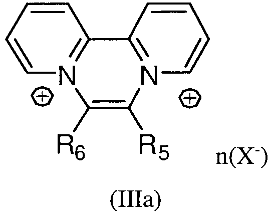 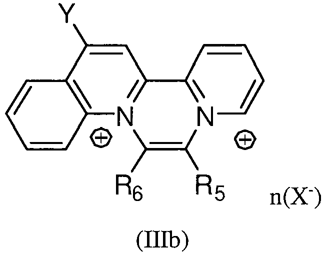 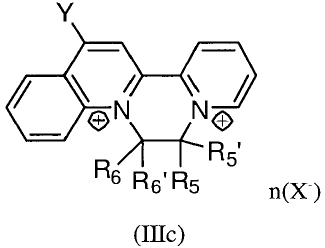 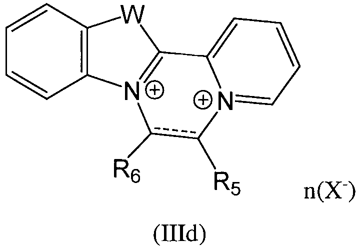 wherein
**R₅** is H, C₁-C₁₈ alkyl, C₁-C₁₈ alkoxy, optionally substituted aryl or optionally substituted heteroaryl;
**R₆** is H, C₁-C₁₈ alkyl, optionally substituted aryl, optionally substituted heteroaryl or **Z**;
**R_{5'}** and **R**_{**6**'} are present or not and, if present, are both H;
**W** is N-Y, S, O or Se;
and **Z**, **Y**, - - - - - -, **n** and **X** are as defined in aspect 1.
12. The electrochromic polycyclic compound of Formula (I) according to any of aspects 9 to 11, wherein **R₅** is selected from the group consisting of H, C₁-C₆ alkyl, C₁-C₆ alkoxy, phenyl or thienyl, said phenyl being optionally substituted by one to three groups selected from Hal, C₁-C₆ alkyl, C₁-C₆ haloalkyl.
13. The electrochromic polycyclic compound of Formula (I) according to any of aspects 9 to 12, wherein **R₆** is H, phenyl, naphthyl, biphenyl or thienyl, said phenyl being optionally substituted by one to three groups selected from C₁-C₆ alkyl, C₁-C₆ haloalkyl or C₆-C₁₀ aryl.
14. The electrochromic polycyclic compound of Formula (I) according to aspect 1, wherein said compound is represented by Formula (IV): 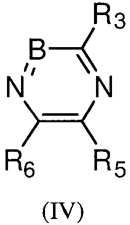 wherein
**B** is C-R₂ or N;
**R₂** is H or optionally substituted aryl; preferably H or phenyl;
**R₃** is optionally substituted aryl or **Z**;
**R₅** is H;
**R₆** is **Z**;

or **R₅** and **R₆** may form together an optionally substituted heteroaromatic ring, such as a thiophene, a pyridinium or an optionally substituted aromatic hydrocarbon ring, such as an arylene, for example a benzene fused to the central nitrogen-containing six-membered heterocyclic ring they are attached to, said heteroaromatic ring or aromatic hydrocarbon ring being for example substituted by at least **Y** or by one or two **Z**;
and **Z**, **Y**, **n** and **X** are as defined in aspect 1.
15. The electrochromic polycyclic aromatic compound of Formula (IV) according to aspect 14, wherein said compound is represented by Formula (VI) or Formula (VII): 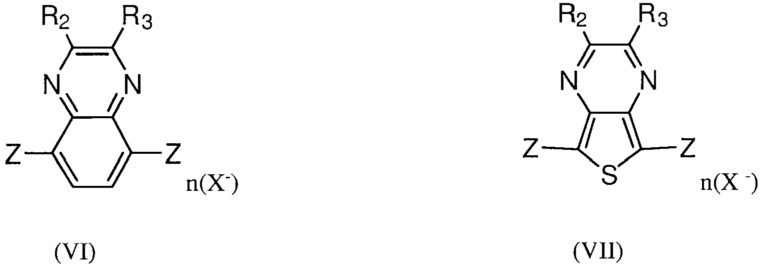 wherein
**R₂** is H or optionally substituted aryl;
**R₃** is H, optionally substituted aryl or **Z**;
**Z**, **Y**, **n** and **X** are as defined in aspect 1.
16. The electrochromic polycyclic compound of Formula (I) according to aspect 14, wherein **Y** is -CH₂-(C₆-C₁₀ aryl).
17. The electrochromic polycyclic compound of Formula (I) according to any of aspects 15 or 16, wherein **R₂** and **R₃** are both phenyl.
18. The electrochromic polycyclic compound of Formula (IV) according to aspect 14, wherein said compound is represented by Formula (V): 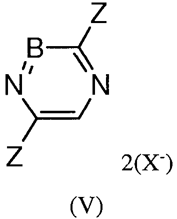 wherein
**B** is CH or N;
**Z**, **Y** and **X** are as defined in aspect 1.
19. The electrochromic polycyclic compound of Formula (V) according to aspect 18, wherein **B** is CH.
20. The electrochromic polycyclic compound of Formula (I) according to any of aspects 18 or 19, wherein **R₆** is **Z** 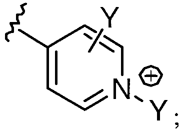 wherein **Y** is n-hexyl, or tolyl.
21. The electrochromic polycyclic compound of Formula (I) according to aspect 1, wherein said compound is represented by Formula (IX) 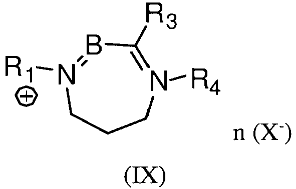 wherein
B is C-R₂;
**R₃** and **R₄** form together an optionally substituted nitrogen-containing aromatic ring, such as a pyridinium or a benzimidazolium, fused to the central nitrogen-containing seven-membered heterocyclic ring they are attached to, said aromatic ring being for example substituted by at least one **Y**, preferably said aromatic ring is not substituted;
**R₁** and **R₂** form together an optionally substituted nitrogen-containing aromatic ring, such as a pyridinium, a quinolinium, or a benzimidazolium fused to the central nitrogen-containing seven-membered heterocyclic ring they are attached to, for example substituted by an optionally substituted aryl;
and **R₂** and **R₃** may form together an optionally substituted nitrogen-containing non-aromatic ring, such as seven-membered ring, a saturated diazepine, fused to the central nitrogen-containing seven-membered heterocyclic ring they are attached to, said non-aromatic ring being preferably not substituted; and **Y**, **n** and **X** are as defined in aspect 1.
22. The electrochromic polycyclic compound of Formula (I) according to aspect 21, wherein **R₃** and **R₄** form together a heteroaromatic group selected from pyridinium or benzimidazolium, wherein said heteroaromatic group fused to the central nitrogen-containing seven-membered heterocyclic ring they are attached to.
23. The electrochromic polycyclic compound of Formula (I) according to any of aspects 21 or 22, wherein **R₁** and **R₂** form together a heteroaromatic group selected from a pyridinium, a quinolinium, or a benzimidazolium, wherein said heteroaromatic group is fused to the central nitrogen-containing seven-membered heterocyclic ring they are attached to, and wherein said heteroaromatic group is optionally substituted by a phenyl group.
24. The electrochromic polycyclic compound of Formula (I) according to aspects 21 to 23, wherein **R₂** and **R₃** form together a saturated diazepine fused to the central nitrogen nitrogen-containing heterocyclic ring they are attached to.
25. The electrochromic polycyclic compound of Formula (I) according to any of aspects 1 to 24, wherein **n** is 2.
26. The electrochromic polycyclic compound of Formula (I) according to any of aspects 1 to 25, wherein the counterion **X-** is selected from halide, preferably fluoride and chloride, tetrafluoroborate, tetraphenylborate, hexafluorophosphate, nitrate, methanesulfonate, trifluoromethane sulfonate, toluene sulfonate, hexachloroantimonate, bis(trifluoromethanesulfonyl)imide, perchlorate, acetate and sulfate, or any mixture thereof.
27. The electrochromic polycyclic compound of Formula (I) according to aspect 26, wherein **X⁻** is tetrafluoroborate or hexafluorophosphate.
28. The electrochromic polycyclic compound of Formula (I) according to aspect 1, wherein said compound is selected from:

| | |
|---|---|
| Compound 1 | 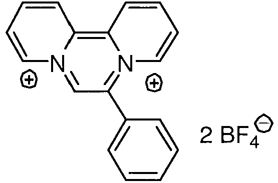 |

| | |
|---|---|
| Compound 2 | 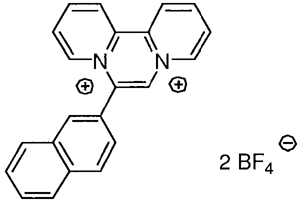 |
| Compound 3 | 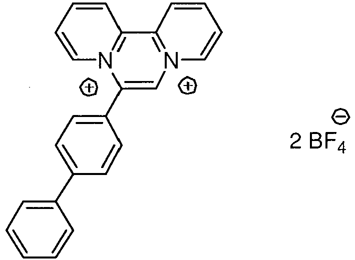 |
| Compound 4 | 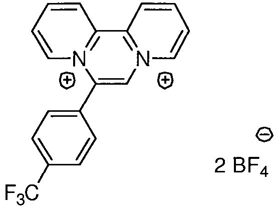 |
| Compound 5 | 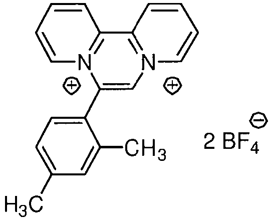 |
| Compound 6 | 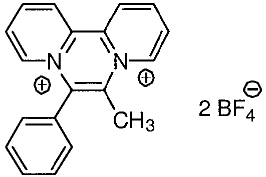 |
| Compound 7 | 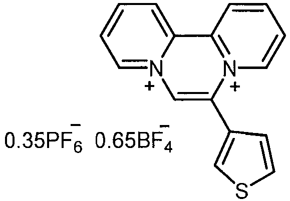 |

| | |
|---|---|
| Compound 8 | 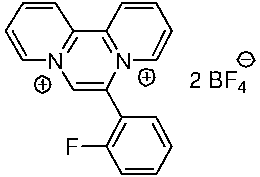 |
| Compound 9 | 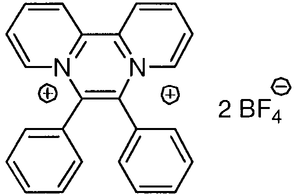 |
| Compound 10 | 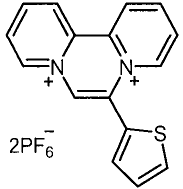 |
| Compound 11 | 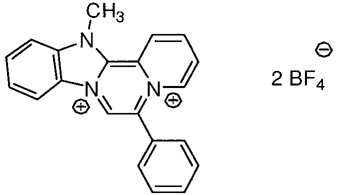 |
| Compound 12 | 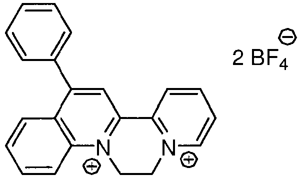 |
| Compound 13 | 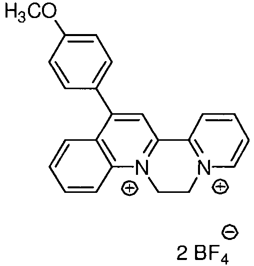 |

| | |
|---|---|
| Compound 14 | 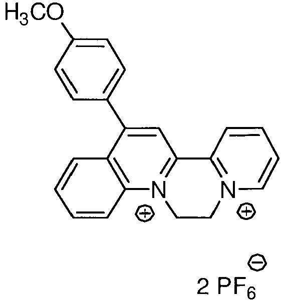 |
| Compound 15 | 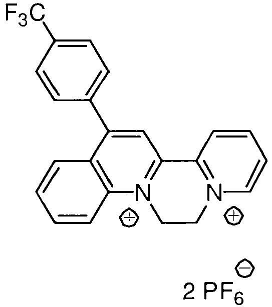 |
| Compound 16 | 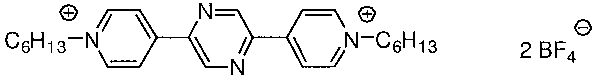 |
| Compound 17 | 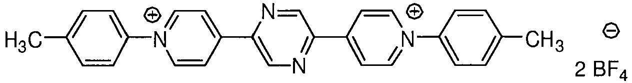 |
| Compound 18 | 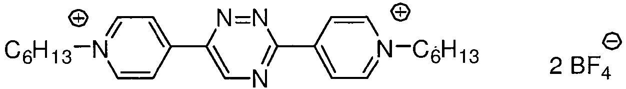 |
| Compound 19 | 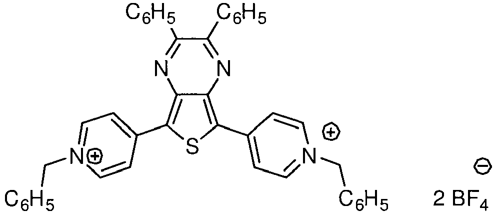 |
| Compound 20 | 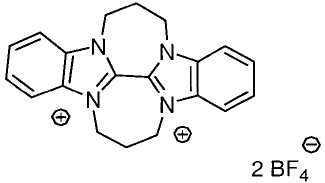 |

| | |
|---|---|
| Compound 21 | 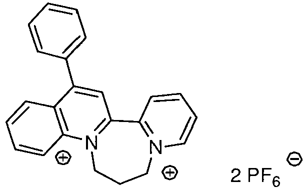 |
| Compound 22 | 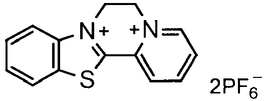 |

29. An electrochromic composition comprising at least one electrochromic polycyclic compound as defined in any one of aspects 1 to 28, and a host medium.
30. The electrochromic composition according to aspect 29, wherein said composition further comprises a host medium that is a fluid, a mesomorphous media or a gel.
31. The electrochromic composition according to any of aspects 29 or 30, wherein the host medium is selected from the group consisting of organic solvents, liquid crystals, polymers, liquid crystal polymers and mixtures thereof.
32. The electrochromic composition according to aspect 31, wherein the organic solvent is selected from the group consisting of ethylene carbonate, propylene carbonate, γ-butyrolactone, γ-valerolactone, acetronitrile, propionitrile, benzonitrile, glutaronitrile, methylglutaronitrile, dimethylformamide, N-methylpyrrolidone, sulfolane, 3-methyl sulfolane, benzene, toluene, methyl ethyl ketone, acetone, ethanol, tetrahydrofurfuryl alcohol, 2-methoxyethyl ether, xylene, cyclohexane, 3-methylcyclohexanone, ethyl acetate, ethyl phenylacetate, tetrahydrofuran, methanol, methyl propionate, ethylene glycol, ethylene carbonate, ionic liquids, and mixtures thereof.
33. The electrochromic composition according to any of aspects 31 or 32, wherein the host medium is a mixture of liquid crystal polymers with an organic solvent.
34. The electrochromic composition according to aspect 31, wherein the polymer is selected from the group consisting of and acrylate polymer, polyurethane, polyethylene oxide, polypropylene oxide, polyvinyl acetate, poly(N-vinyl pyrrolidone), and polyvinylidene fluoride.
35. An electrochromic device comprising at least one electrochromic polycyclic compound as defined in any aspects 1 to 28, or an electrochromic composition as defined in any of aspects 29 to 34.
36. The electrochromic device according to aspect 35, wherein said electrochromic device is selected from an optical article, preferably an optical lens or an optical filter, a window, notably aircraft or automotive or building window, a visor, a mirror, a head mounted device and a display element, more preferably an optical article selected from optical lenses, and most preferably an optical article selected from ophthalmic lenses.
37. The electrochromic device according to aspect 36, wherein the ophthalmic lens is a corrective or a non-corrective lens.
38. The electrochromic device according to any of aspects 36 or 37, wherein the ophthalmic lens is selected from the group consisting of contact lenses, intra-ocular lenses, sunglasses, ski goggles, magnifying lenses, protective lenses and visors.
39. The electrochromic device according to aspect 36, wherein the display element is a screen or a monitor.
40. The electrochromic device according to aspect 36, wherein the window is selected from the group consisting of an automotive window, a marine window, an aircraft window, a building window, and a variable transmission device.
41. The electrochromic device according to aspect 35, wherein said electrochromic device comprises a pair of opposed substrates having a gap there between for receiving a mixture of the host medium and at least one electrochromic polycyclic compound as defined in any aspects 1 to 28 or an electrochromic composition as defined in any of aspects 29 to 34, and a frame for holding said pair of substrates adjacent one another.
42. The electrochromic device according to aspect 35, wherein said electrochromic device comprises an optical component provided with at least one transparent cell arrangement juxtaposed in a parallel direction to the surface thereof, each cell being tightly closed and containing at least one electrochromic polycyclic compound as defined in any aspects 1 to 28, or an electrochromic composition as defined in any of aspects 29 to 34.

## Claims

1. An electrochromic polycyclic compound represented by one of Formulae (IIIa), (IIIb), (IIIc), (IIId), (V), and (VII): 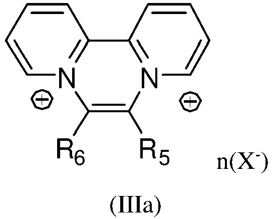 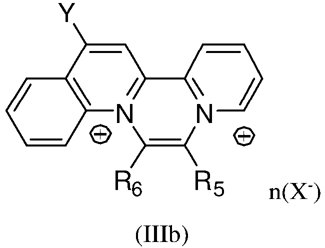 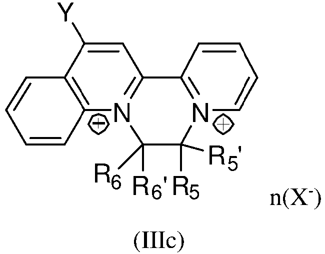 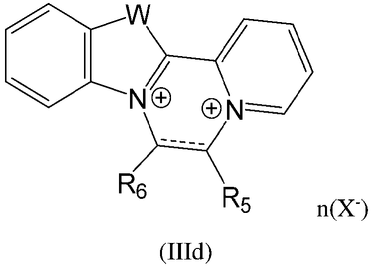 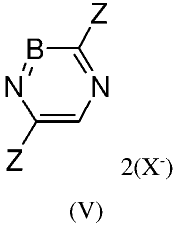 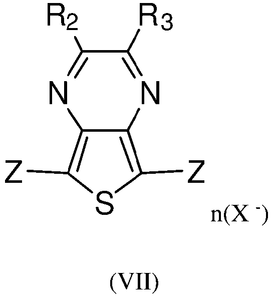 wherein
**B** is N
**R₂** is H or optionally substituted aryl;
**R₃** is H, optionally substituted aryl or **Z**;
**R₅** is H, C₁-C₁₈ alkyl, C₁-C₁₈ alkoxy, optionally substituted aryl or optionally substituted heteroaryl;
**R₆** is H, C₁-C₁₈ alkyl, optionally substituted aryl, optionally substituted heteroaryl or **Z**; **R_{5'}** and **R**_{**6**'} are present or not and, if present, are both H;
**W** is N-Y, S, O or Se;
and
**Z** is 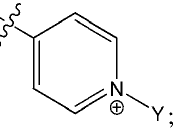
**Y** is C₁-C₁₈ alkyl, optionally substituted aryl or -CH₂-aryl;
**n** is selected to counterbalance the number of positive charges and **n is at least 2**;
**X** is an anion or a mixture thereof;
- - - - - - is a single bond or a double bond;
wherein in formula (IIIa), **R6** is optionally substituted aryl, or optionally substituted heteroaryl.

2. The electrochromic polycyclic compound according to claim 1, wherein, in formula (IIIa), **R₅** is selected from the group consisting of H, C₁-C₆ alkyl, C₁-C₆ alkoxy, phenyl or thienyl, said phenyl being optionally substituted by one to three groups selected from Hal, C₁-C₆ alkyl, C₁-C₆ haloalkyl.

3. The electrochromic polycyclic compound according to claim 1 or 2, wherein, in formula (IIIb), **R₅** is selected from the group consisting of H, C₁-C₆ alkyl, C₁-C₆ alkoxy, phenyl or thienyl, said phenyl being optionally substituted by one to three groups selected from Hal, C₁-C₆ alkyl, C₁-C₆ haloalkyl.

4. The electrochromic polycyclic compound according to any one of claims 1 to 3, wherein, in formula (IIIc), **R₅** is selected from the group consisting of H, C₁-C₆ alkyl, C₁-C₆ alkoxy, phenyl or thienyl, said phenyl being optionally substituted by one to three groups selected from Hal, C₁-C₆ alkyl, C₁-C₆ haloalkyl.

5. The electrochromic polycyclic compound according to any one of claims 1 to 4, wherein, in formula (IIId), **R₅** is selected from the group consisting of H, C₁-C₆ alkyl, C₁-C₆ alkoxy, phenyl or thienyl, said phenyl being optionally substituted by one to three groups selected from Hal, C₁-C₆ alkyl, C₁-C₆ haloalkyl.

6. The electrochromic polycyclic compound according to any one of claims 1 to 5, wherein, in formula (IIIa), **R₆** is phenyl, naphthyl, biphenyl or thienyl, said phenyl being optionally substituted by one to three groups selected from C₁-C₆ alkyl, C₁-C₆ haloalkyl or C₆-C₁₀ aryl.

7. The electrochromic polycyclic compound according to any one of claims 1 to 6, wherein, in formula (IIIb), **R₆** is H, phenyl, naphthyl, biphenyl or thienyl, said phenyl being optionally substituted by one to three groups selected from C₁-C₆ alkyl, C₁-C₆ haloalkyl or C₆-C₁₀ aryl.

8. The electrochromic polycyclic compound according to any one of claims 1 to 7, wherein, in formulae (IIIc), **R₆** is H, phenyl, naphthyl, biphenyl or thienyl, said phenyl being optionally substituted by one to three groups selected from C₁-C₆ alkyl, C₁-C₆ haloalkyl or C₆-C₁₀ aryl.

9. The electrochromic polycyclic compound according to any one of claims 1 to 8, wherein, in formulae (IIId), **R₆** is H, phenyl, naphthyl, biphenyl or thienyl, said phenyl being optionally substituted by one to three groups selected from C₁-C₆ alkyl, C₁-C₆ haloalkyl or C₆-C₁₀ aryl.

10. The electrochromic polycyclic compound according to any one of claims 1 to 9, wherein **R₂** and **R₃** are both phenyl.

11. The electrochromic polycyclic compound according to any one of claims 1 to 10, wherein wherein, in formula (V), **Y** is *n*-hexyl, or tolyl.

12. The electrochromic polycyclic compound according to any one of claims 1 to 11, wherein the counterion X- is selected from halide, preferably fluoride and chloride, tetrafluoroborate, tetraphenylborate, hexafluorophosphate, nitrate, methanesulfonate, trifluoromethane sulfonate, toluene sulfonate, hexachloroantimonate, bis(trifluoromethanesulfonyl)imide, perchlorate, acetate and sulfate, or any mixture thereof.

13. An electrochromic composition comprising at least one electrochromic polycyclic compound as defined in any one of claims 1 to 12, and a host medium that is a fluid, a mesomorphous media or a gel.

14. An electrochromic device comprising at least one electrochromic compound as defined in any one of claims 1 to 12, or an electrochromic composition as defined in claim 13.

15. The electrochromic device according to claim 14, wherein said electrochromic device is selected from an optical article; preferably an optical lens or an optical filter; a window, notably an aircraft or an automotive or a building window; a visor; a mirror; a head mounted device; and a variable transmission device; preferably wherein said optical article is an optical lens, and most preferably wherein said optical article is an ophthalmic lens.
